# EUROPEAN PATENT APPLICATION

(11) **EP 2 377 559 A1**
(43) Date of publication of application: **19.10.2011**
(21) Application number: 11161737.9
(22) Date of filing: 02.06.2006
(51) Int. Cl.: A61K 48/00, A61P 35/00, C12Q 1/68, G01N 33/48, C12N 15/11, C12N 15/85

(54) **Targeting cells with altered microRNA expression**

(30) Priority: 03.06.2005 US 687547 P
(62) Divisional of application: 06741167.8
(71) Applicant: Southern Adelaide Health Service - Flinders Medical Centre, Bedford Park, SA 5042 (AU)
(72) Inventor: Michael, Michael Zenon, Belair South Australia 5052 (AU)
(74) Representative: Dzieglewska, Hanna Eva

(57) **Abstract**

The present invention relates to a method of modulating development of a cell. The method includes the step of introducing into the cell a nucleic acid with the capacity to modulate development of the cell, the nucleic acid including a target site for binding of a microRNA, wherein the activity and/or concentration of the microRNA in the cell results in a level of activity and/or concentration of the nucleic acid in the cell sufficient to modulate development of the cell.

## Description

This application claims priority from United States Provisional Patent Application No. 60/687,547 filed on 3 June 2005, the contents of which are to be taken as incorporated herein by this reference.

### Field of the Invention

The present invention relates to a method of modulating the development of a cell with altered microRNA activity, and to a method of preventing and/or treating a disease, condition or state associated with altered microRNA activity.

The present invention also relates to nucleic acids having a binding site for a microRNA, cells and animals including such nucleic acids, and compositions including the nucleic acids.

### Background of the Invention

Targeted gene expression is one of the most difficult and important goals in the development of effective therapies for a variety of disorders, including cell proliferative disorders such as cancer. The ultimate aim of such therapies is the provision of controlled, sustained, and site-specific expression of a therapeutic agent such that surrounding healthy tissue remains relatively unaffected by the effects of the therapeutic agent.

However, one major limitation of current gene therapy protocols has been the inability to control expression of the therapeutic gene, and in particular, the inability to restrict expression of the delivered gene to the desired tissue or type of cell. In this regard, although tissue specific promoters may be adequate to achieve specific expression of an agent in some target tissues, for diseases such as cancer they have proved to be of less value as non-diseased cells may also express from the promoter. Accordingly, there is a need to identify new methods of targeting expression of therapeutic nucleic acids. Recently, short 20-22 nucleotide RNA molecules known as microRNAs (miRNAs) have been identified as regulating gene expression in variety of eukaryotic systems. In *Caenorhabditis elegans*, miRNAs coordinate the transitions between stages of larval development by regulating the translation of heterochromic genes. A specific miRNA in *Arabidopsis* has been shown to direct the cleavage of transcripts encoding several putative transcription factors. The Drosophila *bantam* gene encodes a miRNA that regulates cell proliferation and the proapoptotic gene *hid.* More recently, human miR143 has been shown to regulate adipocyte differentiation.

miRNAs are formed from larger transcripts that fold to produce hairpin structures and serve as substrates for the Dicer family of RNase III enzymes. They share this process with an experimental system, RNA interference (RNAi), which may be used to silence the expression of endogenous genes in eukaryotic cells. The products of Dicer cleavage are short dsRNA molecules, one strand of which is retained in a ribonucleoprotein complex called the RNA-induced silencing complex (RISC). The retained RNA acts as a guide to target this complex to a complementary mRNA sequence which is inactivated either by cleavage or translational interference, depending on the degree of complementarity between the miRNA and its target.

The present invention relates to a method of modulating the development of cells with altered microRNA levels, and arises from the recognition that some diseased cells have altered expression levels of endogenous microRNAs and that expression of therapeutic nucleic acids may be effectively targeted to such cells by exploiting the altered expression of the microRNAs in these cells.

A reference herein to a patent document or other matter which is given as prior art is not to be taken as an admission that that document or matter was known or that the information it contains was part of the common general knowledge as at the priority date of any of the claims.

### Summary of the Invention

The present invention provides a method of modulating development of a cell, the method including the step of introducing into the cell a nucleic acid with the capacity to modulate development of the cell, the nucleic acid including a target site for binding of a microRNA, wherein the activity and/or concentration of the microRNA in the cell results in a level of activity and/or concentration of the nucleic acid in the cell sufficient to modulate development of the cell.

The present invention also provides a nucleic acid with the capacity to modulate development of a cell, the nucleic acid including a binding site for a microRNA.

The present invention also provides a nucleic acid including a non-naturally occurring binding site for a microRNA that is differentially expressed and/or has differential activity.

The present invention also provides a cancerous cell including an exogenous nucleic acid including a binding site for a microRNA, wherein the cancerous cell has a reduced activity and/or concentration of the microRNA as compared to a similar non-cancerous cell.

The present invention also provides an animal including cancerous cells, the cancerous cells including an exogenous nucleic acid including a target site for binding of a microRNA.

The present invention also provides a method of preventing and/or treating a disease, condition or state associated with target cells in a subject, the method including the step of introducing into cells in the subject a nucleic acid with the capacity to modulate development of a cell, the nucleic acid including a target site for binding of the microRNA, wherein the activity of the microRNA in the target cells results in a level of activity of the nucleic acid sufficient to modulate development of the target cells in the subj ect.

The present invention also provides a method of detecting altered microRNA activity and/or concentration in a cancerous or pre-cancerous cell, the method including the steps of:
determining the level of expression of a reporter nucleic acid in the cancerous or pre-cancerous cells and determining the level of expression of a reporter nucleic acid in non-cancerous cells; and
detecting a reduced activity of the microRNA in the cancerous cells by an increase in the expression of the reporter nucleic acid in the cancerous cells as compared to the level of expression of the reporter nucleic acid in the non-cancerous cells.

The present invention also provides a method of modulating the concentration of a nucleic acid expressed in a cancerous cell, the cancerous cell having an altered activity and/or concentration of a microRNA as compared to a similar non-cancerous cell, the method including the step of introducing a target site for binding of the microRNA into the nucleic acid to be expressed in the cell.

The present invention arises from the recognition that many cells, including cancerous cells, have altered expression levels of endogenous microRNAs, and that expression of therapeutic nucleic acids may be more effectively targeted to such cells by exploiting the altered expression of the microRNAs in these cells.

In addition, it has also been recognised that the levels of some microRNAs are modulated during differentiation of cells or during their normal developmental programme, and accordingly, expression of nucleic acids may also be targetted to these cells at specific times in their developmental programme and/or at times when the level of the microRNAs changes.

Various terms that will be used throughout the specification have meanings that will be well understood by a skilled addressee. However, for ease of reference, some of these terms will now be defined.

The term "development" as used throughout the specification in relation to the development of a cell is to be understood to mean the continuance of a cell in its current state or the continuance of a cell in its normal developmental and/or metabolic program.

In this regard, modulation of the development of a cell may, for example, result in an inhibition or cessation of cell growth, cell death, or an alteration in the normal developmental pathway that a cell undergoes. Alternatively, modulation of the development of a cell may result, for example, in increased cell survival or rescue, or increased cell proliferation.

The term "modulate" or variants thereof as used throughout the specification is to be understood to mean any alteration (increase or decrease) in the activity of a process. For example, alteration may result in activation of a process, inhibition of a process, a change in the timing of a process or a change in probability that a process may occur.

The term "nucleic acid" as used throughout the specification is to be understood to mean to any oligonucleotide or polynucleotide. The nucleic acid may be DNA or RNA and may be single stranded or double stranded. The nucleic acid may be any type of nucleic acid, including a nucleic acid of genomic origin, cDNA origin (ie derived from a mRNA), derived from a virus, or of synthetic origin. It will be appreciated that a nucleic acid with the capacity to modulate development of a cell is a nucleic acid that in a particular cell either inhibits or promotes development of the cell.

In this regard, an oligonucleotide or polynucleotide may be modified at the base moiety, sugar moiety, or phosphate backbone, and may include other appending groups to facilitate the function of the nucleic acid. The oligonucleotide or polynucleotide may be modified at any position on its structure with constituents generally known in the art. For example, an oligonucleotide may include at least one modified base moiety which is selected from the group including 5-fluorouracil, 5-bromouracil, 5-chlorouracil, 5-iodouracil, hypoxanthine, xanthine, 4-acetylcytosine, 5- (carboxyliydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta D-mannosylqueosine, 5'-methoxycarboxymethyluracil, 5-methoxyuracil, 2-methylthio-N6- isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil5-oxyacetic acid methylester, uracil-5-oxyacetic acid (v), 5-methyl-2-thiouracil, 3- (3-amino-3-N-2-carboxypropyl) uracil, (acp3) w, and 2,6-diaminopurine.

The oligonucleotide or polynucleotide may also include at least one modified sugar moiety selected from the group including, but not limited to, arabinose, 2-fluoroarabinose, xylulose, and hexose. In addition, the oligonucleotide or polynucleotide may include at least one modified phosphate backbone, such as a phosphorothioate, a phosphorodithioate, a phosphoramidothioate, a phosphoramidate, a phosphordiamidate, a methylphosphonate, an alkyl phosphotriester, and a formacetal or any analogue thereof.

The term "subject" as used throughout the specification is to be understood to mean any multicellular organism, including an animal or human subject. For example, the subject may be a mammal such as a primate, a livestock animal (eg. A horse, a cow, a sheep, a pig, a goat), a companion animal (eg. a dog, a cat), a laboratory test animal (eg. a mouse, a rat, a guinea pig, a bird), an animal of veterinary significance, or an animal of economic significance.

The term "variant" as used throughout the specification is to be understood to mean an amino acid sequence of a polypeptide or protein that is altered by one or more amino acids. The variant may have "conservative" changes, wherein a substituted amino acid has similar structural or chemical properties to the replaced amino acid (e.g., replacement of leucine with isoleucine). A variant may also have "non-conservative" changes (e.g., replacement of a glycine with a tryptophan) or a deletion and/or insertion of one or more amino acids. The term also includes within its scope any insertions/deletions of amino acids for a particular polypeptide or protein. A "functional variant" will be understood to mean a variant that retains the functional capacity of a reference protein or polypeptide.

### Brief Description of the Figures

Figure 1 shows in the top panel the stem-loop structure of the hsa-miR-143 precursor and the nucleotide sequence of the mature hsa-miR-143 microRNA. The bottom panel shows the stem-loop structure of the hsa-miR-145 precursor and the nucleotide sequence of the mature hsa-miR-145 microRNA.
Figure 2 shows EGFP fluorescence (three days post-transfection) from HeLa cells cotransfected with 0.1µg EGFP/RICS-miR145 target sequence expression vector (pMM095) and varying levels of pri-miR145 (single- pMM109, and tandem- pMM107) expression plasmids, antisense (A/S; pMM106) and empty vector controls (pcDNA3.1). Data are representative of two experiments.
Figure 3 shows the map for plasmid pMM043.

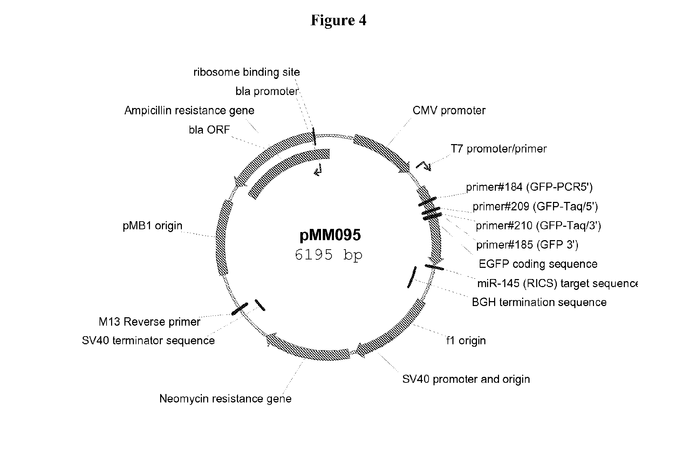

Figure 4 shows the map for plasmid pMM095. The nucleotide sequence of pMM095 is provided in the sequence listing and is designated SEQ ID NO. 153.

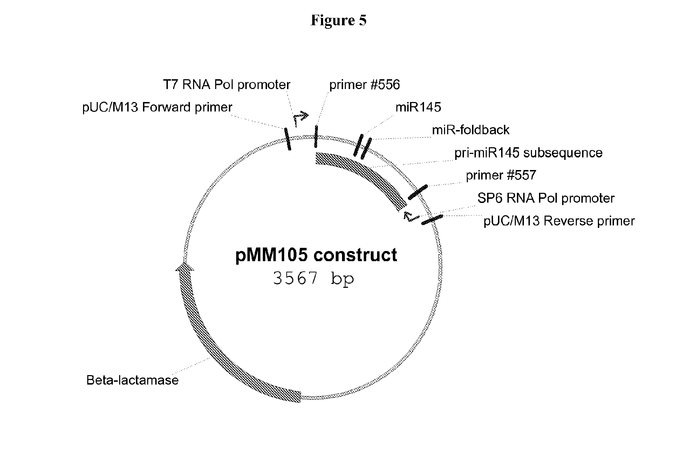

Figure 5 shows the map for plasmid pMM105. The nucleotide sequence of pMM105 is provided in the sequence listing and is designated SEQ ID NO. 154.
Figure 6 shows the map for plasmid pMM106. The nucleotide sequence of pMM106 is provided in the sequence listing and is designated SEQ ID NO. 155.
Figure 7 shows the map for plasmid pMM107. The nucleotide sequence of pMM107 is provided in the sequence listing and is designated SEQ ID NO. 156.
Figure 8 shows the map for plasmid pMM109. The nucleotide sequence of pMM109 is provided in the sequence listing and is designated SEQ ID NO. 157.
Figure 9 shows the map for plasmid pMM-TK/miRTarg. The nucleotide sequence of pMM-TK/miRTarg is provided in the sequence listing and is designated SEQ ID NO. 158.
Figure 10 shows the map for plasmid pMM-CD/miRTarg. The nucleotide sequence of pMM-CD/miRTarg is provided in the sequence listing and is designated SEQ ID NO. 159.
Figure 11 shows in the top panel real time RT-PCR quantitation of relative pri-miR145 levels 24h post Dox-induction. The lower panel shows accumulation of mature miR145 in HeLa Tet-On/pMM110d line, following 24 h incubation in 1µg/mL doxycycline, by Northern analysis: 20µg total RNA/sample, 15% denaturing PAGE minigel. The ethidium bromide stained gel is shown to compare loading of samples.

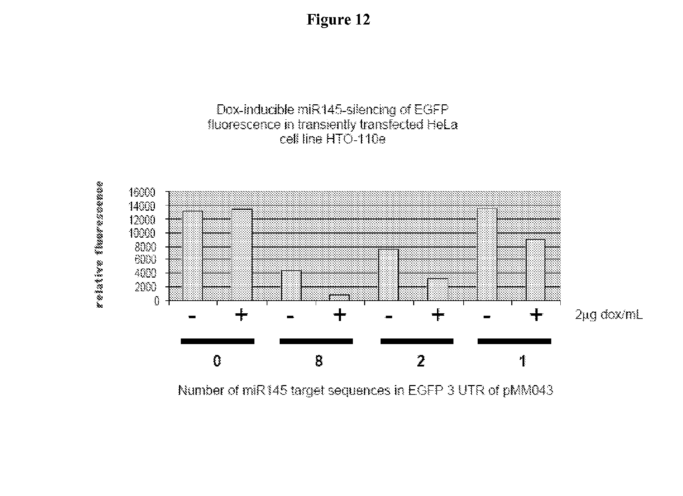

Figure 12 shows the effect of increasing miRNA target sequences in the 3'UTR of a transgene. Cells of the stable pMM110 transgenic HeLa Tet On cell line, HT0110e, were grown in the presence, or absence, of 2µg doxycycline/mL medium and FuGene6-transfected, one after plating, with 80 ng plasmid. The plasmids used for transfection were all derived from pMM043, with varying numbers of miR145 target sequences inserted in the EGFP 3'UTR NotI site. Plasmids were: pMM043 (no targets), pMM095 (1 target), pMM117 (2 targets), pMM119 (8 targets). Values displayed are the mean fluorescence (n=3) at 46 hours after transfection.

### General Description of the Invention

As mentioned above, in one form the present invention provides a method of modulating development of a cell, the method including the step of introducing into the cell a nucleic acid with the capacity to modulate development of the cell, the nucleic acid including a target site for binding of a microRNA, wherein the activity and/or concentration of the microRNA in the cell results in a level of activity and/or concentration of the nucleic acid in the cell sufficient to modulate development of the cell.

The present invention arises from the recognition that some diseased cells have altered expression levels of endogenous microRNAs, and that expression of nucleic acids may be effectively targeted to these cells by exploiting the altered expression of the microRNAs in the cells.

It will be appreciated that in the case of a reduced miRNA expression, the expression of the nucleic acids is in fact effectively de-targeted.

Thus, the present invention has application in fields such as genetic engineering and therapeutic gene transfer.

The present invention is suitable for example for targeting expression of a cytotoxic nucleic acid to a diseased cell so as to ablate the cell, or alternatively, for targeting expression of a therapeutic nucleic acid to a diseased cell to improve one or more characteristics of the cell (eg for gene therapy purposes).

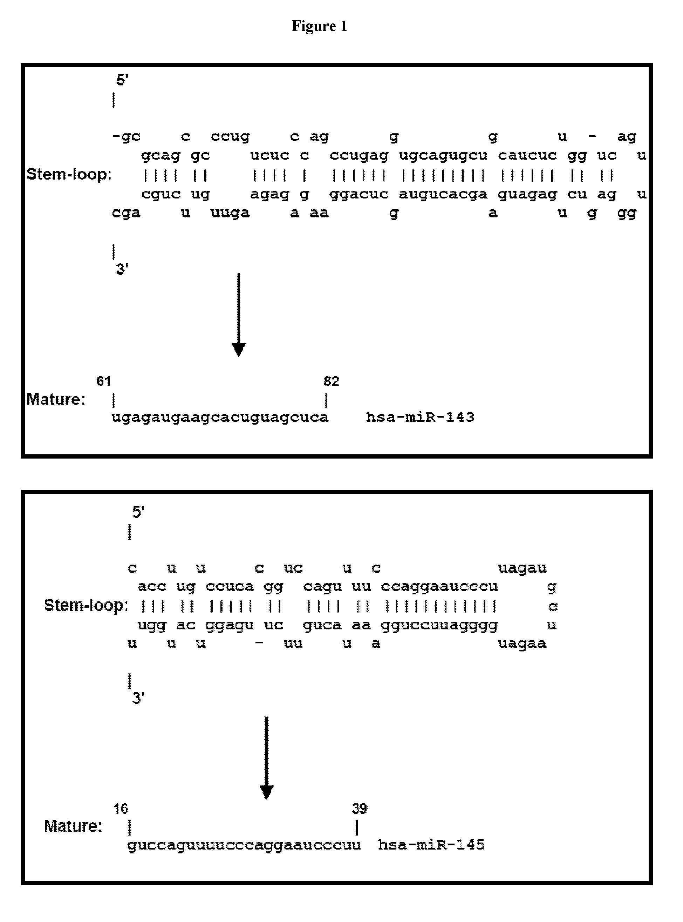

For example, previous studies of microRNAs differentially expressed between colonic adenocarcinoma cells and matched normal mucosa have identified two microRNAs, miR-143 and miR-145, that show significantly reduced levels of the fully processed miRNA in tumors compared to normal tissues. These miRNAs are produced from hairpin precursor (pre-miRNAs) that are cleaved to the shorter mature miRNA, as shown in Figure 1. Thus, the present invention may be used, for example, to modulate the development of cells having reduced expression of the miR-143 or miR-145 microRNAs.

The alteration in the level and/or activity of the one or more microRNAs in the cell may be a constitutive alteration, or alternatively, may be an alteration that occurs at a specific point(s) in the developmental programme of the cell. For example, the present invention is suitable for modulating development of a cancer cell that shows a reduced constitutive expression and/or activity of a particular miRNA, for modulating development of a cell infected with a virus which causes altered expression and/or activity of a miRNA, or for modulating development of an embryonic or adult stem cell that modulates the activity of a specific miRNA at specific stages of its developmental programme.

Confirmation that a cell has an altered activity and/or expression of a specific miRNA may be achieved by a suitable method known in the art, such as Northern analysis, reverse transcription PCR (RT-PCR) or RNase protection.

Alternatively confirmation that a cell has an altered activity and/or expression of a specific miRNA may be achieved by way of expression of a reporter gene linked to a target site for the particular miRNA. In this case, the level of expression of the reporter gene will inversely reflect the activity and/or expression level of the miRNA in the cell.

Methods for the construction of reporter genes including a target site for a miRNA are known in the art. For example, a target site may be cloned into the 3'UTR of GFP, and the construct introduced into the cell. Methods for the cloning of nucleic acid sequences are as described in Sambrook, J, Fritsch, E.F. and Maniatis, T. Molecular Cloning: A Laboratory Manual 2nd. ed. Cold Spring Harbor Laboratroy Press, New York. (1989).

As discussed previously, a reduced activity and/or expression of a miRNA may be correlated with a disease, condition *or state*. For example, cancerous cells often have a reduced level of one or more specific miRNAs. Indeed, for many cancers a reduced level of miRNA activity and/or expression is associated with the progression of a normal cell to a cancerous cell. Thus, the cell in this form of the present invention may be a cancerous cell or a pre-cancerous cell.

Examples of cancerous cells that show a reduced activity and/or expression of a miRNA include colorectal cancer cells, lung cancer cells, thymus cancer cells, bladder cancer cells, breast cancer cells and prostate cancer cells.

Examples of cancerous cells generally include cells associated with the cancers such as bladder cancer, bone cancer, brain tumours, breast cancer, cervical cancer, colorectal cancer including cancer of the colon, rectum, anus, and appendix, cancer of the esophagus, Hodgkin's disease, kidney cancer, cancer of the larynx, leukemia, liver cancer, lung cancer, lymphoma, melanoma, moles and dysplastic nevi, multiple myeloma, muscular cancer, non-Hodgkin's lymphoma, oral cancer, ovarian cancer, cancer of the pancreas, prostate cancer, skin cancer, stomach cancer, testicular cancer, teratoma, thyroid cancer, and cancer of the uterus. The present invention also includes pre-cancerous cells, including for example, pre-cancerous cells associated with the aforementioned cancers.

In one form, the cell in the various forms of the present invention is cancerous cell from a colorectal cancer or colorectal polyp, including a cancerous cell from a colorectal adenocarcinoma or an adenomatous polyp.

However, as discussed above, the cell in the various forms of the present invention may also be for example an embryonic stem (ES) cell or an adult stem cell. In this regard, microRNAs have been identified in the mouse for which expression is repressed as the ES cells differentiate into embryoid bodies and is undetectable in adult organs, indicating that these miRNAs may have a role in the maintenance of the pluripotent cell state and in the regulation of early mammalian development.

Examples of other cells suitable for the various forms of the present invention include haemopoietic cells including haemopoietic precursor cells, adipocytes, chronic lymphocytic B cells, neuronal cells, sperm cells or sperm producing cells, pancreatic endocrine cells including pancreatic islet cells, and virally infected cells including EBV, HIV, Hepatitis and Herpes infected cells.

It will be appreciated that the cell for which development is modulated in the various forms of the present invention may be an isolated cell *in vitro,* or a cell present in a biological system such as a cell in an organ or tissue, or a cell present in an entire organism (eg animal or human subject). In this regard, the term "biological system" is to be understood to mean any multi-cellular system, and includes isolated groups of cells to whole organisms.

Thus, the present invention may be used to modulate the development of a target cell in a biological system.

Accordingly, in another form the present invention provides a method of modulating development of a target cell in a biological system, the method including the step of introducing into a target cell in the biological system a nucleic acid with the capacity to modulate development of the cells in the biological system, the nucleic acid including a target site for binding of a microRNA, wherein the activity and/or concentration of the microRNA in the target cell results in a level of activity and/or concentration of the nucleic acid in the target cell sufficient to modulate the development of the target cell.

In one form, the biological system is an animal or human subject, including an animal or human that is susceptible to, or suffering from, a disease, condition or state associated with altered microRNA activity and/or expression.

The present invention is therefore suitable for preventing and/or treating a disease, condition or state associated with target cells having an altered activity of a microRNA in a subj ect.

Accordingly, in another form the present invention provides a method of preventing and/or treating a disease, condition *or state* associated with target cells in a subject, the method including the step of introducing into cells in the subject a nucleic acid with the capacity to modulate development of a cell, the nucleic acid including a target site for binding of the microRNA, wherein the altered activity of the microRNA in the target cells results in a level of activity of the nucleic acid sufficient to modulate development of the target cells in the subject.

Examples of diseases, conditions or states associated with altered microRNA activity and/or expression in the various forms of the present invention are as previously discussed, including cancers such as colorectal cancer, lung cancer, thymus cancer, bladder cancer, breast cancer and prostate cancer; human B cell chronic lymphocytic leukemia; B cell (Burkitt) Lymphoma; disorders of pancreatic endocrine cells such as diabetes; diseases and conditions associated with virally infection of cells such as EBV, HIV, Hepatitis and Herpes infected cells; 5q-myelodysplastic syndrome (macrocytic anaemia); diseases and conditions associated with haemopoietic dysfunction; autoimmune and inflammatory diseases (eg Crohn's); fragile X mental retardation; Di George syndrome; Wilms tumour; a disease or condition associated with adipocyte dysfunction; a disease that can be treated with embryonic or adult stem cells; and a disease or condition associated with sperm producing cells.

It will be appreciated that an amount of the nucleic acid effective to provide a therapeutic or desired effect will be introduced/administered to the cell, biological system or subject in the various relevant forms of the present invention.

For example, methods for introducing exogenous DNAs into cells are as described in Sambrook, J, Fritsch, E.F. and Maniatis, T. Molecular Cloning: A Laboratory Manual 2nd. ed. Cold Spring Harbor Laboratory Press, New York. (1989).

The present invention may be used to either inhibit the development of target cells by use of a nucleic acid that inhibits the development of target cells, or alternatively, to promote the development of target cells by the use of a nucleic acid that promotes the development of target cells. For example, the present invention may be used to selectively ablate or rescue cells. Methods for determining whether the development of a cell have been inhibited or promoted are known in the art.

Thus, in one form of the present invention the development of a cell may be inhibited.

In this case, the nucleic acid introduced into the cell will have the capacity to inhibit development of the cell. As the nucleic acid will include one or more target sites for binding of one or more miRNAs, a reduction in activity and/or expression of the one or more miRNAs in a cell will result in an increased expression of the nucleic acid (as compared to a similar cell which does not have a reduced activity of the one or more miRNAs) and consequently result in a level of expression of the nucleic acid sufficient to inhibit development of the cell.

Accordingly, in another form the present invention provides a method of inhibiting development of a cell, the cell having a reduced activity and/or concentration of a microRNA, the method including the step of introducing into the cell a nucleic acid with the capacity to inhibit development of the cell, the nucleic acid including a target site for binding of the microRNA, wherein the reduced activity and/or concentration of the microRNA in the cell results in a level of activity and/or concentration of the nucleic acid in the cell sufficient to inhibit development of the cell.

In one form, the nucleic acid with the capacity to inhibit development of a cell is a nucleic acid with cytotoxic or cytostatic activity, or a nucleic acid encoding a suicide gene. In this case, the introduction of such a nucleic acid including one or more target sites for binding of one or more miRNAs into cells will result in an inhibition of development in those cells that have a reduced activity and/or expression of the one or more miRNAs. In cells that do not have a reduced activity and/or expression of the one or more miRNAs, the miRNAs will act to reduce the expression of the nucleic acid sufficiently that cell development is not inhibited, or at least less inhibited than the cells with the reduced activity and/or expression of the miRNA.

Thus, the present invention allows the selective inhibition of cells with a reduced activity and/or expression of a specific miRNA. In one form, the present invention may be used to selectively ablate target cells with a reduced activity and/or expression of a specific miRNA.

Examples of cytotoxic or suicide genes are generally as described in Greco and Dachs (2001) J Cell Physiol. 187(1):22-36 and include herpes simplex thymidine kinase [NC_001798:c48000-46870 (HSV2 genome)][ gi| 9629267], *E. coli* cytosine deaminase [NC_000913: 355395-356678 (*E. coli* genome)][ gi| 49175990], bacterial *E. coli* nitroreductase [NC_000913: c603994-604647][ gi| 49175990, *P. aeruginosa* carboxypeptidase G2 [AE004706.1: 3474-4712], horseradish peroxidase [X57564] [g|16095], and *E. coli* purine nucleoside phosphorylase [U00096.2: 4618906-4619625 (*E. coli* genome)][ gi| 48994873]. It will also be appreciated that active fragments of these genes may be used, or a functional variant may be used.

In one form, the nucleic acid with the capacity to inhibit development of the cell is selected from one of the group consisting of herpes simplex thymidine kinase, a purine nucleoside phosphorylase and a cytosine deaminase.

The nucleotide sequence of the HSV thymidine kinase gene is designated SEQ ID NO. 151.

The nucleotide sequence of *E.coli* cytosine deaminase is designated SEQ ID NO. 152. In this case, it should be noted that the initiation codon is modified from GTG to ATG for mammalian expression constructs.

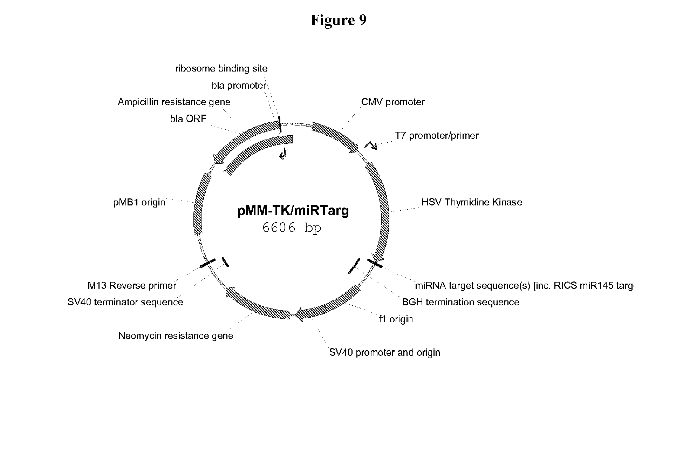

An example of a plasmid encoding the HSV thymidine kinase gene and the miRNA target sequence for miR-145 is plasmid pMM-TK/miR-Targ, which is shown in Figure 9. The nucleotide sequence of this plasmid is designated SEQ ID NO.158.

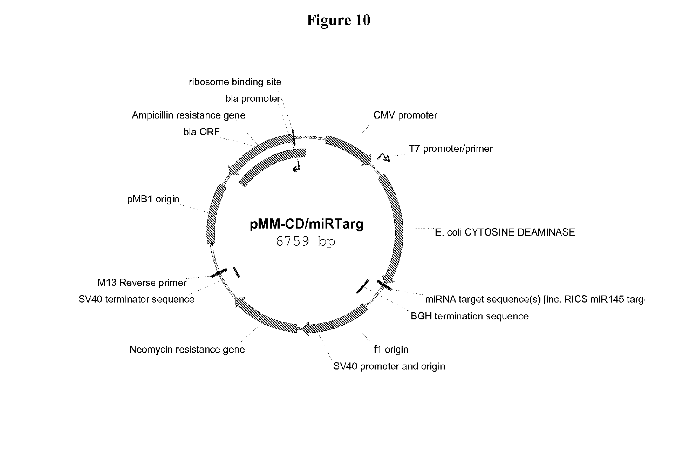

An example of a plasmid encoding the *E.coli* cytosine deaminase and the miRNA target sequence for miR-145 is plasmid pMM-CD/miR-Targ, which is shown in Figure 10. The nucleotide sequence of this plasmid is designated SEQ ID NO.159.

In another form, the present invention allows the development of a cell to be promoted.

In this case, the nucleic acid introduced into the cell will have the capacity to promote development of the cell. As the nucleic acid will include one or more target sites for binding of one or more miRNAs, a reduction in activity and/or expression of the one or more miRNAs will result in an increased expression of the nucleic acid (as compared to a similar cell which does not have a reduced activity of the one or more miRNAs) and consequently result in a level of expression of the nucleic acid sufficient to promote development of the cell.

Accordingly, in another form the present invention provides a method of promoting development of a cell, the cell having a reduced activity and/or concentration of a microRNA, the method including the step of introducing into the cell a nucleic acid with the capacity to promote development of the cell, the nucleic acid including a target site for binding of the microRNA, wherein the reduced activity and/or concentration of the microRNA in the cell results in a level of activity and/or concentration of the nucleic acid in the cell sufficient to promote development of the cell.

Examples of nucleic acids that have the capacity to promote development in a cell include therapeutic genes or a cytokine gene such as granulocyte macrophage-colony stimulating factor (GM-CSF; human: NM_000758), G-CSF (human: NM_000759; NM_172219; NM_172220), Interleukin *11 (human: NM_000641)*, *and Tumour Necrosis Factor alpha (human: NM_000594*). Thus, the nucleic acid may encode a cytokine, a therapeutic protein or an active fragment of a cytokine or a therapeutic protein.

In the case of a cytokine gene, the introduction of a nucleic acid encoding a cytokine into cells will result in a promotion of development in cells that have a reduced activity and/or expression of the one or more miRNAs. In this case, it will be appreciated that the cells will be susceptible to the effects of the cytokine. In cells that do not have a reduced activity and/or expression of the one or more miRNAs, the miRNAs will act to reduce the expression of the nucleic acid sufficiently that cell development is not promoted, or at least less promoted to an extent that is less than the cells with the reduced activity and/or expression of the miRNA.

Thus, the present invention allows the selective promotion of development of target cells with a reduced activity and/or expression of a specific miRNA.

It will be appreciated, that the expression of the nucleic acid with the capacity to modulate development of a cell will require various regulatory elements known in the art for the expression of the inserted nucleic acids in particular cell types, such as promoters for driving the expression of an inserted nucleic acid in a particular cell, poly A signals for efficient polyadenylation of mRNA transcribed from inserted nucleic acids, or other regulatory elements to control translation, transcription or mRNA stability.

Depending upon the cell type to be modulated, the promoter driving the expression may be a constitutive promoter, an inducible promoter or a cell or tissue specific promoter. Constitutive mammalian promoters include hypoxanthine phosphoribosyl transferase (HPTR), adenosine deaminase, pyruvate kinase, phosphoglycerate kinase (which has intrinsic bi-directional activity) and β-actin. Exemplary viral promoters which function constitutively in eukaryotic cells include promoters from the simian virus, papilloma virus, adenovirus, human immunodeficiency virus (HIV), Rous sarcoma virus, cytomegalovirus, the long terminal repeats (LTR) of moloney leukemia virus and other retroviruses, and the thymidine kinase promoter of herpes simplex virus.

Inducible promoters include synthetic promoters regulated by the TetO/TetR system and inducible promoters such as metallothionein promoter, which may be used to induce transcription in the presence of certain metal ions. Other inducible promoters are known in the art.

The tissue-specific promoter will depend upon the particular cell type. For example, promoters that allow expression in colon cancer cells include the regulatory sequences of human carcinoembryonic antigen (CEA) [accession:U17131; gi|967132].

Examples of microRNAs that are correlated with human cancer, or the progression of a normal cell to a cancerous cell in humans, are as follows (5' to 3'):
hsa-1et-7a-1 pre-miRNA precursor: 
ugggaugagguaguagguuguauaguuuuagggucacacccaccacugggagauaacuauacaaucuacugucu
uuccua (SEQ ID NO.1)
hsa-let-7 mature miRNA:
   ugagguaguagguuguauaguu (SEQ ID NO.2)
hsa-let-7a-2 pre-miRNA precursor: 
agguugagguaguagguuguauaguuuagaauuacaucaagggagauaacuguacagccu
ccuagcuuuccu (SEQ ID NO.3)
hsa-let-7a mature miRNA:
   ugagguaguagguuguauaguu (SEQ ID NO.4)
hsa-let-7a-3 pre-miRNA precursor: 
gggugagguaguagguuguauaguuuggggcucugcccugcuaugggauaacuauacaaucuacugucuuucc
u (SEQ ID NO.5)
hsa-let-7a mature miRNA:
   ugagguaguagguuguauaguu (SEQ ID NO.6)
hsa-let-7b pre-miRNA precursor: 
cggggugagguaguagguugugugguuucagggcagugauguugccccucggaagauaacuauacaaccuacug
ccuucccug (SEQ ID NO.7)
hsa-let-7b mature miRNA:
   ugagguaguagguugugugguu (SEQ ID NO.8)
hsa-let-7c pre-miRNA precursor: 
gcauccggguugagguaguagguuguaugguuuagaguuacacccugggaguuaacuguacaaccuucuagcuu
uccuuggagc (SEQ ID NO.9)
hsa-let-7c mature miRNA:
   ugagguaguagguuguaugguu (SEQ ID NO.10)
hsa-1et-7f-1 pre-miRNA precursor: 
ucagagugagguaguagauuguauaguugugggguagugauuuuacccuguucaggagauaacuauacaaucua
uugccuucccuga (SEQ ID NO.11)
hsa-let-7f mature miRNA:
   ugagguaguagauuguauaguu (SEQ ID NO.12)
hsa-let-7f-2 pre-miRNA precursor: 
ugugggaugagguaguagauuguauaguuuuagggucauaccccaucuuggagauaacuauacagucuacuguc
uuucccacg (SEQ ID NO.13)
hsa-let-7f mature miRNA:
   ugagguaguagauuguauaguu (SEQ ID NO.14)
hsa-mir-10b pre-miRNA precursor: 
ccagagguuguaacguugucuauauauacccuguagaaccgaauuuguggguauccguauagucacagauucg
auucuaggggaauauauggucgaugcaaaaacuuca (SEQ ID NO.15)
hsa-miR-10b mature miRNA:
   uacccuguagaaccgaauuugu (SEQ ID NO.16)
hsa-mir-15b pre-miRNA precursor: 
uugaggccuuaaaguacuguagcagcacaucaugguuuacaugcuacagucaagaugcgaaucauuauuugcug
cucuagaaauuuaaggaaauucau (SEQ ID NO.17)
hsa-miR-15b mature miRNA:
   uagcagcacaucaugguuuaca (SEQ ID NO.18)
hsa-mir-16-1 pre-miRNA precursor: 
gucagcagugccuuagcagcacguaaauauuggcguuaagauucuaaaauuaucuccaguauuaacugugcugc
ugaaguaagguugac (SEQ ID NO.19)
hsa-miR-16 mature miRNA:
   uagcagcacguaaauauuggcg (SEQ ID NO.20)
hsa-mir-16-2 pre-miRNA precursor: 
guuccacucuagcagcacguaaauauuggcguagugaaauauauuaaacaccaauauuacugugcugcuuuag
ugugac (SEQ ID NO.21)
hsa-miR-16 mature miRNA:
   uagcagcacguaaauauuggcg (SEQ ID NO.22)
hsa-mir-19b-1 pre-miRNA precursor: 
cacuguucuaugguuaguuuugcagguuugcauccagcugugugauauucugcugugcaaauccaugcaaaacu
gacugugguagug (SEQ ID NO.23)
hsa-miR-19b mature miRNA:
   ugugcaaauccaugcaaaacuga (SEQ ID NO.24)
hsa-mir-19b-2 pre-miRNA precursor: 
acauugcuacuuacaauuaguuuugcagguuugcauuucagcguauauauguauauguggcugugcaaauccau
gcaaaacugauugugauaaugu (SEQ ID NO.25)
hsa-miR-19b mature miRNA:
   ugugcaaauccaugcaaaacuga (SEQ ID NO.26)
hsa-mir-20 pre-miRNA precursor:
   guagcacuaaagugcuuauagugcagguaguguuuaguuaucuacugcauuaugagcacuuaaaguacugc (SEQ ID NO.27)
hsa-miR-20 mature miRNA:
   uaaagugcuuauagugcagguag (SEQ ID NO.28)
hsa-mir-21 pre-miRNA precursor:
   ugucggguagcuuaucagacugauguugacuguugaaucucauggcaacaccagucgaugggcugucugaca (SEQ ID NO.29)
hsa-miR-21 mature miRNA:
   uagcuuaucagacugauguuga (SEQ ID NO.30)
hsa-mir-22 pre-miRNA precursor: 
ggcugagccgcaguaguucuucaguggcaagcuuuauguccugacccagcuaaagcugccaguugaagaacugu
ugcccucugcc (SEQ ID NO.31)
hsa-miR-22 mature miRNA:
   aagcugccaguugaagaacugu (SEQ ID NO.32)
hsa-mir-23a pre-miRNA precursor:
   ggccggcugggguuccuggggaugggauuugcuuccugucacaaaucacauugccagggauuuccaaccgacc (SEQ ID NO.33)
hsa-miR-23a mature miRNA:
   aucacauugccagggauuucc (SEQ ID NO.34)
hsa-mir-24-1 pre-miRNA precursor:
   cuccggugccuacugagcugauaucaguucucauuuuacacacuggcucaguucagcaggaacaggag (SEQ ID NO.35)
hsa-miR-24 mature miRNA:

   uggcucaguucagcaggaacag (SEQ ID NO.36)
hsa-miR-189 mature miRNA:

   gugccuacugagcugauaucagu (SEQ ID NO.37)
hsa-mir-24-2 pre-miRNA precursor:
   cucugccucccgugccuacugagcugaaacacaguugguuuguguacacuggcucaguucagcaggaacaggg (SEQ ID NO.38)
hsa-miR-24 mature miRNA:
   uggcucaguucagcaggaacag (SEQ ID NO.39)
hsa-mir-26a-1 pre-miRNA 
guggccucguucaaguaauccaggauaggcugugcaggucccaaugggccuauucuugguuacuugcacgggga
cgc (SEQ ID NO.40)
hsa-miR-26 mature miRNA:
   auucaaguaauccaggauaggc (SEQ ID NO.41)
hsa-mir-26b pre-miRNA precursor: 
ccgggacccaguucaaguaauucaggauagguugugugcuguccagccuguucuccauuacuuggcucggggac
cgg (SEQ ID NO.42)
hsa-miR-26b mature miRNA:
   uucaaguaauucaggauagguu (SEQ ID NO.43)
hsa-mir-26a-2 pre-miRNA precursor: 
ggcuguggcuggauucaaguaauccaggauaggcuguuuccaucugugaggccuauucuugauuacuuguuuc
uggaggcagcu (SEQ ID NO.44)
hsa-miR-26a mature miRNA:
   uucaaguaauccaggauaggc (SEQ ID NO.45)
hsa-mir-27b pre-miRNA precursor: 
accucucuaacaaggugcagagcuuagcugauuggugaacagugauugguuuccgcuuuguucacaguggcuaa
guucugcaccugaagagaaggug (SEQ ID NO.46)
hsa-miR-27b mature miRNA:
   uucacaguggcuaaguucugc (SEQ ID NO.47)
hsa-mir-29a pre-miRNA precursor:
   augacugauuucuuuugguguucagagucaauauaauuuucuagcaccaucugaaaucgguuau (SEQ ID NO.48)
hsa-miR-29a mature miRNA:
   uagcaccaucugaaaucgguu (SEQ ID NO.49)
hsa-mir-30a pre-miRNA precursor:
   gcgacuguaaacauccucgacuggaagcugugaagccacagaugggcuuucagucggauguuugcagcugc (SEQ ID NO.50)
hsa-miR-30a-3p mature miRNA:
   cuuucagucggauguuugcagc (SEQ ID NO.51)
hsa-miR-30a-5p mature miRNA:
   uguaaacauccucgacuggaag (SEQ ID NO.52)
hsa-mir-141 pre-miRNA precursor: 
cggccggcccugggguccaucuuccaguacaguguuggauggucuaauugugaagcuccuaacacugucugguaa
agauggcucccggguggguuc (SEQ ID NO.53)
hsa-miR-141 mature miRNA:
   uaacacugucugguaaagaugg (SEQ ID NO.54)
hsa-mir-142 pre-miRNA precursor: 
gacagugcagucacccauaaaguagaaagcacuacuaacagcacuggaggguaguguuuccuacuuuauggau
gaguguacugug (SEQ ID NO.55)
hsa-miR-142-5p mature miRNA:
   cauaaaguagaaagcacuac (SEQ ID NO.56)
hsa-miR-142-3p mature miRNA:
   uguaguguuuccuacuuuaugga (SEQ ID NO.57)
hsa-mir-143 pre-miRNA precursor: 
gcgcagcgcccugucucccagccugaggugcagugcugcaucucuggucaguugggagucugagaugaagcacu
guagcucaggaagagagaaguuguucugcagc (SEQ ID NO.58)
hsa-miR-143 mature miRNA:
   ugagaugaagcacuguagcuca (SEQ ID NO.59)
hsa-mir-145 pre-miRNA precursor: 
caccuuguccucacgguccaguuuucccaggaaucccuuagaugcuaagauggggauuccuggaaauacuguuc
uugaggucaugguu (SEQ ID NO.60)
hsa-miR-145 mature miRNA:
   guccaguuuucccaggaaucccuu (SEQ ID NO.61)
hsa-mir-192 pre-miRNA precursor: 
gccgagaccgagugcacagggcucugaccuaugaauugacagccagugcucucgucuccccucuggcugccaauu
ccauaggucacagguauguucgccucaaugccagc (SEQ ID NO.62)
hsa-miR-192 mature miRNA:
   cugaccuaugaauugacagcc (SEQ ID NO.63)
hsa-mir-194-1 pre-miRNA precursor: 
augguguuaucaaguguaacagcaacuccauguggacuguguaccaauuuccaguggagaugcuguuacuuuug
augguuaccaa (SEQ ID NO.64)
hsa-miR-194 mature miRNA:
   uguaacagcaacuccaugugga (SEQ ID NO.65)
hsa-mir-194-2 pre-miRNA precursor: 
ugguucccgcccccuguaacagcaacuccauguggaagugcccacugguuccaguggggcugcuguuaucuggg
gcgagggccag (SEQ ID NO.66)
hsa-miR-194 mature miRNA:
   uguaacagcaacuccaugugga (SEQ ID NO.67)
hsa-mir-199b pre-miRNA precursor: 
ccagaggacaccuccacuccgucuacccaguguuuagacuaucuguucaggacucccaaauuguacaguagucug
cacauugguuaggcugggcugguuagacccucgg (SEQ ID NO.68)
hsa-miR-199b mature miRNA:
   cccaguguuuagacuaucuguuc (SEQ ID NO.69)
hsa-mir-200b pre-miRNA precursor: 
ccagcucgggcagccguggccaucuuacugggcagcauuggauggagucaggucucuaauacugccugguaaug
augacggcggagcccugcacg (SEQ ID NO.70)
hsa-miR-200b mature miRNA:
   uaauacugccugguaaugaugac (SEQ ID NO.71)
hsa-mir-200c pre-miRNA precursor:
   cccucgucuuacccagcaguguuugggugcgguugggagucucuaauacugccggguaaugauggagg (SEQ ID NO.72)
hsa-miR-200c mature miRNA:
   uaauacugccggguaaugaugg (SEQ ID NO.73)
hsa-mir-320 pre-miRNA precursor: 
gcuucgcuccccuccgccuucucuucccgguucuucccggagucgggaaaagcuggguugagagggcgaaaaag
gaugaggu (SEQ ID NO.74)
hsa-miR-320 mature miRNA:
   aaaagcuggguugagagggcgaa (SEQ ID NO.75)
hsa-miR-321 mature miRNA:
   uaagccagggauuguggguuc (SEQ ID NO.76)
hsa-mir-30a pre-miRNA precursor:
   gcgacuguaaacauccucgacuggaagcugugaagccacagaugggcuuucagucggauguuugcagcugc (SEQ ID NO.77)
hsa-miR-30a-3p mature miRNA:
   cuuucagucggauguuugcagc (SEQ ID NO.78)
hsa-miR-30a-5p mature miRNA:
   uguaaacauccucgacuggaag (SEQ ID NO.79)
hsa-mir-29b-1 pre-miRNA precursor: 
cuucaggaagcugguuucauauggugguuuagauuuaaauagugauugucuagcaccauuugaaaucaguguuc
uuggggg (SEQ ID NO.80)
hsa-miR-29b mature miRNA:
   uagcaccauuugaaaucaguguu (SEQ ID NO.81)
hsa-mir-125b-1 pre-miRNA precursor: 
ugcgcuccucucagucccugagacccuaacuugugauguuuaccguuuaaauccacggguuaggcucuugggag
cugcgagucgugcu (SEQ ID NO.82)
hsa-miR-125b mature miRNA:
   ucccugagacccuaacuuguga (SEQ ID NO.83)
hsa-mir-125a pre-miRNA precursor: 
ugccagucucuaggucccugagacccuuuaaccugugaggacauccagggucacaggugagguucuugggagcc
uggcgucuggcc (SEQ ID NO.84)
hsa-miR-125a mature miRNA:
   ucccugagacccuuuaaccugug (SEQ ID NO.85)
hsa-mir-125b-2 pre-miRNA precursor: 
accagacuuuuccuagucccugagacccuaacuugugagguauuuuaguaacaucacaagucaggcucuugggac
cuaggcggaggga (SEQ ID NO.86)
hsa-miR-125b mature miRNA:
   ucccugagacccuaacuuguga (SEQ ID NO.87)
hsa-mir-15a pre-miRNA precursor: 
ccuuggaguaaaguagcagcacauaaugguuuguggauuuugaaaaggugcaggccauauugugcugccucaaa
aauacaagg (SEQ ID NO.88)
hsa-miR-15a mature miRNA:
   uagcagcacauaaugguuugug (SEQ ID NO.89)
hsa-mir-126 pre-miRNA precursor: 
cgcuggcgacgggacauuauuacuuuugguacgcgcugugacacuucaaacucguaccgugaguaauaaugcgc
cguccacggca (SEQ ID NO.90)
hsa-miR-126* mature miRNA:
   cauuauuacuuuugguacgcg (SEQ ID NO.91) hsa-miR-126 mature miRNA:
   ucguaccgugaguaauaaugc (SEQ ID NO.92)
hsa-mir-188 pre-miRNA precursor: 
ugcucccucucucacaucccuugcauggugagggugagcuuucugaaaaccccucccac
augcaggguuugcaggauggcgagcc (SEQ ID NO.93)
hsa-miR-188 mature miRNA:
   caucccuugcaugguggagggu (SEQ ID NO.94)
hsa-mir-331 pre-miRNA precursor: 
gaguuugguuuuguuugguuuguucuagguaugguccccagggaucccagaucaaaccag
gccccugggccuauccuagaaccaaccuaagcuc (SEQ ID NO.95)
hsa-miR-331 mature miRNA:
   gccccugggccuauccuagaa (SEQ ID NO.96)
hsa-mir-155 pre-miRNA precursor: 
cuguuaaugcuaaucgugauagggguuuuugccuccaacugacuccuacauauuagcauu
aacag (SEQ ID NO.97)
hsa-miR-155 mature miRNA:
   uuaaugcuaaucgugauagggg (SEQ ID NO.98)

An example of a microRNA that is associated with the regulation of insulin secretion is hsa-miR-375:
hsa-mir-375 pre-miRNA precursor:
   ccccgcgacgagccccucgcacaaaccggaccugagcguuuuguucguucggcucgcgugaggc (SEQ ID NO. 160)
hsa-miR-375 mature miRNA:
   uuuguucguucggcucgcguga (SEQ ID NO.161)

Based on experiments in mice, hsa-miR-181b-1 is likley to be involved in haemopoiesis (B lineage cell differentiation):
hsa-mir-181b-1 pre-miRNA precursor: 
ccugugcagagauuauuuuuuaaaaggucacaaucaacauucauugcugucgguggguugaacuguguggacaa
gcucacugaacaaugaaugcaacuguggccccgcuu (SEQ ID NO. 162)
hsa-miR-181b mature miRNA:
   aacauucauugcugucgguggg (SEQ ID NO. 163)
hsa-mir-124a-3 pre-miRNA precursor: 
UGAGGGCCCCUCUGCGUGUUCACAGCGGACCUUGAUUUAAUGUCUAUACA
AUUAAGGCACGCGGUGAAUGCCAAGAGAGGCGCCUCC (SEQ ID NO. 166)
hsa-miR-124a mature miRNA:
   UUAAGGCACGCGGUGAAUGCCA (SEQ ID NO. 167)
hsa-mir-9-2 pre-miRNA precursor: 
GGAAGCGAGUUGUUAUCUUUGGUUAUCUAGCUGUAUGAGUGUAUUGGUC
UUCAUAAAGCUAGAUAACCGAAAGUAAAAACUCCUUCA (SEQ ID NO. 168)
hsa-miR-9 mature miRNA:
   UCUUUGGUUAUCUAGCUGUAUGA (SEQ ID NO. 169)

The present invention also provides orthologues of the above human miRNAs, which may identified by method known in the art. A database of miRNAs is found at the miRBase registry (http://microrna.sanger.ac.uk/sequences/).

Thus, the present invention specifically provides in its various forms the following microRNAs: hsa-let-7a-1, hsa-let-7a-2, hsa-let-7a-3, hsa-let-7b, hsa-let-7c, hsa-let-7f, hsa-miR-10b, hsa-miR-15a, hsa-miR-15b, hsa-miR-16, hsa-miR-19b mature miRNA, hsa-miR-20, hsa-miR-21, hsa-miR-22, hsa-miR-23a, hsa-miR-24, hsa-miR-189, hsa-miR-24, hsa-miR-26, hsa-miR-26b, hsa-miR-26a, hsa-miR-27b, hsa-miR-29a, hsa-miR-30a-3p, hsa-miR-141, hsa-miR-142-5p, hsa-miR-142-3p, hsa-miR-143, hsa-miR-145, hsa-miR-192, hsa-miR-194, hsa-miR-199b, hsa-miR-200b, hsa-miR-200c, hsa-miR-320, hsa-miR-321, hsa-miR-30a-3p, hsa-miR-30a-5p, hsa-miR-29b, hsa-miR-125b, hsa-miR-125a, hsa-miR-125b, hsa-miR-126*, hsa-miR-126, hsa-miR-188, hsa-miR-331, hsa-miR-155, hsa-miR-181b-1, hsa-miR-124a, hsa-miR-9 and corresponding orthologues from other species.

Methods for identifying a corresponding orthologue are known in the art, such as Weber MJ. (2005) New human and mouse microRNA genes found by homology search. FEBS J. 272(1):59-73.

As described previously, miRNAs are small RNA molecules endogenously encoded in the genome of many species that regulate gene expression by binding to specific mRNAs. miRNAs are formed from larger transcripts that fold to produce hairpin structures and serve as substrates for the Dicer family of RNase III enzymes. The products of Dicer cleavage are short dsRNA molecules, one strand of which is retained in a ribonucleoprotein complex called the RNA-induced silencing complex (RISC). The retained RNA acts as a guide to direct this complex to a target site in the mRNA which is then inactivated either by cleavage or translational interference, depending on the degree of complementarity between the miRNA and its target site.

Accordingly, the modulation of the development of a cell in the various forms of the present invention may be by way of cleavage (or lack of cleavage) of the nucleic acid with the capacity to modulate the development of the cell, and/or by way of translational interference (or lack of translational interference) of the nucleic acid with the capacity to modulate the development of the cell.

The target site in the various forms of the present invention may be a nucleotide sequence that has exact complementarity to the miRNA, or alternatively, be a target site with a reduced degree of complementarity. Methods for determining the extent of complementarity required for binding of a miRNA (so as to cleave the target or translationally interfere with the target) are known in the art, for example Lewis BP, Burge CB, Bartel DP., (2005) Conserved seed pairing, often flanked by adenosines, indicates that thousands of human genes are microRNA targets. Cell 120(1): 15-20 and Saetrom O, Snove O Jr, Saetrom P., (2005) Weighted sequence motifs as an improved seeding step in microRNA target prediction algorithms. RNA 11.(7):995-1003.

The target site may be a natural target site or a non-naturally occurring target site. In the case of a target site occurring in a gene, it will be appreciated that the target site may be introduced into the nucleic acid with one or more addition nucleotides from the gene. For example, the target site may be introduced by way of using the entire 3'UTR of a gene having a suitable target site in that untranslated region of the mRNA.

The ability of a miRNA to bind to a target site and cleave the mRNA and/or interfere with translation may be confirmed experimentally by a suitable method known in the art. For example, the psiCHECK™2 Luciferase assay system (Promega) can be used to determine miRNA activity both *in vitro* and *in vivo.* Northern blot and RT-PCR analyses can also be used to determine cleavage of a target transcript, while Western blot analysis will detect reduced translation of encoded proteins.

In the case of the miRNAs discussed previously herein, complementary target sites for the binding of the miRNAs are as follows (5' to 3'):
hsa-let-7 mature miRNA target site:
   AACUAUACAACCUACUACCUCA (SEQ ID NO. 99)
hsa-let-7a mature miRNA target site:
   AACUAUACAACCUACUACCUCA (SEQ ID NO. 100)
hsa-let-7a mature miRNA target site:
   AACUAUACAACCUACUACCUCA (SEQ ID NO. 101)
hsa-let-7b mature miRNA target site:
   AACCACACAACCUACUACCUCA (SEQ ID NO. 102)
hsa-let-7c mature miRNA target site:
   AACCAUACAACCUACUACCUCA (SEQ ID NO. 103)
hsa-let-7f mature miRNA target site:
   AACUAUACAAUCUACUACCUCA (SEQ ID NO. 104)
hsa-let-7f mature miRNA target site:
   AACUAUACAAUCUACUACCUCA (SEQ ID NO. 105)
hsa-miR-10b mature miRNA target site:
   ACAAAUUCGGUUCUACAGGGUA (SEQ ID NO. 106)
hsa-miR-15b mature miRNA target site:
   UGUAAACCAUGAUGUGCUGCUA (SEQ ID NO. 107)
hsa-miR-16 mature miRNA target site:
   CGCCAAUAUUUACGUGCUGCUA (SEQ ID NO. 108)
hsa-miR-16 mature miRNA target site:
   CGCCAAUAUUUACGUGCUGCUA (SEQ ID NO. 109)
hsa-miR-19b mature miRNA target site:
   UCAGUUUUGCAUGGAUUUGCACA (SEQ ID NO. 110)
hsa-miR-19b mature miRNA target site:
   UCAGUUUUGCAUGGAUUUGCACA (SEQ ID NO. 111)
hsa-miR-20 mature miRNA target site:
   CUACCUGCACUAUAAGCACUUUA (SEQ ID NO. 112)
hsa-miR-21 mature miRNA target site:
   UCAACAUCAGUCUGAUAAGCUA (SEQ ID NO. 113)
hsa-miR-22 mature miRNA target site:
   ACAGUUCUUCAACUGGCAGCUU (SEQ ID NO. 114)
hsa-miR-23a mature miRNA target site:
   GGAAAUCCCUGGCAAUGUGAU (SEQ ID NO. 115)
hsa-miR-24 mature miRNA target site:
   CUGUUCCUGCUGAACUGAGCCA (SEQ ID NO. 116)
hsa-miR-189 mature miRNA target site:
   ACUGAUAUCAGCUCAGUAGGCAC (SEQ ID NO. 117)
hsa-miR-24 mature miRNA target site:
   CUGUUCCUGCUGAACUGAGCCA (SEQ ID NO. 118)
hsa-miR-26 mature miRNA target site:
   GCCUAUCCUGGAUUACUUGAAU (SEQ ID NO. 119)
hsa-miR-26b mature miRNA target site:
   AACCUAUCCUGAAUUACUUGAA (SEQ ID NO. 120)
hsa-miR-26a mature miRNA target site:
   GCCUAUCCUGGAUUACUUGAA (SEQ ID NO. 121)
hsa-miR-27b mature miRNA target site:
   GCAGAACUUAGCCACUGUGAA (SEQ ID NO. 122)
hsa-miR-29a mature miRNA target site:
   AACCGAUUUCAGAUGGUGCUA (SEQ ID NO. 123)
hsa-miR-30a-3p mature miRNA target site:
   GCUGCAAACAUCCGACUGAAAG (SEQ ID NO. 124)
hsa-miR-30a-5p mature miRNA target site:
   CUUCCAGUCGAGGAUGUUUACA (SEQ ID NO. 125)
hsa-miR-141 mature miRNA target site:
   CCAUCUUUACCAGACAGUGUUA (SEQ ID NO. 126)
hsa-miR-142-5p mature miRNA target site:
   GUAGUGCUUUCUACUUUAUG (SEQ ID NO. 127)
hsa-miR-142-3p mature miRNA:
   UCCAUAAAGUAGGAAACACUACA (SEQ ID NO. 128)
hsa-miR-143 mature miRNA target site:
   UGAGCUACAGUGCUUCAUCUCA (SEQ ID NO. 129)
hsa-miR-145 mature miRNA target site:
   AAGGGAUUCCUGGGAAAACUGGAC (SEQ ID NO. 130)
hsa-miR-192 mature miRNA target site:
   GGCUGUCAAUUCAUAGGUCAG (SEQ ID NO. 131)
hsa-miR-194 mature miRNA target site:
   UCCACAUGGAGUUGCUGUUACA (SEQ ID NO. 132)
hsa-miR-194 mature miRNA target site:
   UCCACAUGGAGUUGCUGUUACA (SEQ ID NO. 133)
hsa-miR-199b mature miRNA target site:
   GAACAGAUAGUCUAAACACUGGG (SEQ ID NO. 134)
hsa-miR-200b mature miRNA target site:
   GUCAUCAUUACCAGGCAGUAUUA (SEQ ID NO. 135)
hsa-miR-200c mature miRNA target site:
   CCAUCAUUACCCGGCAGUAUUA (SEQ ID NO. 136)
hsa-miR-320 mature miRNA target site:
   UUCGCCCUCUCAACCCAGCUUUU (SEQ ID NO. 137)
hsa-miR-321 mature miRNA target site:
   GAACCCACAAUCCCUGGCUUA (SEQ ID NO. 138)
hsa-miR-30a-3p mature miRNA target site:
   GCUGCAAACAUCCGACUGAAAG (SEQ ID NO. 139)
hsa-miR-30a-5p mature miRNA target site:
   CUUCCAGUCGAGGAUGUUUACA (SEQ ID NO. 140)
hsa-miR-29b mature miRNA target site:
   AACACUGAUUUCAAAUGGUGCUA (SEQ ID NO. 141)
hsa-miR-125b mature miRNA target site:
   UCACAAGUUAGGGUCUCAGGGA (SEQ ID NO. 142)
hsa-miR-125a mature miRNA target site:
   CACAGGUUAAAGGGUCUCAGGGA (SEQ ID NO. 143)
hsa-miR-125b mature miRNA target site:
   UCACAAGUUAGGGUCUCAGGGA (SEQ ID NO. 144)
hsa-miR-15a mature miRNA target site:
   CACAAACCAUUAUGUGCUGCUA (SEQ ID NO. 145)
hsa-miR-126* mature miRNA target site:
   CGCGUACCAAAAGUAAUAAUG (SEQ ID NO. 146)
hsa-miR-126 mature miRNA target site:
   GCAUUAUUACUCACGGUACGA (SEQ ID NO. 147)
hsa-miR-188 mature miRNA target site:
   ACCCUCCACCAUGCAAGGGAUG (SEQ ID NO. 148)
hsa-miR-331 mature miRNA target site:
   UUCUAGGAUAGGCCCAGGGGC (SEQ ID NO. 149)
hsa-miR-155 mature miRNA target site:
   CCCCUAUCACGAUUAGCAUUAA (SEQ ID NO. 150)
hsa-miR-375 mature miRNA target site:
   UCACGCGAGCCGAACGAACAAA (SEQ ID NO.164)
hsa-miR-181b mature miRNA target site:
   CCCACCGACAGCAAUGAAUGUU (SEQ ID NO. 165)
hsa-miR-124a mature miRNA target site:
   UGGCAUUCACCGCGUGCCUUAA (SEQ ID NO. 170)
hsa-miR-9 mature miRNA target site:
   UCAUACAGCUAGAUAACCAAAGA (SEQ ID NO. 171)

In the case of the nucleic acid molecule being a DNA, a person skilled in the art will appreciate that the above target sequences will have the U bases substituted for T bases. The target site for binding of a microRNA in the various forms of the present invention may be a non-naturally occurring binding site for the particular miRNA, or alternatively, may be a target site present in a naturally occurring gene or mRNA, such as a target site found in the 3'UTR of a gene. In this regard, naturally and non-naturally occurring targets for a microRNA may be identified as described in Krek et al. (2005) Nature Genetics 37(5): 495-500.

For example, in the case of the miR-143 miRNA, Table 1 provides a listing of human mRNAs predicted to contain target sites for hsa-miR-143, using the method as described in Krek et al. (2005) Nature Genetics 37(5): 495-500, using default parameters.

**Table 1**

| **Rank** | **human Refseq Id** | **PicTar score** | **annotation** |
|---|---|---|---|
| 1 | NM_138962 | 8.75 | Homo sapiens musashi homolog 2 (Drosophila) (MSI2), transcript variant 1, mRNA. |
| 2 | NM_033389 | 6.92 | Homo sapiens slingshot homolog 2 (Drosophila) (SSH2), mRNA. |
| 3 | NM_004720 | 6.71 | Homo sapiens endothelial differentiation, lysophosphatidic acid G-protein-coupled receptor, 4 (EDG4), mRNA. |
| 4 | NM_203445 | 6.71 | Homo sapiens ATP-binding cassette, sub-family B (MDR/TAP), member 9 (ABCB9), transcript variant 3, mRNA. |
| 5 | NM_004738 | 6.67 | Homo sapiens VAMP (vesicle-associated membrane protein)-associated protein B and C (VAPB), mRNA. |
| 6 | NM_006517 | 6.47 | Homo sapiens solute carrier family 16 (monocarboxylic acid transporters), member 2 (SLC16A2), mRNA. |
| 7 | NM_005104 | 6.27 | Homo sapiens bromodomain containing 2 (BRD2), mRNA. |
| 8 | NM_198452 | 6.23 | Homo sapiens pregnancy upregulated non-ubiquitously expressed CaM kinase (PNCK), mRNA. |
| 9 | NM_015575 | 5.42 | Homo sapiens trinucleotide repeat containing 15 (TNRC15), mRNA. |
| 10 | NM_005644 | 5.26 | Homo sapiens TAF12 RNA polymerase II, TATA box binding protein (TBP)-associated factor, 20kDa (TAF12), mRNA. |
| 11 | NM_004985 | 5.1 | Homo sapiens v-Ki-ras2 Kirsten rat sarcoma 2 viral oncogene homolog (KRAS2), transcript variant b, mRNA. |
| 12 | NM_033360 | 5.1 | Homo sapiens v-Ki-ras2 Kirsten rat sarcoma 2 viral oncogene homolog (KRAS2), transcript variant a, mRNA. |
| 13 | NM_007306 | 5.06 | Homo sapiens breast cancer 1, early onset (BRCA1), transcript variant BRCA1-exon4, mRNA. |
| 14 | NM_144633 | 5.05 | Homo sapiens potassium voltage-gated channel, subfamily H (eag-related), member 8 (KCNH8), mRNA. |
| 15 | NM_153649 | 4.98 | Homo sapiens tropomyosin 3 (TPM3), mRNA. |
| 16 | NM_004798 | 4.78 | Homo sapiens kinesin family member 3B (KIF3B), mRNA. |
| 17 | NM_021907 | 4.69 | Homo sapiens dystrobrevin, beta (DTNB), transcript variant 1, mRNA. |
| 18 | NM_183360 | 4.69 | Homo sapiens dystrobrevin, beta (DTNB), transcript variant 4, mRNA. |
| 19 | NM_033148 | 4.69 | Homo sapiens dystrobrevin, beta (DTNB), transcript variant 3, mRNA. |
| 20 | NM_183361 | 4.69 | Homo sapiens dystrobrevin, beta (DTNB), transcript variant 5, mRNA. |
| 21 | NM_033147 | 4.69 | Homo sapiens dystrobrevin, beta (DTNB), transcript variant 2, mRNA. |
| 22 | NM_014112 | 4.56 | Homo sapiens trichorhinophalangeal syndrome I (TRPS1), mRNA. |
| 23 | NM_014502 | 4.51 | Homo sapiens PRP19/PS04 homolog (S. cerevisiae) (PRP19), mRNA. |
| 24 | NM_004210 | 4.48 | Homo sapiens neuralized-like (Drosophila) (NEURL), mRNA. |
| 25 | NM_003284 | 4.46 | Homo sapiens transition protein 1 (during histone to protamine replacement) (TNP1), mRNA. |
| 26 | NM_001282 | 4.42 | Homo sapiens adaptor-related protein complex 2, beta 1 subunit (AP2B1), mRNA. |
| 27 | NM_007200 | 4.41 | Homo sapiens A kinase (PRKA) anchor protein 13 (AKAP13), transcript variant 2, mRNA. |
| 28 | NM_144767 | 4.41 | Homo sapiens A kinase (PRKA) anchor protein 13 (AKAP13), transcript variant 3, mRNA. |
| 29 | NM_006738 | 4.41 | Homo sapiens A kinase (PRKA) anchor protein 13 (AKAP13), transcript variant 1, mRNA. |
| 30 | NM_182901 | 4.29 | Homo sapiens chromosome 11 open reading frame 17 (C11orf17), transcript variant 1, mRNA. |
| 31 | NM_016018 | 4.27 | Homo sapiens CGI-72 protein (CGI-72), transcript variant 1, mRNA. |
| 32 | NM_018013 | 4.23 | Homo sapiens hypothetical protein FLJ10159 (FLJ10159), mRNA. |
| 33 | NM_006302 | 4.23 | Homo sapiens glucosidase I (GCS1), mRNA. |
| 34 | NM_024915 | 4.17 | Homo sapiens transcription factor CP2-like 3 (TFCP2L3), mRNA. |
| 35 | NM_000168 | 4.13 | Homo sapiens GLI-Kruppel family member GLI3 (Greig cephalopolysyndactyly syndrome) (GLI3), mRNA. |
| 36 | NM_014450 | 3.99 | Homo sapiens SHP2-interacting transmembrane adaptor protein (SIT), mRNA. |
| 37 | NM_014346 | 3.95 | Homo sapiens chromosome 22 open reading frame 4 (C22orf4), mRNA. |
| 38 | NM_000633 | 3.86 | Homo sapiens B-cell CLL/lymphoma 2 (BCL2), nuclear : gene encoding mitochondrial protein, transcript variant alpha, mRNA. |
| 39 | NM_002314 | 3.86 | Homo sapiens LIM domain kinase 1 (LIMK1), transcript variant 1, mRNA. |
| 40 | NM_173478 | 3.84 | Homo sapiens hypothetical protein FLJ40137 (FLJ40137), mRNA. |
| 41 | NM_030952 | 3.84 | Homo sapiens likely ortholog of rat SNF1/AMP-activated protein kinase (SNARK), mRNA. |
| 42 | NM_018579 | 3.8 | Homo sapiens mitochondrial solute carrier protein (MSCP), mRNA. |
| 43 | NM_004089 | 3.75 | Homo sapiens delta sleep inducing peptide, immunoreactor (DSIPI), transcript variant 2, mRNA. |
| 44 | NM_198057 | 3.75 | Homo sapiens delta sleep inducing peptide, immunoreactor (DSIPI), transcript variant 1, mRNA. |
| 45 | NM_002830 | 3.72 | Homo sapiens protein tyrosine phosphatase, non-receptor type 4 (megakaryocyte) (PTPN4), mRNA. |
| 46 | NM_006206 | 3.68 | Homo sapiens platelet-derived growth factor receptor, alpha polypeptide (PDGFRA), mRNA. |
| 47 | NM_033004 | 3.64 | Homo sapiens NACHT, leucine rich repeat and PYD (pyrin domain) containing 1 (NALP1), transcript variant 1, mRNA. |
| 48 | NM_033007 | 3.64 | Homo sapiens NACHT, leucine rich repeat and PYD (pyrin domain) containing 1 (NALP1), transcript variant 4, mRNA. |
| 49 | NM_001987 | 3.64 | Homo sapiens ets variant gene 6 (TEL oncogene) (ETV6), mRNA. |
| 50 | NM_033006 | 3.64 | Homo sapiens NACHT, leucine rich repeat and PYD (pyrin domain) containing 1 (NALP1), transcript variant 3, mRNA. |
| 51 | NM_014922 | 3.64 | Homo sapiens NACHT, leucine rich repeat and PYD (pyrin domain) containing 1 (NALP1), transcript variant 2, mRNA. |
| 52 | NM_018398 | 3.63 | Homo sapiens calcium channel, voltage-dependent, alpha 2/delta 3 subunit (CACNA2D3), mRNA. |
| 53 | NM_147159 | 3.6 | Homo sapiens opioid receptor, sigma 1 (OPRS1), transcript variant 4, mRNA. |
| 54 | NM_000920 | 3.6 | Homo sapiens pyruvate carboxylase (PC), nuclear gene encoding mitochondrial protein, transcript variant A, mRNA. |
| 55 | NM_022172 | 3.6 | Homo sapiens pyruvate carboxylase (PC), nuclear gene encoding mitochondrial protein, transcript variant 2, mRNA. |
| 56 | NM_005139 | 3.54 | Homo sapiens annexin A3 (ANXA3), mRNA. |
| 57 | NM_152933 | 3.49 | Homo sapiens protein phosphatase, EF hand calcium-binding domain 2 (PPEF2), transcript variant 2, mRNA. |
| 58 | NM_020665 | 3.45 | Homo sapiens transmembrane protein 27 (TMEM27), mRNA. |
| 59 | NM_015513 | 3.44 | Homo sapiens cysteine-rich with EGF-like domains 1 (CRELD1), mRNA. |
| 60 | NM_006357 | 3.41 | Homo sapiens ubiquitin-conjugating enzyme E2E 3 (UBC4/5 homolog, yeast) (UBE2E3), transcript variant 1, mRNA. |
| 61 | NM_182678 | 3.41 | Homo sapiens ubiquitin-conjugating enzyme E2E 3 (UBC4/5 homolog, yeast) (UBE2E3), transcript variant 2, mRNA. |
| 62 | NM_138731 | 3.4 | Homo sapiens mirror-image polydactyly 1 (MIPOL1), mRNA. |
| 63 | NM_052953 | 3.39 | Homo sapiens leucine rich repeat containing 3B (LRRC3B), mRNA. |
| 64 | NM_017896 | 3.39 | Homo sapiens chromosome 20 open reading frame 11 (C20orf11), mRNA. |
| 65 | NM_152410 | 3.39 | Homo sapiens PARK2 co-regulated (PACRG), mRNA. |
| 66 | NM_014892 | 3.36 | Homo sapiens RNA binding motif protein 16 (RBM16), mRNA. |
| 67 | NM_015516 | 3.36 | Homo sapiens likely ortholog of chicken tsukushi (TSK), mRNA. |
| 68 | NM_017699 | 3.35 | Homo sapiens SID1 transmembrane family, member 1 (SIDT1), mRNA. |
| 69 | NM_144709 | 3.35 | Homo sapiens hypothetical protein FLJ32312 (FLJ32312), mRNA. |
| 70 | NM_015509 | 3.32 | Homo sapiens DKFZP566B183 protein (DKFZP566B183), mRNA. |
| 71 | NM_022822 | 3.28 | Homo sapiens likely ortholog of kinesin light chain 2 (KLC2), mRNA. |
| 72 | NM_182579 | 3.25 | Homo sapiens hypothetical protein FLJ40343 (FLJ40343), mRNA. |
| 73 | NM_079834 | 3.21 | Homo sapiens secretory carrier membrane protein 4 (SCAMP4), mRNA. |
| 74 | NM_021120 | 3.18 | Homo sapiens discs, large homolog 3 (neuroendocrine-dlg, Drosophila) (DLG3), mRNA. |
| 75 | NM_198261 | 3.15 | Homo sapiens similar to splicing factor, arginine/serine-rich 4 (FLJ11021), transcript variant 2, mRNA. |
| 76 | NM_198263 | 3.15 | n/a |
| 77 | NM_023012 | 3.15 | Homo sapiens similar to splicing factor, arginine/serine-rich 4 (FLJ11021), transcript variant 1, mRNA. |
| 78 | NM_198262 | 3.15 | Homo sapiens similar to splicing factor, arginine/serine-rich 4 (FLJ11021), transcript variant 3, mRNA. |
| 79 | NM_018036 | 3.15 | Homo sapiens chromosome 14 open reading frame 103 (C14orf103), mRNA. |
| 80 | NM_003188 | 3.14 | Homo sapiens mitogen-activated protein kinase kinase kinase 7 (MAP3K7), transcript variant A, mRNA. |
| 81 | NM_145331 | 3.14 | Homo sapiens mitogen-activated protein kinase kinase kinase 7 (MAP3K7), transcript variant B, mRNA. |
| 82 | NM_001795 | 3.12 | Homo sapiens cadherin 5, type 2, VE-cadherin (vascular epithelium) (CDH5), mRNA. |
| 83 | NM_024490 | 3.12 | Homo sapiens ATPase, Class V, type 10A (ATP10A), mRNA. |
| 84 | NM_019102 | 3.08 | Homo sapiens homeo box A5 (HOXA5), mRNA. |
| 85 | NM_007375 | 3.06 | Homo sapiens TAR DNA binding protein (TARDBP), mRNA. |
| 86 | NM_006769 | 3.04 | Homo sapiens LIM domain only 4 (LMO4), mRNA. |
| 87 | NM_014603 | 2.99 | Homo sapiens paraneoplastic antigen (HUMPPA), mRNA. |
| 88 | NM_031913 | 2.98 | Homo sapiens chr3 synaptotagmin (CHR3SYT), mRNA. |
| 89 | NM_014399 | 2.97 | Homo sapiens transmembrane 4 superfamily member 13 (TM4SF13), mRNA. |
| 90 | NM_005369 | 2.96 | Homo sapiens MCF.2 cell line derived transforming sequence (MCF2), mRNA. |
| 91 | NM_021999 | 2.92 | Homo sapiens integral membrane protein 2B (ITM2B), mRNA. |
| 92 | NM_144776 | 2.88 | Homo sapiens formyltetrahydrofolate dehydrogenase (FTHFD), transcript variant 2, mRNA. |
| 93 | NM_001901 | 2.88 | Homo sapiens connective tissue growth factor (CTGF), mRNA. |
| 94 | NM_004637 | 2.87 | Homo sapiens RAB7, member RAS oncogene family (RAB7), mRNA. |
| 95 | NM_000210 | 2.86 | Homo sapiens integrin, alpha 6 (ITGA6), mRNA. |
| 96 | NM_014939 | 2.85 | Homo sapiens KIAA1012 (KIAA1012), mRNA. |
| 97 | NM_138717 | 2.83 | Homo sapiens palmitoyl-protein thioesterase 2 (PPT2), transcript variant 2, mRNA. |
| 98 | NM_152934 | 2.83 | Homo sapiens protein phosphatase, EF hand calcium-binding domain 2 (PPEF2), transcript variant 3, MRNA. |
| 99 | NM_145333 | 2.83 | Homo sapiens mitogen-activated protein kinase kinase kinase 7 (MAP3K7), transcript variant D, mRNA. |
| 100 | NM_145332 | 2.83 | Homo sapiens mitogen-activated protein kinase kinase kinase 7 (MAP3K7), transcript variant C, mRNA. |
| 101 | NM_005155 | 2.82 | Homo sapiens palmitoyl-protein thioesterase 2 (PPT2), transcript variant 1, mRNA. |
| 102 | NM_001001330 | 2.81 | Homo sapiens chromosome 10 open reading frame 74 (C10orf74), mRNA. |
| 103 | NM_206909 | 2.8 | Homo sapiens pleckstrin and Sec7 domain containing 3 (PSD3), transcript variant 2, mRNA. |
| 104 | NM_178450 | 2.8 | Homo sapiens membrane-associated RING-CH protein III (MARCH-III), mRNA. |
| 105 | NM_016626 | 2.76 | Homo sapiens ring finger and KH domain containing 2 (RKHD2), mRNA. |
| 106 | NM_005584 | 2.73 | Homo sapiens mab-21-like 1 (C. elegans) (MAB21L1), mRNA. |
| 107 | NM_022051 | 2.72 | Homo sapiens egl nine homolog 1 (C. elegans) (EGLN1), mRNA. |
| 108 | NM_006621 | 2.72 | Homo sapiens S-adenosylhomocysteine hydrolase-like 1 (AHCYL1), mRNA. |
| 109 | NM_003077 | 2.72 | Homo sapiens SWI/SNF related, matrix associated, actin dependent regulator of chromatin, subfamily d, member 2 (SMARCD2), mRNA. |
| 110 | NM_005668 | 2.72 | Homo sapiens sialyltransferase 8D (alpha-2, 8-polysialyltransferase) (SIAT8D), transcript variant 1, mRNA. |
| 111 | NM_019903 | 2.68 | Homo sapiens adducin 3 (gamma) (ADD3), transcript variant 2, mRNA. |
| 112 | NM_032975 | 2.68 | Homo sapiens dystrobrevin, alpha (DTNA), transcript variant 2, mRNA. |
| 113 | NM_001390 | 2.68 | Homo sapiens dystrobrevin, alpha (DTNA), transcript variant 1, mRNA. |
| 114 | NM_032980 | 2.68 | Homo sapiens dystrobrevin, alpha (DTNA), transcript variant 6, mRNA. |
| 115 | NM_016824 | 2.68 | Homo sapiens adducin 3 (gamma) (ADD3), transcript variant 1, mRNA. |
| 116 | NM_000291 | 2.68 | Homo sapiens phosphoglycerate kinase 1 (PGK1), mRNA. |
| 117 | NM_153184 | 2.67 | Homo sapiens immunoglobulin superfamily, member 4D (IGSF4D), mRNA. |
| 118 | NM_004384 | 2.67 | Homo sapiens casein kinase 1, gamma 3 (CSNK1G3), mRNA. |
| 119 | NM_022552 | 2.65 | Homo sapiens DNA (cytosine-5-)-methyltransferase 3 alpha (DNMT3A), transcript variant 3, mRNA. |
| 120 | NM_175629 | 2.65 | Homo sapiens DNA (cytosine-5-)-methyltransferase 3 alpha (DNMT3A), transcript variant 1, mRNA. |
| 121 | NM_153759 | 2.65 | Homo sapiens DNA (cytosine-5-)-methyltransferase 3 alpha (DNMT3A), transcript variant 2, mRNA. |
| 122 | NM_178835 | 2.62 | Homo sapiens hypothetical protein LOC152485 (LOC152485), mRNA. |
| 123 | NM_004999 | 2.61 | Homo sapiens myosin VI (MY06), mRNA. |
| 124 | NM_002222 | 2.6 | Homo sapiens inositol 1,4,5-triphosphate receptor, type 1 (ITPR1), mRNA. |
| 125 | NM_015719 | 2.59 | Homo sapiens collagen, type V, alpha 3 (COL5A3), mRNA. |
| 126 | NM_013316 | 2.57 | Homo sapiens CCR4-NOT transcription complex, subunit 4 (CNOT4), mRNA. |
| 127 | NM_001094 | 2.57 | Homo sapiens amiloride-sensitive cation channel 1, neuronal (degenerin) (ACCN1), transcript variant 2, mRNA. |
| 128 | NM_031412 | 2.57 | Homo sapiens GABA(A) receptor-associated protein like 1 (GABARAPL1), mRNA. |
| 129 | NM_183377 | 2.57 | Homo sapiens amiloride-sensitive cation channel 1, neuronal (degenerin) (ACCN1), transcript variant 1, mRNA. |
| 130 | NM_014614 | 2.53 | Homo sapiens proteasome (prosome, macropain) activator subunit 4 (PSME4), mRNA. |
| 131 | NM_005871 | 2.51 | Homo sapiens survival motor neuron domain containing 1 (SMNDC1), mRNA. |
| 132 | NM_003744 | 2.49 | Homo sapiens numb homolog (Drosophila) (NUMB), transcript variant 3, mRNA. |
| 133 | NM_001005745 | 2.49 | Homo sapiens numb homolog (Drosophila) (NUMB), transcript variant 4, mRNA. |
| 134 | NM_001005744 | 2.49 | Homo sapiens numb homolog (Drosophila) (NUMB), transcript variant 2, mRNA. |
| 135 | NM_001005743 | 2.49 | Homo sapiens numb homolog (Drosophila) (NUMB), transcript variant 1, mRNA. |
| 136 | NM_004274 | 2.48 | Homo sapiens A kinase (PRKA) anchor protein 6 (AKAP6), mRNA. |
| 137 | NM_003489 | 2.47 | Homo sapiens nuclear receptor interacting protein 1 (NRIP1), mRNA. |
| 138 | NM_017903 | 2.47 | Homo sapiens hypothetical protein FLJ20618 (FLJ20618), mRNA. |
| 139 | NM_156036 | 2.47 | Homo sapiens homeo box B6 (HOXB6), transcript variant 3, mRNA. |
| 140 | NM_002356 | 2.46 | Homo sapiens myristoylated alanine-rich protein kinase C substrate (MARCKS), mRNA. |
| 141 | NM_004768 | 2.46 | Homo sapiens splicing factor, arginine/serine-rich 11 (SFRS11), mRNA. |
| 142 | NM_145734 | 2.45 | Homo sapiens septin 3 (SEPT3), transcript variant C, mRNA. |
| 143 | NM_001331 | 2.45 | Homo sapiens catenin (cadherin-associated protein), delta 1 (CTNND1), mRNA. |
| 144 | NM_030571 | 2.43 | Homo sapiens Nedd4 family interacting protein 1 (NDFIP1), mRNA. |
| 145 | NM_004059 | 2.41 | Homo sapiens cysteine conjugate-beta lyase; cytoplasmic (glutamine transaminase K, kyneurenine aminotransferase) (CCBLl), mRNA. |
| 146 | NM_080670 | 2.41 | Homo sapiens solute carrier family 35, member A4 (SLC35A4), mRNA. |
| 147 | NM_052822 | 2.38 | Homo sapiens secretory carrier membrane protein 1 (SCAMP1), transcript variant 2, mRNA. |
| 148 | NM_030802 | 2.38 | Homo sapiens C/EBP-induced protein (LOC81558), mRNA. |
| 149 | NM_080417 | 2.37 | Homo sapiens peanut-like 2 (Drosophila) (PNUTL2), transcript variant 4, mRNA. |
| 150 | NM_032458 | 2.36 | Homo sapiens PHD finger protein 6 (PHF6), mRNA. |
| 151 | NM_006148 | 2.35 | Homo sapiens LIM and SH3 protein 1 (LASP1), mRNA. |
| 152 | NM_024994 | 2.35 | Homo sapiens hypothetical protein FLJ12595 (FLJ12595), mRNA. |
| 153 | NM_013437 | 2.34 | Homo sapiens low density lipoprotein-related protein 12 (LRP12), mRNA. |
| 154 | NM_020925 | 2.33 | Homo sapiens KIAA1573 protein (KIAA1573), mRNA. |
| 155 | NM_138799 | 2.32 | Homo sapiens O-acyltransferase (membrane bound) domain containing 2 (OACT2), mRNA. |
| 156 | NM_017623 | 2.28 | Homo sapiens cyclin M3 (CNNM3), transcript variant 1, mRNA. |
| 157 | NM_199078 | 2.28 | Homo sapiens cyclin M3 (CNNM3), transcript variant 2, mRNA. |
| 158 | NM_014424 | 2.28 | Homo sapiens heat shock 27kDa protein family, member 7 (cardiovascular) (HSPB7), mRNA. |
| 159 | NM_000599 | 2.27 | Homo sapiens insulin-like growth factor binding protein 5 (IGFBP5), mRNA. |
| 160 | NM_021212 | 2.25 | Homo sapiens HCF-binding transcription factor Zhangfei (ZF), mRNA. |
| 161 | NM_005110 | 2.24 | Homo sapiens glutamine-fructose-6-phosphate transaminase 2 (GFPT2), mRNA. |
| 162 | NM_022780 | 2.24 | Homo sapiens hypothetical protein FLJ13910 (FLJ13910), mRNA. |
| 163 | NM_014574 | 2.23 | Homo sapiens striatin, calmodulin binding protein 3 (STRN3), mRNA. |
| 164 | NM_153020 | 2.21 | Homo sapiens RNA binding motif protein 24 (RBM24), mRNA. |
| 165 | NM_032221 | 2.2 | Homo sapiens chromodomain helicase DNA binding protein 6 (CHD6), mRNA. |
| 166 | NM_004080 | 2.19 | Homo sapiens diacylglycerol kinase, beta 90kDa (DGKB), transcript variant 1, mRNA. |
| 167 | NM_000800 | 2.19 | Homo sapiens fibroblast growth factor 1 (acidic) (FGF1), transcript variant 1, mRNA. |
| 168 | NM_000963 | 2.18 | Homo sapiens prostaglandin-endoperoxide synthase 2 (prostaglandin G/H synthase and cyclooxygenase) (PTGS2), mRNA. |
| 169 | NM_003413 | 2.16 | Homo sapiens Zic family member 3 heterotaxy 1 (odd-paired homolog, Drosophila) (ZIC3), mRNA. |
| 170 | NM_199182 | 2.1 | Homo sapiens hLAT1-3TM (IMAA), mRNA. |
| 171 | NM_033137 | 2.09 | Homo sapiens fibroblast growth factor 1 (acidic) (FGF1), transcript variant 3, mRNA. |
| 172 | NM_033136 | 2.09 | Homo sapiens fibroblast growth factor 1 (acidic) (FGF1), transcript variant 2, mRNA. |
| 173 | NM_004132 | 2.08 | Homo sapiens hyaluronan binding protein 2 (HABP2), mRNA. |
| 174 | NM_173557 | 2.05 | Homo sapiens ring finger protein 152 (RNF152), mRNA. |
| 175 | NM_014906 | 2.04 | Homo sapiens protein phosphatase 1E (PP2C domain containing) (PPM1E), mRNA. |
| 176 | NM_032010 | 2.02 | Homo sapiens microtubule-associated protein 1B (MAP1B), transcript variant 2, mRNA. |
| 177 | NM_005909 | 2.02 | Homo sapiens microtubule-associated protein 1B (MAP1B), transcript variant 1, mRNA. |
| 178 | NM_020432 | 2.02 | Homo sapiens putative homeodomain transcription factor 2 (PHTF2), mRNA. |
| 179 | NM_018712 | 2 | Homo sapiens ELMO domain containing 1 (ELMOD1), mRNA. |
| 180 | NM_004529 | 2 | Homo sapiens myeloid/lymphoid or mixed-lineage leukemia (trithorax homolog, Drosophila); translocated to, 3 (MLLT3), mRNA. |
| 181 | NM_032385 | 2 | Homo sapiens chromosome 5 open reading frame 4 (C5orf4), mRNA. |
| 182 | NM_002310 | 1.99 | Homo sapiens leukemia inhibitory factor receptor (LIFR), mRNA. |
| 183 | NM_000393 | 1.99 | Homo sapiens collagen, type V, alpha 2 (COL5A2), mRNA. |
| 184 | NM_000321 | 1.98 | Homo sapiens retinoblastoma 1 (including osteosarcoma) (RB1), mRNA. |
| 185 | NM_031211 | 1.98 | Homo sapiens LAT1-3TM protein (LAT1-3TM), mRNA. |
| 186 | NM_207044 | 1.86 | Homo sapiens endosulfine alpha (ENSA), transcript variant 4, mRNA. |
| 187 | NM_207047 | 1.86 | Homo sapiens endosulfine alpha (ENSA), transcript variant 7, mRNA. |
| 188 | NM_207043 | 1.86 | Homo sapiens endosulfine alpha (ENSA), transcript variant 2, mRNA. |
| 189 | NM_006489 | 1.86 | Homo sapiens neuro-oncological ventral antigen 1 (NOVA1), transcript variant 2, mRNA. |
| 190 | NM_019087 | 1.86 | Homo sapiens ADP-ribosylation factor related protein 2 (ARFRP2), mRNA. |
| 191 | NM_002515 | 1.86 | Homo sapiens neuro-oncological ventral antigen 1 (NOVA1), transcript variant 1, mRNA. |
| 192 | NM_015200 | 1.81 | Homo sapiens SCC-112 protein (SCC-112), mRNA. |
| 193 | NM_017423 | 1.81 | Homo sapiens UDP-N-acetyl-alpha-D-galactosamine:polypeptide N-acetylgalactosaminyltransferase 7 (GalNAc-T7) (GALNT7), mRNA. |
| 194 | NM_014876 | 1.8 | Homo sapiens KIAA0063 gene product (KIAA0063), mRNA. |
| 195 | NM_032228 | 1.78 | Homo sapiens male sterility domain containing 2 (MLSTD2), mRNA. |
| 196 | NM_022845 | 1.77 | Homo sapiens core-binding factor, beta subunit (CBFB), transcript variant 1, mRNA. |
| 197 | NM_001755 | 1.74 | Homo sapiens core-binding factor, beta subunit (CBFB), transcript variant 2, mRNA. |
| 198 | NM_006788 | 1.73 | Homo sapiens ralA binding protein 1 (RALBP1), mRNA. |
| 199 | NM_001560 | 1.68 | Homo sapiens interleukin 13 receptor, alpha 1 (IL13RA1), mRNA. |
| 200 | NM_004926 | 1.66 | Homo sapiens zinc finger protein 36, C3H type-like 1 (ZFP36L1), mRNA. |
| 201 | NM_018976 | 1.64 | Homo sapiens solute carrier family 38, member 2 (SLC38A2), mRNA. |
| 202 | NM_000868 | 1.63 | Homo sapiens 5-hydroxytryptamine (serotonin) receptor 2C (HTR2C), mRNA. |
| 203 | NM_014810 | 1.63 | Homo sapiens centrosome-associated protein 350 (CAP350), mRNA. |
| 204 | NM_000721 | 1.61 | Homo sapiens calcium channel, voltage-dependent, alpha 1E subunit (CACNA1E), mRNA. |
| 205 | NM_002076 | 1.6 | Homo sapiens glucosamine (N-acetyl)-6-sulfatase (Sanfilippo disease IIID) (GNS), mRNA. |
| 206 | NM_182646 | 1.58 | Homo sapiens cytoplasmic polyadenylation element binding protein 2 (CPEB2), transcript variant A, mRNA. |
| 207 | NM_182485 | 1.58 | Homo sapiens cytoplasmic polyadenylation element binding protein 2 (CPEB2), transcript variant B, mRNA. |
| 208 | NM_021079 | 1.55 | Homo sapiens N-myristoyltransferase 1 (NMT1), mRNA. |
| 209 | NM_199324 | 1.47 | Homo sapiens HIV-1 induced protein HIN-1 (HSHIN1), transcript variant 1, mRNA. |
| 210 | NM_145808 | 1.44 | Homo sapiens myotrophin (MTPN), mRNA. |
| 211 | NM_005400 | 1.43 | Homo sapiens protein kinase C, epsilon (PRKCE), mRNA. |
| 212 | NM_181897 | 1.42 | Homo sapiens protein phosphatase 2 (formerly 2A), regulatory subunit B", alpha (PPP2R3A), transcript variant 2, mRNA. |
| 213 | NM_002718 | 1.42 | Homo sapiens protein phosphatase 2 (formerly 2A), regulatory subunit B", alpha (PPP2R3A), transcript variant 1, mRNA. |
| 214 | NM_001438 | 1.2 | Homo sapiens estrogen-related receptor gamma (ESRRG), transcript variant 1, mRNA. |
| 215 | NM_206594 | 1.2 | Homo sapiens estrogen-related receptor gamma (ESRRG), transcript variant 2, mRNA. |
| 216 | NM_206595 | 1.2 | Homo sapiens estrogen-related receptor gamma (ESRRG), transcript variant 3, mRNA. |

In the case of the miR-145 miRNA, for example, Table 2 provides a listing of human mRNAs predicted to contain target sites for hsa-miR-145, using the method as described in Krek et al. (2005) Nature Genetics37(5): 495-500, using default parameters.

**Table 2**

| **Rank** | **Human Refseq Id** | **PicTar score** | **annotation** |
|---|---|---|---|
| 1 | NM_013994 | 13.62 | Homo sapiens discoidin domain receptor family, member 1 (DDR1), transcript variant 3, mRNA. |
| 2 | NM_013993 | 13.62 | Homo sapiens discoidin domain receptor family, member 1 (DDR1), transcript variant 1, mRNA. |
| 3 | NM_001954 | 13.62 | Homo sapiens discoidin domain receptor family, member 1 (DDR1), transcript variant 2, mRNA. |
| 4 | NM_015094 | 10 | Homo sapiens hypermethylated in cancer 2 (HIC2), mRNA. |
| 5 | NM_002017 | 9.85 | Homo sapiens Friend leukemia virus integration 1 (FLI1), mRNA. |
| 6 | NM_005797 | 7.62 | Homo sapiens epithelial V-like antigen 1 (EVA1), transcript variant 1, mRNA. |
| 7 | NM_014871 | 7.58 | Homo sapiens ubiquitin specific protease 52 (USP52), mRNA. |
| 8 | NM_001128 | 7.54 | Homo sapiens adaptor-related protein complex 1, gamma 1 subunit (AP1G1), mRNA. |
| 9 | NM_015271 | 7.44 | Homo sapiens tripartite motif-containing 2 (TRIM2), mRNA. |
| 10 | NM_017999 | 7.08 | Homo sapiens ring finger protein 31 (RNF31), mRNA. |
| 11 | NM_018011 | 6.83 | Homo sapiens hypothetical protein FLJ10154 (FLJ10154), mRNA. |
| 12 | NM_199072 | 6.47 | Homo sapiens I-mfa domain-containing protein (HIC), mRNA. |
| 13 | NM_033046 | 6.27 | Homo sapiens rhotekin (RTKN), mRNA. |
| 14 | NM_173797 | 6.25 | Homo sapiens PAP associated domain containing 4 (PAPD4), mRNA. |
| 15 | NM_016824 | 6.16 | Homo sapiens adducin 3 (gamma) (ADD3), transcript variant 1, mRNA. |
| 16 | NM_019903 | 6.16 | Homo sapiens adducin 3 (gamma) (ADD3), transcript variant 2, mRNA. |
| 17 | NM_006506 | 6.05 | Homo sapiens RAS p21 protein activator 2 (RASA2), mRNA. |
| 18 | NM_021832 | 5.84 | Homo sapiens a disintegrin and metalloproteinase domain 17 (tumor necrosis factor, alpha, converting enzyme) (ADAM17), transcript variant 2, mRNA. |
| 19 | NM_182492 | 5.67 | Homo sapiens hypothetical protein DKFZp43400213 (DKFZp43400213), mRNA. |
| 20 | NM_144497 | 5.5 | Homo sapiens A kinase (PRKA) anchor protein (gravin) 12 (AKAP12), transcript variant 2, mRNA. |
| 21 | NM_005100 | 5.5 | Homo sapiens A kinase (PRKA) anchor protein (gravin) 12 (AKAP12), transcript variant 1, mRNA. |
| 22 | NM_005717 | 5.45 | Homo sapiens actin related protein 2/3 complex, subunit 5,16kDa (ARPC5), mRNA. |
| 23 | NM_014923 | 5.35 | Homo sapiens fibronectin type III domain containing 3 (FNDC3), mRNA. |
| 24 | NM_020762 | 5.31 | Homo sapiens SLIT-ROBO Rho GTPase activating protein 1 (SRGAP1), mRNA. |
| 25 | NM_005347 | 5.3 | Homo sapiens heat shock 70kDa protein 5 (glucose-regulated protein, 78kDa) (HSPA5), mRNA. |
| 26 | NM_015184 | 5.21 | Homo sapiens phospholipase C-like 2 (PLCL2), mRNA. |
| 27 | NM_052830 | 5.18 | Homo sapiens gamma-glutamyltransferase-like 3 (GGTL3), transcript variant 1, mRNA. |
| 28 | NM_002657 | 5.13 | Homo sapiens pleiomorphic adenoma gene-like 2 (PLAGL2), mRNA. |
| 29 | NM_001002924 | 5.13 | Homo sapiens adaptor-related protein complex 3, sigma 1 subunit (AP3S1), transcript variant 2, mRNA. |
| 30 | NM_013279 | 5.06 | Homo sapiens chromosome 11 open reading frame 9 (C11orf9), mRNA. |
| 31 | NM_014903 | 4.9 | Homo sapiens neuron navigator 3 (NAV3), mRNA. |
| 32 | NM_032726 | 4.8 | Homo sapiens phospholipase C, delta 4 (PLCD4), mRNA. |
| 33 | NM_004714 | 4.77 | Homo sapiens dual-specificity tyrosine-(Y)-phosphorylation regulated kinase 1B (DYRK1B), transcript variant a, mRNA. |
| 34 | NM_006484 | 4.77 | Homo sapiens dual-specificity tyrosine-(Y)-phosphorylation regulated kinase 1B (DYRK1B), transcript variant c, mRNA. |
| 35 | NM_006483 | 4.77 | Homo sapiens dual-specificity tyrosine-(Y)-phosphorylation regulated kinase 1B (DYRK1B), transcript variant b, mRNA. |
| 36 | NM_033274 | 4.7 | Homo sapiens a disintegrin and metalloproteinase domain 19 (meltrin beta) (ADAM19), transcript variant 2, mRNA. |
| 37 | NM_020909 | 4.67 | Homo sapiens erythrocyte membrane protein band 4.1 like 5 (EPB41L5), mRNA. |
| 38 | NM_024643 | 4.48 | Homo sapiens chromosome 14 open reading frame 140 (C14orf140), mRNA. |
| 39 | NM_017913 | 4.44 | Homo sapiens cell division cycle 37 homolog (S. cerevisiae)-like 1 (CDC37L1), mRNA. |
| 40 | NM_005384 | 4.42 | Homo sapiens nuclear factor, interleukin 3 regulated (NFIL3), mRNA. |
| 41 | NM_178026 | 4.42 | Homo sapiens gamma-glutamyltransferase-like 3 (GGTL3), transcript variant 3, mRNA. |
| 42 | NM_018360 | 4.32 | Homo sapiens chromosome X open reading frame 15 (CXorf15), mRNA. |
| 43 | NM_016032 | 4.29 | Homo sapiens zinc finger, DHHC domain containing (ZDHHC9), mRNA. |
| 44 | NM_016258 | 4.28 | Homo sapiens YTH domain family, member 2 (YTHDF2), mRNA. |
| 45 | NM_182664 | 4.23 | Homo sapiens Ras association (RalGDS/AF-6) domain family 5 (RASSF5), transcript variant 2, mRNA. |
| 46 | NM_006702 | 4.22 | Homo sapiens neuropathy target esterase (NTE), mRNA. |
| 47 | NM_006080 | 4.2 | Homo sapiens sema domain, immunoglobulin domain (Ig), short basic domain, secreted, (semaphorin) 3A (SEMA3A), mRNA. |
| 48 | NM_004276 | 4.12 | Homo sapiens calcium binding protein 1 (calbrain) (CABP1), transcript variant 2, mRNA. |
| 49 | NM_031205 | 4.12 | Homo sapiens calcium binding protein 1 (calbrain) (CABP1), transcript variant 1, mRNA. |
| 50 | NM_147193 | 4.05 | Homo sapiens GLIS family zinc finger 1 (GLIS1), mRNA. |
| 51 | NM_004865 | 4.02 | Homo sapiens TBP-like 1 (TBPL1), mRNA. |
| 52 | NM_001284 | 4.02 | Homo sapiens adaptor-related protein complex 3, sigma 1 subunit (AP3S1), transcript variant 1, mRNA. |
| 53 | NM_022652 | 3.98 | Homo sapiens dual specificity phosphatase 6 (DUSP6), transcript variant 2, mRNA. |
| 54 | NM_001946 | 3.98 | Homo sapiens dual specificity phosphatase 6 (DUSP6), transcript variant 1, mRNA. |
| 55 | NM_001967 | 3.93 | Homo sapiens eukaryotic translation initiation factor 4A, isoform 2 (EIF4A2), mRNA. |
| 56 | NM_000944 | 3.92 | Homo sapiens protein phosphatase 3 (formerly 2B), catalytic subunit, alpha isoform (calcineurin A alpha) (PPP3CA), mRNA. |
| 57 | NM_002013 | 3.88 | Homo sapiens FK506 binding protein 3, 25kDa (FKBP3), mRNA. |
| 58 | NM_005433 | 3.82 | Homo sapiens v-yes-1 Yamaguchi sarcoma viral oncogene homolog 1 (YES1), mRNA. |
| 59 | NM_002973 | 3.81 | Homo sapiens ataxin 2 (ATXN2), mRNA. |
| 60 | NM_145799 | 3.81 | Homo sapiens septin 6 (SEPT6), transcript variant I, mRNA. |
| 61 | NM_001386 | 3.79 | Homo sapiens dihydropyrimidinase-like 2 (DPYSL2), mRNA. |
| 62 | NM_005723 | 3.78 | Homo sapiens transmembrane 4 superfamily member 9 (TM4SF9), mRNA. |
| 63 | NM_007146 | 3.71 | Homo sapiens zinc finger protein 161 (ZNF161), mRNA. |
| 64 | NM_024586 | 3.7 | Homo sapiens oxysterol binding protein-like 9 (OSBPL9), transcript variant 6, mRNA. |
| 65 | NM_148909 | 3.7 | Homo sapiens oxysterol binding protein-like 9 (OSBPL9), transcript variant 7, mRNA. |
| 66 | NM_148906 | 3.7 | Homo sapiens oxysterol binding protein-like 9 (OSBPL9), transcript variant 3, mRNA. |
| 67 | NM_052887 | 3.7 | Homo sapiens toll-interleukin 1 receptor (TIR) domain containing adaptor protein (TIRAP), transcript variant 1, mRNA. |
| 68 | NM_148907 | 3.7 | Homo sapiens oxysterol binding protein-like 9 (OSBPL9), transcript variant 4, mRNA. |
| 69 | NM_148908 | 3.7 | Homo sapiens oxysterol binding protein-like 9 (OSBPL9), transcript variant 5, mRNA. |
| 70 | NM_148905 | 3.7 | Homo sapiens oxysterol binding protein-like 9 (OSBPL9), transcript variant 2, mRNA. |
| 71 | NM_148904 | 3.7 | Homo sapiens oxysterol binding protein-like 9 (OSBPL9), transcript variant 1, mRNA. |
| 72 | NM_020307 | 3.68 | Homo sapiens cyclin L1 (CCNL1), mRNA. |
| 73 | NM_184042 | 3.63 | Homo sapiens Cohen syndrome 1 (COH1), transcript variant 2, mRNA. |
| 74 | NM_001457 | 3.63 | Homo sapiens filamin B, beta (actin binding protein 278) (FLNB), mRNA. |
| 75 | NM_032511 | 3.56 | Homo sapiens chromosome 6 open reading frame 168 (C6orf168), mRNA. |
| 76 | NM_016389 | 3.55 | Homo sapiens influenza virus NS1A binding protein (IVNS1ABP), transcript variant 2, mRNA. |
| 77 | NM_001326 | 3.54 | Homo sapiens cleavage stimulation factor, 3' pre-RNA, subunit 3, 77kDa (CSTF3), mRNA. |
| 78 | NM_152600 | 3.52 | Homo sapiens zinc finger protein 579 (ZNF579), mRNA. |
| 79 | NM_014016 | 3.5 | Homo sapiens SAC1 suppressor of actin mutations 1-like (yeast) (SACM1L), mRNA. |
| 80 | NM_052925 | 3.48 | Homo sapiens leukocyte receptor cluster (LRC) member 8 (LENG8), mRNA. |
| 81 | NM_052911 | 3.47 | Homo sapiens establishment factor-like protein (EFO1), mRNA. |
| 82 | NM_015129 | 3.46 | Homo sapiens septin 6 (SEPT6), transcript variant II, mRNA. |
| 83 | NM_002924 | 3.43 | Homo sapiens regulator of G-protein signalling 7 (RGS7), mRNA. |
| 84 | NM_002229 | 3.42 | Homo sapiens jun B proto-oncogene (JUNB), mRNA. |
| 85 | NM_153498 | 3.35 | Homo sapiens calcium/calmodulin-dependent protein kinase ID (CAMK1D), transcript variant 2, mRNA. |
| 86 | NM_005119 | 3.34 | Homo sapiens thyroid hormone receptor associated protein 3 (THRAP3), mRNA. |
| 87 | NM_173078 | 3.34 | Homo sapiens SLIT and NTRK-like family, member 4 (SLITRK4), mRNA. |
| 88 | NM_003893 | 3.31 | Homo sapiens LIM domain binding 1 (LDB1), mRNA. |
| 89 | NM_000346 | 3.3 | Homo sapiens SRY (sex determining region Y)-box 9 (campomelic dysplasia, autosomal sex-reversal) (SOX9), mRNA. |
| 90 | NM_032222 | 3.27 | Homo sapiens hypothetical protein FLJ22374 (FLJ22374), mRNA. |
| 91 | NM_133370 | 3.24 | Homo sapiens splicing factor YT521-B (YT521), mRNA. |
| 92 | NM_015069 | 3.23 | Homo sapiens zinc finger protein 423 (ZNF423), mRNA. |
| 93 | NM_018271 | 3.23 | Homo sapiens hypothetical protein FLJ10916 (FLJ10916), mRNA. |
| 94 | NM_030962 | 3.2 | Homo sapiens SET binding factor 2 (SBF2), mRNA. |
| 95 | NM_014363 | 3.19 | Homo sapiens spastic ataxia of Charlevoix-Saguenay (sacsin) (SACS), mRNA. |
| 96 | NM_017640 | 3.15 | Homo sapiens leucine rich repeat containing 16 (LRRC16), mRNA. |
| 97 | NM_005544 | 3.14 | Homo sapiens insulin receptor substrate 1 (IRS1), mRNA. |
| 98 | NM_002912 | 3.09 | Homo sapiens REV3-like, catalytic subunit of DNA polymerase zeta (yeast) (REV3L), mRNA. |
| 99 | NM_001614 | 3.09 | Homo sapiens actin, gamma 1 (ACTG1), mRNA. |
| 100 | NM_015361 | 3.09 | Homo sapiens R3H domain (binds single-stranded nucleic acids) containing (R3HDM), mRNA. |
| 101 | NM_152390 | 3.09 | Homo sapiens hypothetical protein MGC33926 (MGC33926), mRNA. |
| 102 | NM_025076 | 3.07 | Homo sapiens UDP-glucuronate decarboxylase 1 (UXS1), mRNA. |
| 103 | NM_152945 | 3.06 | Homo sapiens developmentally regulated RNA-binding protein 1 (DRB1), mRNA. |
| 104 | NM_014267 | 3.04 | Homo sapiens small acidic protein (SMAP), mRNA. |
| 105 | NM_007345 | 3.04 | Homo sapiens zinc finger protein 236 (ZNF236), mRNA. |
| 106 | NM_023071 | 3.03 | Homo sapiens spermatogenesis associated, serine-rich 2 (SPATS2), mRNA. |
| 107 | NM_004755 | 3.01 | Homo sapiens ribosomal protein S6 kinase, 90kDa, polypeptide 5 (RPS6KA5), transcript variant 1, mRNA. |
| 108 | NM_001901 | 2.99 | Homo sapiens connective tissue growth factor (CTGF), mRNA. |
| 109 | NM_018270 | 2.98 | Homo sapiens chromosome 20 open reading frame 20 (C20orf20), mRNA. |
| 110 | NM_001097 | 2.98 | Homo sapiens active BCR-related gene (ABR), transcript variant 2, mRNA. |
| 111 | NM_021962 | 2.98 | Homo sapiens active BCR-related gene (ABR), transcript variant 1, mRNA. |
| 112 | NM_182527 | 2.97 | Homo sapiens calcium binding protein 7 (CABP7), mRNA. |
| 113 | NM_021612 | 2.96 | Homo sapiens a disintegrin and metalloproteinase domain 11 (ADAM11), transcript variant 2, mRNA. |
| 114 | NM_020806 | 2.96 | Homo sapiens gephyrin (GPHN), mRNA. |
| 115 | NM_000959 | 2.95 | Homo sapiens prostaglandin F receptor (FP) (PTGFR), mRNA. |
| 116 | NM_016322 | 2.94 | Homo sapiens RAB14, member RAS oncogene family (RAB 14), mRNA. |
| 117 | NM_138440 | 2.94 | Homo sapiens vasorin (LOC114990), mRNA. |
| 118 | NM_194293 | 2.93 | Homo sapiens cardiomyopathy associated 1 (CMYA1), mRNA. |
| 119 | NM_022650 | 2.92 | Homo sapiens RAS p21 protein activator (GTPase activating protein) 1 (RASA1), transcript variant 2, mRNA. |
| 120 | NM_002890 | 2.92 | Homo sapiens RAS p21 protein activator (GTPase activating protein) 1 (RASA1), transcript variant 1, mRNA. |
| 121 | NM_001616 | 2.92 | Homo sapiens activin A receptor, type II (ACVR2), mRNA. |
| 122 | NM_018607 | 2.9 | Homo sapiens hypothetical protein PRO1853 (PRO1853), transcript variant 2, mRNA. |
| 123 | NM_033346 | 2.87 | Homo sapiens bone morphogenetic protein receptor, type II (serine/threonine kinase) (BMPR2), transcript variant 2, mRNA. |
| 124 | NM_07005 | 2.87 | Homo sapiens transducin-like enhancer of split 4 (E(sp1) homolog, Drosophila) (TLE4), mRNA. |
| 125 | NM_033505 | 2.87 | Homo sapiens selenoprotein I (SELI), mRNA. |
| 126 | NM_014800 | 2.85 | Homo sapiens engulfment and cell motility 1 (ced-12 homolog, C. elegans) (ELMO1), transcript variant 1, mRNA. |
| 127 | NM_130442 | 2.85 | Homo sapiens engulfment and cell motility 1 (ced-12 homolog, C. elegans) (ELMO1), transcript variant 2, mRNA. |
| 128 | NM_023929 | 2.82 | Homo sapiens zinc finger and BTB domain containing (ZBTB10), mRNA. |
| 129 | NM_198138 | 2.82 | Homo sapiens SEC31-like 2 (S. cerevisiae) (SEC31L2), transcript variant 2, mRNA. |
| 130 | NM_015277 | 2.8 | Homo sapiens neural precursor cell expressed, developmentally down-regulated 4-like (NEDD4L), mRNA. |
| 131 | NM_017732 | 2.8 | Homo sapiens hypoxia-inducible factor prolyl 4-hydroxylase (PH-4), transcript variant 2, mRNA. |
| 132 | NM_133476 | 2.79 | Homo sapiens zinc finger protein 384 (ZNF384), mRNA. |
| 133 | NM_022845 | 2.79 | Homo sapiens core-binding factor, beta subunit (CBFB), transcript variant 1, mRNA. |
| 134 | NM_207424 | 2.77 | Homo sapiens FLJ40536 protein (FLJ40536), mRNA. |
| 135 | NM_017641 | 2.76 | Homo sapiens kinesin family member 21A (KIF21A), mRNA. |
| 136 | NM_001755 | 2.76 | Homo sapiens core-binding factor, beta subunit (CBFB), transcript variant 2, mRNA. |
| 137 | NM_014840 | 2.76 | Homo sapiens AMP-activated protein kinase family member 5 (ARK5), mRNA. |
| 138 | NM_022977 | 2.75 | Homo sapiens acyl-CoA synthetase long-chain family member 4 (ACSL4), transcript variant 2, mRNA. |
| 139 | NM_004458 | 2.75 | Homo sapiens acyl-CoA synthetase long-chain family member 4 (ACSL4), transcript variant 1, mRNA. |
| 140 | NM_022832 | 2.73 | Homo sapiens ubiquitin specific protease 46 (USP46), mRNA. |
| 141 | NM_025057 | 2.73 | Homo sapiens chromosome 14 open reading frame 45 (C14orf45), mRNA. |
| 142 | NM_130436 | 2.72 | Homo sapiens dual-specificity tyrosine-(Y)-phosphorylation regulated kinase 1A (DYRK1A), transcript variant 2, mRNA. |
| 143 | NM_001396 | 2.72 | Homo sapiens dual-specificity tyrosine-(Y)-phosphorylation regulated kinase 1A (DYRK1A), transcript variant 1, mRNA. |
| 144 | NM_004096 | 2.72 | Homo sapiens eukaryotic translation initiation factor 4E binding protein 2 (EIF4EBP2), mRNA. |
| 145 | NM_052900 | 2.71 | Homo sapiens CUB and Sushi multiple domains 3 (CSMD3), transcript variant c, mRNA. |
| 146 | NM_198123 | 2.71 | Homo sapiens CUB and Sushi multiple domains 3 (CSMD3), transcript variant a, mRNA. |
| 147 | NM_198124 | 2.71 | Homo sapiens CUB and Sushi multiple domains 3 (CSMD3), transcript variant b, mRNA. |
| 148 | NM_016231 | 2.7 | Homo sapiens nemo like kinase (NLK), mRNA. |
| 149 | NM_031284 | 2.7 | Homo sapiens ADP-dependent glucokinase (ADPGK), mRNA. |
| 150 | NM_020772 | 2.7 | Homo sapiens 82-kD FMRP Interacting Protein (182-FIP), mRNA. |
| 151 | NM_018993 | 2.69 | Homo sapiens Ras and Rab interactor 2 (RIN2), mRNA. |
| 152 | NM_173640 | 2.68 | Homo sapiens likely ortholog of mouse roof plate-specific spondin (R-spondin), mRNA. |
| 153 | NM_014212 | 2.67 | Homo sapiens homeo box C11 (HOXC11), mRNA. |
| 154 | NM_001259 | 2.67 | Homo sapiens cyclin-dependent kinase 6 (CDK6), mRNA. |
| 155 | NM_005721 | 2.64 | Homo sapiens ARP3 actin-related protein 3 homolog (yeast) (ACTR3), mRNA. |
| 156 | NM_014603 | 2.64 | Homo sapiens paraneoplastic antigen (HUMPPA), mRNA. |
| 157 | NM_015187 | 2.62 | Homo sapiens KIAA0746 protein (KIAA0746), mRNA. |
| 158 | NM_138638 | 2.6 | Homo sapiens cofilin 2 (muscle) (CFL2), transcript variant 2, mRNA. |
| 159 | NM_021914 | 2.6 | Homo sapiens cofilin 2 (muscle) (CFL2), transcript variant 1, mRNA. |
| 160 | NM_052905 | 2.58 | Homo sapiens formin-like 2 (FMNL2), transcript variant 2, mRNA. |
| 161 | NM_018227 | 2.56 | Homo sapiens hypothetical protein FLJ10808 (FLJ10808), mRNA. |
| 162 | NM_020796 | 2.56 | Homo sapiens sema domain, transmembrane domain (TM), and cytoplasmic domain, (semaphorin) 6A (SEMA6A), mRNA. |
| 163 | NM_017903 | 2.54 | Homo sapiens hypothetical protein FLJ20618 (FLJ20618), mRNA. |
| 164 | NM_002745 | 2.53 | Homo sapiens mitogen-activated protein kinase 1 (MAPK1), transcript variant 1, mRNA. |
| 165 | NM_001004422 | 2.52 | Homo sapiens formin-like 2 (FMNL2), transcript variant 3, mRNA. |
| 166 | NM_006922 | 2.52 | Homo sapiens sodium channel, voltage-gated, type III, alpha (SCN3A), mRNA. |
| 167 | NM_032017 | 2.52 | Homo sapiens Ser/Thr-like kinase (MGC4796), mRNA. |
| 168 | NM_024595 | 2.51 | Homo sapiens hypothetical protein FLJ12666 (FLJ12666), mRNA. |
| 169 | NM_004393 | 2.5 | Homo sapiens dystroglycan 1 (dystrophin-associated glycoprotein 1) (DAG1), mRNA. |
| 170 | NM_198793 | 2.48 | Homo sapiens CD47 antigen (Rh-related antigen, integrin-associated signal transducer) (CD47), transcript variant 2, mRNA. |
| 171 | NM_001004417 | 2.48 | Homo sapiens formin-like 2 (FMNL2), transcript variant 4, mRNA. |
| 172 | NM_001777 | 2.48 | Homo sapiens CD47 antigen (Rh-related antigen, integrin-associated signal transducer) (CD47), transcript variant 1, mRNA. |
| 173 | NM_152253 | 2.48 | Homo sapiens choline kinase beta (CHKB), transcript variant 2, mRNA. |
| 174 | NM_032432 | 2.47 | Homo sapiens actin binding LIM protein family, member 2 (ABLIM2), mRNA. |
| 175 | NM_001004421 | 2.46 | Homo sapiens formin-like 2 (FMNL2), transcript variant 1, mRNA. |
| 176 | NM_173809 | 2.46 | Homo sapiens biogenesis of lysosome-related organelles complex-1, subunit 2 (BLOC1S2), transcript variant 1, mRNA. |
| 177 | NM_002859 | 2.46 | Homo sapiens paxillin (PXN), mRNA. |
| 178 | NM_001001342 | 2.46 | Homo sapiens biogenesis of lysosome-related organelles complex-1, subunit 2 (BLOC1S2), transcript variant 2, mRNA. |
| 179 | NM_021224 | 2.45 | Homo sapiens zinc finger protein 462 (ZNF462), mRNA. |
| 180 | NM_032196 | 2.45 | Homo sapiens homolog of yeast IN080 (IN080), transcript variant 2, mRNA. |
| 181 | NM_015455 | 2.43 | Homo sapiens carbon catabolite repression 4 protein (KIAA1194), mRNA. |
| 182 | NM_032229 | 2.43 | Homo sapiens SLIT and NTRK-like family, member 6 (SLITRK6), mRNA. |
| 183 | NM_130438 | 2.41 | Homo sapiens dual-specificity tyrosine-(Y)-phosphorylation regulated kinase 1A (DYRK1A), transcript variant 5, mRNA. |
| 184 | NM_145686 | 2.39 | Homo sapiens mitogen-activated protein kinase kinase kinase kinase 4 (MAP4K4), transcript variant 2, mRNA. |
| 185 | NM_004834 | 2.39 | Homo sapiens mitogen-activated protein kinase kinase kinase kinase 4 (MAP4K4), transcript variant 1, mRNA. |
| 186 | NM_145687 | 2.39 | Homo sapiens mitogen-activated protein kinase kinase kinase kinase 4 (MAP4K4), transcript variant 3, mRNA. |
| 187 | NM_130437 | 2.38 | Homo sapiens dual-specificity tyrosine-(Y)-phosphorylation regulated kinase 1A (DYRK1A), transcript variant 4, mRNA. |
| 188 | NM_101395 | 2.38 | Homo sapiens dual-specificity tyrosine-(Y)-phosphorylation regulated kinase 1A (DYRK1A), transcript variant 3, mRNA. |
| 189 | NM_014666 | 2.38 | Homo sapiens enthoprotin (ENTH), mRNA. |
| 190 | NM_024713 | 2.36 | Homo sapiens chromosome 15 open reading frame 29 (C15orf29), mRNA. |
| 191 | NM_003076 | 2.35 | Homo sapiens SWI/SNF related, matrix associated, actin dependent regulator of chromatin, subfamily d, member 1 (SMARCD1), transcript variant 1, mRNA. |
| 192 | NM_015074 | 2.35 | Homo sapiens kinesin family member 1B (KIF1B), transcript variant 1, mRNA. |
| 193 | NM_014742 | 2.34 | Homo sapiens transmembrane 9 superfamily protein member 4 (TM9SF4), mRNA. |
| 194 | NM_139071 | 2.34 | Homo sapiens SWI/SNF related, matrix associated, actin dependent regulator of chromatin, subfamily d, member 1 (SMARCD1), transcript variant 2, mRNA. |
| 195 | NM_017553 | 2.32 | Homo sapiens homolog of yeast IN080 (IN080), transcript variant 1, mRNA. |
| 196 | NM_206909 | 2.32 | Homo sapiens pleckstrin and Sec7 domain containing 3 (PSD3), transcript variant 2, mRNA. |
| 197 | NM_005863 | 2.27 | Homo sapiens neuroepithelial cell transforming gene 1 (NET 1), mRNA. |
| 198 | NM_145341 | 2.25 | Homo sapiens programmed cell death 4 (neoplastic transformation inhibitor) (PDCD4), transcript variant 2, mRNA. |
| 199 | NM_014456 | 2.25 | Homo sapiens programmed cell death 4 (neoplastic transformation inhibitor) (PDCD4), transcript variant 1, mRNA. |
| 200 | NM_015176 | 2.25 | Homo sapiens F-box protein 28 (FBXO28), mRNA. |
| 201 | NM_002142 | 2.23 | Homo sapiens homeo box A9 (HOXA9), transcript variant 2, mRNA. |
| 202 | NM_017772 | 2.22 | Homo sapiens chromosome 6 open reading frame 197 (C6orf197), mRNA. |
| 203 | NM_017582 | 2.22 | Homo sapiens ubiquitin-conjugating enzyme E2Q (putative) (UBE2Q), mRNA. |
| 204 | NM_006469 | 2.22 | Homo sapiens influenza virus NS1A binding protein (IVNS1ABP), transcript variant 1, mRNA. |
| 205 | NM_002840 | 2.19 | Homo sapiens protein tyrosine phosphatase, receptor type, F (PTPRF), transcript variant 1, mRNA. |
| 206 | NM_130440 | 2.19 | Homo sapiens protein tyrosine phosphatase, receptor type, F (PTPRF), transcript variant 2, mRNA. |
| 207 | NM_199437 | 2.17 | Homo sapiens PR domain containing 10 (PRDM10), transcript variant 2, mRNA. |
| 208 | NM_199439 | 2.17 | Homo sapiens PR domain containing 10 (PRDM10), transcript variant 4, mRNA. |
| 209 | NM_199438 | 2.17 | Homo sapiens PR domain containing 10 (PRDM10), transcript variant 3, mRNA. |
| 210 | NM_005604 | 2.17 | Homo sapiens POU domain, class 3, transcription factor 2 (POU3F2), mRNA. |
| 211 | NM_020228 | 2.17 | Homo sapiens PR domain containing 10 (PRDM10), transcript variant 1, mRNA. |
| 212 | NM_019084 | 2.16 | Homo sapiens cyclin J (CCNJ), mRNA. |
| 213 | NM_173619 | 2.16 | Homo sapiens hypothetical protein MGC34761 (MGC34761), mRNA. |
| 214 | NM_017902 | 2.15 | Homo sapiens hypoxia-inducible factor 1, alpha subunit inhibitor (HIF1AN), mRNA. |
| 215 | NM_022735 | 2.14 | Homo sapiens acyl-Coenzyme A binding domain containing 3 (ACBD3), mRNA. |
| 216 | NM_002193 | 2.13 | Homo sapiens inhibin, beta B (activin AB beta polypeptide) (INHBB), mRNA. |
| 217 | NM_207438 | 2.12 | Homo sapiens FLJ43808 protein (FLJ43808), mRNA. |
| 218 | NM_020689 | 2.12 | Homo sapiens solute carrier family 24 (sodium/potassium/calcium exchanger), member 3 (SLC24A3), mRNA. |
| 219 | NM_012271 | 2.1 | Homo sapiens huntingtin interacting protein B (HYPB), transcript variant 2, mRNA. |
| 220 | NM_023079 | 2.09 | Homo sapiens hypothetical protein FLJ13855 (FLJ13855), mRNA. |
| 221 | NM_001759 | 2.09 | Homo sapiens cyclin D2 (CCND2), mRNA. |
| 222 | NM_018246 | 2.07 | Homo sapiens hypothetical protein FLJ10853 (FLJ10853), mRNA. |
| 223 | NM_016201 | 2.06 | Homo sapiens angiomotin like 2 (AMOTL2), mRNA. |
| 224 | NM_020925 | 2.06 | Homo sapiens KIAA1573 protein (KIAA1573), mRNA. |
| 225 | NM_207406 | 2.05 | Homo sapiens FLJ43965 protein (FLJ43965), mRNA. |
| 226 | NM_020248 | 2.05 | Homo sapiens catenin, beta interacting protein 1 (CTNNBIP1), mRNA. |
| 227 | NM_003204 | 2.03 | Homo sapiens nuclear factor (erythroid-derived 2)-like 1 (NFE2L1), mRNA. |
| 228 | NM_006459 | 2.02 | Homo sapiens SPFH domain family, member 1 (SPFH1), mRNA. |
| 229 | NM_006403 | 1.99 | Homo sapiens neural precursor cell expressed, developmentally down-regulated 9 (NEDD9), mRNA. |
| 230 | NM_003483 | 1.93 | Homo sapiens high mobility group AT-hook 2 (HMGA2), mRNA. |
| 231 | NM_203351 | 1.92 | Homo sapiens mitogen-activated protein kinase kinase kinase 3 (MAP3K3), transcript variant 1, mRNA. |
| 232 | NM_002401 | 1.92 | Homo sapiens mitogen-activated protein kinase kinase kinase 3 (MAP3K3), transcript variant 2, mRNA. |
| 233 | NM_003887 | 1.92 | Homo sapiens development and differentiation enhancing factor 2 (DDEF2), mRNA. |
| 234 | NM_013449 | 1.85 | Homo sapiens bromodomain adjacent to zinc finger domain, 2A (BAZ2A), mRNA. |
| 235 | NM_003507 | 1.84 | Homo sapiens frizzled homolog 7 (Drosophila) (FZD7), mRNA. |
| 236 | NM_014686 | 1.81 | Homo sapiens KIAA0355 (KIAA0355), mRNA. |
| 237 | NM_003759 | 1.81 | Homo sapiens solute carrier family 4, sodium bicarbonate cotransporter, member 4 (SLC4A4), mRNA. |
| 238 | NM_006380 | 1.79 | Homo sapiens amyloid beta precursor protein (cytoplasmic tail) binding protein 2 (APPBP2), mRNA. |
| 239 | NM_199040 | 1.78 | Homo sapiens nudix (nucleoside diphosphate linked moiety X)-type motif 4 (NUDT4), transcript variant 2, mRNA. |
| 240 | NM_019094 | 1.78 | Homo sapiens nudix (nucleoside diphosphate linked moiety X)-type motif 4 (NUDT4), transcript variant 1, mRNA. |
| 241 | NM_014919 | 1.76 | Homo sapiens Wolf-Hirschhorn syndrome candidate 1 (WHSC1), transcript variant 4, mRNA. |
| 242 | NM_080760 | 1.75 | Homo sapiens dachshund homolog 1 (Drosophila) (DACH1), transcript variant 2, mRNA. |
| 243 | NM_004392 | 1.75 | Homo sapiens dachshund homolog 1 (Drosophila) (DACH1), transcript variant 3, mRNA. |
| 244 | NM_080759 | 1.75 | Homo sapiens dachshund homolog 1 (Drosophila) (DACH1), transcript variant 1, mRNA. |
| 245 | NM_133333 | 1.72 | Homo sapiens Wolf-Hirschhorn syndrome candidate 1 (WHSC1), transcript variant 6, mRNA. |
| 246 | NM_133332 | 1.7 | Homo sapiens Wolf-Hirschhorn syndrome candidate 1 (WHSC1), transcript variant 5, mRNA. |
| 247 | NM_025146 | 1.62 | Homo sapiens Mak3 homolog (S. cerevisiae) (MAK3), mRNA. |
| 248 | NM_002015 | 1.56 | Homo sapiens forkhead box O1A (rhabdomyosarcoma) (FOXO1A), mRNA. |
| 249 | NM_003458 | 1.56 | Homo sapiens bassoon (presynaptic cytomatrix protein) (BSN), mRNA. |
| 250 | NM_001204 | 1.53 | Homo sapiens bone morphogenetic protein receptor, type II (serine/threonine kinase) (BMPR2), transcript variant 1, mRNA. |
| 251 | NM_004921 | 1.48 | Homo sapiens chloride channel, calcium activated, family member 3 (CLCA3), mRNA. |
| 252 | NM_005502 | 1.47 | Homo sapiens ATP-binding cassette, sub-family A (ABC1), member 1 (ABCA1), mRNA. |
| 253 | NM_148171 | 1.4 | Homo sapiens ubiquitin associated protein 2 (UBAP2), transcript variant 3, mRNA. |
| 254 | NM_015071 | 1.38 | Homo sapiens Rho GTPase activating protein 26 (ARHGAP26), mRNA. |
| 255 | NM_030627 | 1.34 | Homo sapiens cytoplasmic polyadenylation element binding protein 4 (CPEB4), mRNA. |
| 256 | NM_030918 | 1.19 | Homo sapiens sorting nexin family member 27 (SNX27), mRNA. |
| 257 | NM_004171 | 1.11 | Homo sapiens solute carrier family 1 (glial high affinity glutamate transporter), member 2 (SLC1A2), mRNA. |
| 258 | NM_021813 | 0.97 | Homo sapiens BTB and CNC homology 1, basic leucine zipper transcription factor 2 (BACH2), mRNA. |

A target site for the binding of the microRNA may be introduced into the nucleic acid with the capacity to modulate the development of cell at a suitable position in the nucleic acid. For example, in the case of the target site being introduced into a mRNA (by way, for example, of cloning the target site into the appropriate position in a plasmid encoding a gene to be transcribed), the target site(s) may be introduced into one or more of the 3'UTR, coding region and 5'UTR of the mRNA. Methods for the cloning of nucleic acid sequences are essentially as described in Sambrook, J, Fritsch, E.F. and Maniatis, T. Molecular Cloning: A Laboratory Manual 2nd. ed. Cold Spring Harbor Laboratroy Press, New York. (1989).

For example, the target site for the miR-143 and/or miR-145 microRNAs may be cloned into the 3'UTR of the HSV thymidine kinase gene. This construct, when expressed in cells with a reduced activity and/or expression of the miR-143 or miR-145 microRNAs, will lead to selective ablation of these cells.

In the case of a non-naturally occurring target site, the target site for introduction into the nucleic acid may be produced by a method known in the art, such as chemical synthesis. For example, phosphorothioate oligonucleotides may be synthesized by the method as described in Stein et al. (1988) Nucl. Acids Res. 16: 3209. The target site may then be introduced into the nucleic acid by a method known in the art. For example, complementary oligonucleotides containing the binding site may be annealed and then introduced into the appropriate restriction site in a plasmid.

The nucleic acid with the capacity to modulate development of a cell may include more than one copy of a target site for binding of the miRNA. For example, the nucleic acid may contain 2,3, 4 or more copies of a target site. Indeed, it is anticipated that multiple copies of a target site may provide a greater degree of control of the level of expression of the nucleic acid with the capacity to modulate development of the cell.

It will also be appreciated that the copies of the target site may be copies of the same or a similar target sequence, or one or more copies of a target sequence for one or more different microRNA(s).

The present invention also provides a cell including an exogenous nucleic acid including a binding site for a microRNA, or a cell including a nucleic acid with a non-naturally occuring binding site for a microRNA.

In one form, the cell is a cancerous or pre-cancerous cell with a reduced activity of a microRNA. Examples of cancerous and pre-cancerous cells are as previously discussed herein.

Accordingly, in another form the present invention provides a cancerous or pre-cancerous cell including an exogenous nucleic acid including a binding site for a microRNA, wherein the cancerous or pre-cancerous cell has a reduced activity and/or concentration of the microRNA as compared to a similar non-cancerous cell.

The cell, such as a pre-cancerous or cancerous cell, may be present *in vitro* or *in vivo.* For example, the cell may be an isolated cell *in vitro*, or a cell present in a biological system such as an organ, tissue, or an entire organism (eg an animal or human subject).

Thus, the present invention also provides an animal or human including non-cancerous cells and cancerous cells, the non-cancerous and cancerous cells both including and/or expressing an exogenous nucleic acid with a target site for binding of a microRNA.

Accordingly, in another form the present invention provides an animal including cancerous cells, the cancerous cells including an exogenous nucleic acid including a target site for binding of a microRNA.

In one form, the target site for binding of a microRNA is a target site for a microRNA that has reduced expression and/or activity in the cancerous cell, as compared to a similar non-cancerous cell. Examples of such binding sites are as previously herein discussed and include the binding site for the miR-143 and/or miR-145 microRNAs.

In one form, the exogenous nucleic acid has the capacity to modulate the development of a cell in the animal. Examples of such nucleic acids are as previously discussed herein.

The expression of an exogenous nucleic acid in a cell may be by way of introducing the nucleic acid into the cells by a method known in the art. Methods for introducing exogenous DNAs into prokaryotic and eukaryotic cells are essentially as described in Sambrook, J, Fritsch, E.F. and Maniatis, T. Molecular Cloning: A Laboratory Manual 2nd. ed. Cold Spring Harbor Laboratory Press, New York. (1989).

In the case of an animal, the animal may also be a transgenic animal with the nucleic acid stably integrated into the genome of the cells of the animal. Methods for producing transgenic animals are known in the art.

The present invention also provides a nucleic acid that has the capacity to modulate development of a cell and which includes a binding site for a microRNA.

Nucleic acids in the various forms of the present invention may be produced by a method known in the art, such as cloning, in vitro transcription (for a RNA), chemical synthesis or any combination of such methods. The present invention also provides vectors including the nucleic acids of the present invention, and cells including the vectors and nucleic acids.

Examples of nucleic acids with the capacity to modulate the development of cells are as previously discussed herein. Thus, the nucleic acid may have the capacity to inhibit the development of a cell (eg cytostatic or cytotoxic activity), or alternatively, may have the capacity to promote development of a cell.

Accordingly, in another form the present invention provides a nucleic acid with the capacity to modulate development of a cell, the nucleic acid including a binding site for a microRNA. Examples of binding sites for microRNAs are as previously discussed herein.

In one form, the binding site for the microRNA is a binding site for a microRNA that has an altered activity and/or concentration in a cell, as compared to another cell.

Thus, the nucleic acid may include a binding for a microRNA that is differentially expressed and/or differentially active.

The nucleic acid may have the capacity to either inhibit or promote development of a cell. Examples of nucleic acids that have the capacity to modulate development are as discussed previously herein.

It will be appreciated that the nucleic acids of the present invention may be used in a composition for exposure to a cell, so as to introduce the nucleic acid into the cell, or a composition for adminstration to an animal or human subject, or be part of a vector, such as a viral vector, for introducing the nucleic acids into cells *in vitro,* or cells in a biological system, such as cells in an animal or human subject.

In this case, the nucleic acid may be used for example to inhibit the development of a cell(s) in an animal or human subject, such as ablating the cell(s) in an animal or human subj ect.

The nucleic acid in the various forms of the present invention may be an isolated nucleic acid. In this regard, the term "isolated" is to be understood to mean an entity, for example a nucleic acid, a polypeptide, or a cell, which is removed from its natural environment.

The nucleic acid in the various forms of the present invention may also be present and/or expressed in a cell.

Accordingly, in another form the present invention also provides a cell including an exogenous nucleic with the capacity to modulate development of the cell, the nucleic acid including a binding site for a microRNA.

The cell may be a prokaryotic cell or a eukaryotic cell.

An example of a suitable prokaryotic cell is *Escherichia coli.* Such a cell is useful for maintaining and/or propagating plasmids including the nucleic acid. An example of a suitable eukaryotic cell is a human colon cell, or cell derived therefrom.

In one form, the cell is a eukaryotic cell that has an altered level and/or activity of one or more microRNAs. Examples of such cells are as previously discussed herein.

The cell may be an isolated cell *in vitro,* or a cell present in a biological system such as an organ, tissue, or an entire organism (eg an animal or human subject).

In one form, the cell is a cancerous or pre-cancerous cell with a reduced activity of a microRNA.

Accordingly, in another form the present invention provides a cancerous or pre-cancerous cell with reduced activity of a microRNA, the cancerous or pre-cancerous cell including an exogenous nucleic with the capacity to modulate development of the cell, the nucleic acid including a binding site for the microRNA. The reduced activity of the microRNA is as compared to a similar non-cancerous cell.

The cancerous or pre-cancerous cell may be an isolated cell *in vitro,* or a cell present in a biological system such as an organ, tissue, or an entire organism (eg an animal or human subject).

For example, the cells may be present in a whole animal or human that contains non-cancerous cells and cancerous cells, both of which express an exogenous nucleic with the capacity to modulate development of the cell, the nucleic acid including a binding site for a microRNA.

Thus, the present invention also provides an animal including non-cancerous cells and cancerous cells, the non-cancerous and cancerous cells both including and/or expressing an exogenous nucleic acid with the capacity to modulate development of the cell, the nucleic acid including a binding site for a microRNA.

In one form, the binding site for the microRNA is a binding site for a microRNA that has an altered activity and/or concentration in the cancerous cells as compared to the non-cancerous cells.

The present invention also provides a nucleic acid including a non-naturally occurring binding site for a microRNA that is differentially expressed and/or has differential activity.

Accordingly, in another form the present invention provides a nucleic acid including a non-naturally occurring binding site for a microRNA, wherein the microRNA is differentially expressed or differentially active between cells.

The nucleic acid may be an isolated nucleic acid. The nucleic acid may be present and/or expressed in a cell.

The binding site for the differentially expressed or active microRNA is a binding site of a microRNA that has altered expression and/or activity between different types of cells. In one form, the cells are of a similar type.

Examples of differentially expressed microRNAs include the miR-143 and miR-145 microRNAs, which are differentially expressed between colonic tumours and normal colonic tissue.

In one form, the binding site is a non-naturally occurring binding site for a microRNA that has an altered expression and/or activity in cancerous or pre-cancerous cells, as compared to the level of expression and/or activity in normal or non-cancerous cells. For example, the microRNA may have a reduced expression and/or activity in cancerous or pre-cancerous cells, as compared to the level of expression and/or activity in normal or non-cancerous cells.

Accordingly, in another form the present invention provides an isolated nucleic acid including a non-naturally occurring binding site for a microRNA that is downregulated in a cancerous cell as compared to a similar non-cancerous cell.

In one form, the nucleic acid is a nucleic acid with the capacity to modulate development of a cell. Nucleic acids that have the capacity to modulate the development of a cell are as previously discussed herein. Thus, the nucleic acid may inhibit (eg have cytotoxic or cytostatic activity) or promote the development of a cell.

In one form, the nucleic acid includes two or more binding sites for binding of the same or different microRNAs that are differentially expressed and/or differentially active.

It will be appreciated that the nucleic acid may be used in a composition for exposure to a cell, so as to introduce the nucleic acid into the cell, or for adminstration to an animal or human subject, or be part of a vector, such as a viral vector, for introducing into cells, including cells in an animal or human subject.

In one form, the nucleic acid is used to inhibit the development of a cell(s) in an animal or human subject, inclidng the ablation of the cell(s) in an animal or human subject.

Methods for the design of target sites for microRNAs are as previously discussed herein.

Methods for determining whether the binding site occurs naturally are known in the art.

For example, the BLAST algorithm can be used for determining the extent of nucleotide homology between a target sequence and sequences in a specific genome. BLAST identifies local alignments between the sequences in the database and predicts the probability of the local alignment occurring by chance. The BLAST algorithm is as described in Altschul et al. (1990) J. Mol. Biol. 215:403-410.

The nucleic acid may be an exogenous nuclec acid introduced into a cell and then expressed.

Accordingly, the present invention also provides a cell including a nucleic acid including a non-naturally occurring binding site for a microRNA that is differentially expressed and/or differentially active.

The cell may be a prokaryotic cell or a eukaryotic cell.

An example of a suitable prokaryotic cell is *Escherichia coli.* Such a cell is useful for maintaining and/or propagating plasmids including the nucleic acid.

In one form, the cell is a eukaryotic cell that has an altered level and/or activity of one or more microRNAs. Examples of such cells are as previously described herein.

The cell may be an isolated cell *in vitro*, or a cell present in a biological system such as cell present in an organ, tissue, or an entire organism (eg an animal or human subject).

In one form, the cell is a cancerous or pre-cancerous cell.

Accordingly, in another form the present invention provides a cancerous or pre-cancerous cell including a nucleic acid including a non-naturally occurring binding site for a microRNA.

In one form, the cell is a cancerous or pre-cancerous cell with a reduced activity of a microRNA.

Accordingly, in another form the present invention provides a cancerous or pre-cancerous cell with reduced activity of a microRNA, the cancerous or pre-cancerous cell including a nucleic acid including a non-naturally occurring binding site for a microRNA that is differentially expressed and/or differentially active in the cancerous or pre-cancerous cell as compared to a similar non-cancerous cell.

For example, the cells may be present in a whole animal or human that contains non-cancerous cells and cancerous cells, either or both of which include and/or express a nucleic acid including a non-naturally occurring binding site for a microRNA.

Accordingly, in another form the present invention provides an animal including cancerous cells, the cancerous cells including a nucleic acid including a non-naturally occurring binding site for a microRNA.

In one form, the microRNA is differentially expressed or differentially active in the cancerous cells as compared to non-cancerous cells.

Accordingly, in another form the present invention provides an animal including non-cancerous cells and cancerous cells, the non-cancerous and cancerous cells both including and/or expressing a nucleic acid including a non-naturally occurring binding site for a microRNA that is differentially expressed *or differentially active* in the cancerous cell as compared to the non-cancerous cell.

The nucleic acids of the present invention may be introduced into a cell by a suitable method known in art. For example, the nucleic acid may be introduced into a cell by transformation using calcium phosphate, viral infection, electroporation, lipofection, or particle bombardment. In this regard, transformed cells include stably transformed cells in which the inserted DNA is capable of replication either as an autonomously replicating plasmid or as part of the host chromosome, or cells which transiently express the inserted DNA or RNA for limited periods of time. Methods for introducing exogenous DNAs into prokaryotic and eukaryotic cells are essentially as described in Sambrook, J, Fritsch, E.F. and Maniatis, T. Molecular Cloning: A Laboratory Manual 2nd. ed. Cold Spring Harbor Laboratory Press, New York. (1989).

In the case of the nucleic acid being a mRNA, the mRNA may be produced in the cell by transcription of the relevant DNA. Alternatively, the mRNA may be an exogenous mRNA introduced into the cell. Methods for producing mRNAs *in vitro* are known in the art.

For a mRNA expressed in the cell from a DNA template, the target site may be cloned into a suitable expression vector for use in the cell type of interest by methods known in the art. Methods for the isolation of nucleic acid sequences and their cloning into a suitable expression vector are essentially as described in Sambrook, J, Fritsch, E.F. and Maniatis, T. Molecular Cloning: A Laboratory Manual 2nd. ed. Cold Spring Harbor Laboratroy Press, New York. (1989). The recombinant molecule may then be introduced into the cell and the cloned nucleic acid expressed. The vector may be any nucleic acid capable of having a foreign nucleic acid inserted into the vector. For example, the vector may be a plasmid, all or part of a viral genome, or any other nucleic acid capable of autonomous replication in a prokaryotic or eukaryotic host.

The present invention also provides a vector including the various nucleic acids of the present invention.

In one form, the vector is a viral vector that allows the nucleic acid to be introduced into the target cells by infection. Examples of such viral vectors that can be employed to deliver the nucleic acid to a cell include a recombinant adenovirus, a recombinant lentivirus, a recombinant retrovirus, a recombinant adeno-associated virus (AAV), a recombinant herpesvirus, a recombinant SV-40 virus, an Epstein-Barr virus, or a recombinant pox virus, such as a recombinant vaccinia virus.

Accordingly, in one form the present invention provides a viral vector including a nucleic acid with the capacity to modulate development of a cell, the nucleic acid including a binding site for a microRNA.

In another form, the present invention also provides a viral vector including a nucleic acid including a non-naturally occurring binding site for a differentially expressed microRNA.

In this case, it will be appreciated that the viral vector may be formed from all or part of a viral genome. The viral vector may also be a naturally occurring or a recombinant virus and further may be replication deficient or replication proficient.

As will be appreciated, expression of the relevant inserted DNA in plasmid or viral vectors will generally require various regulatory elements known in the art for the expression of inserted nucleic acids, for example promoters for driving the expression of an inserted nucleic acid in a particular cell, poly A signals for efficient polyadenylation of mRNA transcribed from inserted nucleic acids, or other regulatory elements to control translation, transcription or mRNA stability.

Depending upon the cell type to be modulated, the promoter driving the expression may be a constitutive promoter, an inducible promoter or a cell or tissue specific promoter. Constitutive mammalian promoters include hypoxanthine phosphoribosyl transferase (HPTR), adenosine deaminase, phosphoglycerate kinase, pyruvate kinase, and β-actin. Exemplary viral promoters which function constitutively in eukaryotic cells include promoters from the simian virus, papilloma virus, adenovirus, human immunodeficiency virus (HIV), Rous sarcoma virus, cytomegalovirus, the long terminal repeats (LTR) of moloney leukemia virus and other retroviruses, and the thymidine kinase promoter of herpes simplex virus.

Inducible promoters include synthetic promoters regulated by the TetO/TetR system and inducible promoters such as metallothionein promoter, which may be used to induced transcription in the presence of certain metal ions. Other inducible promoters are known in the art.

The tissue-specific promoter will depend upon the particular cell type. For example, promoters that allow expression in colon cancer cells include the regulatory sequences of human carcinoembryonic antigen (CEA) [accession:U17131; gi|967132].

The present invention also provides a composition including the various nucleic acids described herein.

Accordingly, in one form the present invention also provides a composition including a nucleic acid with the capacity to modulate development of a cell, the nucleic acid including a binding site for a microRNA.

In another form, the present provides a composition including a nucleic acid including a non-naturally occurring binding site for a differentially expressed or differentially active microRNA.

The compositions are suitable for exposing the nucleic acids of the present invention to cells. Methods of introducing nucleic acids into cells are as previously discussed herein. The compositions of the present invention are also suitable for administration to a subject to prevent and/or treat a disease, condition *or state* associated with cells that have an altered activity and/or expression of a microRNA.

For example, the nucleic acids may be combined with a pharmaceutically acceptable carrier, stabilizer, buffer or diluent to produce a composition for administration to a subject. Thus, the present invention also provides a pharmaceutical composition including one or more nucleic acids of the invention in an acceptable carrier.

The nucleic acid may be delivered to a cell or a subject by a method known in the art. For example, the nucleic acid molecules can be administered to cells by encapsulation in liposomes, by iontophoresis, or by incorporation into other vehicles, such as biodegradable polymers, hydrogels, cyclodextrins, poly(lactic-co-glycolic)acid (PLGA) and PLCA microspheres, biodegradable nanocapsules, and bioadhesive microspheres.. The nucleic acid molecules may also be formulated or complexed with polyethyleneimine and derivatives thereof, such as polyethyleneimine-polyethyleneglycol-N-acetylgalactosamine (PEI-PEG-GAL) or polyethyleneimine-polyethyleneglycol-tri-N-acetylgalac- tosamine (PEI-PEG-triGAL) derivatives.

For administration to a subject, the nucleic acids can be administered and introduced by standard means, with or without stabilizers, buffers, and the like, to form a pharmaceutical composition. Suitable forms, in part, depend upon the use or the route of entry, for example oral, transdermal, by injection, or delivery by way of infection with a virus.

When it is desired to use a liposome delivery mechanism, standard protocols for formation of liposomes can be followed. The compositions of the present invention can also be formulated and used as tablets, capsules or elixirs for oral administration, suppositories for rectal administration, sterile solutions, suspensions for injectable administration, and the other compositions known in the art.

Surface-modified liposomes containing poly (ethylene glycol) lipids (PEG-modified, or long-circulating liposomes or stealth liposomes) offer a method for increasing the accumulation of drugs in target tissues. Such liposomes have been shown to accumulate selectively in tumors.

Administration routes that lead to systemic absorption include intravenous, subcutaneous, intraperitoneal, inhalation, oral, intrapulmonary and intramuscular routes. Each of these administration routes exposes the nucleic acids of the present invention to cells or tissue.

The nucleic acids of the present invention will generally be delivered at a pharmaceutically effective dose to prevent, inhibit the occurrence, or treat (so as to alleviate a symptom to some extent) a disease, condition or state. The pharmaceutically effective dose depends on the type of disease or condition being treated, the composition used, the route of administration, the type of subject being treated, the physical characteristics of the specific subject under consideration, concurrent medication, and other factors that those skilled in the medical arts will recognize. Generally, an amount between 0.1 mg/kg and 100 mg/kg body weight/day of active ingredients is administered.

The nucleic acid molecules of the present invention, and compositions and formulations thereof, can be administered orally, topically, parenterally, by inhalation or spray, or rectally in dosage unit formulations containing conventional non-toxic pharmaceutically acceptable carriers, adjuvants and/or vehicles. The term "parenteral" includes percutaneous, subcutaneous, intravascular (e.g., intravenous), intramuscular, or intrathecal injection or infusion techniques and the like.

As discussed previously, the present invention also provides a pharmaceutical composition including a nucleic acid molecule of the invention and a pharmaceutically acceptable carrier. One or more nucleic acid molecules of the invention can be present in association with one or more non-toxic pharmaceutically acceptable carriers and/or diluents and/or adjuvants, and if desired other active ingredients. The pharmaceutical compositions containing nucleic acid molecules of the invention can be in a form suitable for oral use, for example, as tablets, troches, lozenges, aqueous or oily suspensions, dispersible powders or granules, emulsion, hard or soft capsules, or syrups or elixirs.

Compositions intended for oral use can be prepared according to a suitable method known in the to the art. The composition may contain one or more such sweetening agents, flavoring agents, coloring agents or preservative agents in order to provide a pharmaceutically acceptable preparations. Tablets contain the active ingredient in admixture with non-toxic pharmaceutically acceptable excipients that are suitable for the manufacture of tablets. These excipients can be, for example, inert diluents; such as calcium carbonate, sodium carbonate, lactose, calcium phosphate or sodium phosphate; granulating and disintegrating agents, for example, corn starch, or alginic acid; binding agents, for example starch, gelatin or acacia; and lubricating agents, for example magnesium stearate, stearic acid or talc. The tablets can be uncoated or they can be coated by known techniques. In some cases such coatings can be prepared by known techniques to delay disintegration and absorption in the gastrointestinal tract and thereby provide a sustained action over a longer period. For example, a time delay material such as glyceryl monosterate or glyceryl distearate can be employed.

Formulations for oral use can also be presented as hard gelatin capsules, wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in a mixture with excipients suitable for the manufacture of aqueous suspensions. Such excipients are suspending agents, for example sodium carboxymethylcellulose, methylcellulose, hydropropyl-methylcellulose, sodium alginate, polyvinylpyrrolidone, gum tragacanth and gum acacia; dispersing or wetting agents can be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions can also contain one or more preservatives, for example ethyl, or n-propyl p-hydroxybenzoate, one or more coloring agents, one or more flavoring agents, and one or more sweetening agents, such as sucrose or saccharin.

Oily suspensions can be formulated by suspending the active ingredients in a vegetable oil, for example arachis oil, olive oil, sesame oil or coconut oil, or in a mineral oil such as liquid paraffin. The oily suspensions can contain a thickening agent, for example beeswax, hard paraffin or cetyl alcohol. Sweetening agents and flavoring agents can be added to provide palatable oral preparations. These compositions can be preserved by the addition of an anti-oxidant such as ascorbic acid

Dispersible powders and granules suitable for preparation of an aqueous suspension by the addition of water provide the active ingredient in admixture with a dispersing or wetting agent, suspending agent and one or more preservatives. Suitable dispersing or wetting agents or suspending agents are exemplified by those already mentioned above. Additional excipients, for example sweetening, flavoring and coloring agents, can also be present.

Pharmaceutical compositions of the present invention can also be in the form of oil-in-water emulsions. The oily phase can be a vegetable oil or a mineral oil or mixtures of these. Suitable emulsifying agents can be naturally-occurring gums, for example gum acacia or gum tragacanth, naturally-occurring phosphatides, for example soy bean, lecithin, and esters or partial esters derived from fatty acids and hexitol, anhydrides, for example sorbitan monooleate, and condensation products of the said partial esters with ethylene oxide, for example polyoxyethylene sorbitan monooleate. The emulsions can also contain sweetening and flavoring agents.

Syrups and elixirs can be formulated with sweetening agents, for example glycerol, propylene glycol, sorbitol, glucose or sucrose. Such formulations can also contain a demulcent, a preservative and flavoring and coloring agents. The pharmaceutical compositions can be in the form of a sterile injectable aqueous or oleaginous suspension. This suspension can be formulated as known in the art using suitable dispersing or wetting agents and suspending agents that have been discussed previoulsy. The sterile injectable preparation can also be a sterile injectable solution or suspension in a non-toxic parentally acceptable diluent or solvent, for example as a solution in 1,3-butanediol. Among the acceptable vehicles and solvents that can be employed are water, Ringer's solution and isotonic sodium chloride solution. In addition, sterile, fixed oils are conventionally employed as a solvent or suspending medium. For this purpose, any bland fixed oil can be employed including synthetic mono-or diglycerides. In addition, fatty acids such as oleic acid find use in the preparation of injectables.

The nucleic acid molecules of the present invention can also be administered in the form of suppositories, e.g., for rectal administration of the drug. These compositions can be prepared by mixing the nucleic acid with a suitable non-irritating excipient that is solid at ordinary temperatures but liquid at the rectal temperature and will therefore melt in the rectum to release the nucleic acid. Such materials include cocoa butter and polyethylene glycols.

The nucleic acid molecules of the present invention can also be administered parenterally in a sterile medium. The nucleic acid, depending on the vehicle and concentration used, can either be suspended or dissolved in the vehicle. Advantageously, adjuvants such as local anesthetics, preservatives and buffering agents can be dissolved in the vehicle.

It is understood that the specific dose level for any particular subject will depend upon a variety of factors including the age, body weight, general health, time of administration, route of administration, and rate of excretion, drug combination and the severity of the particular disease undergoing therapy.

For administration to non-human animals, the composition can also be added to the animal feed or drinking water. It can be convenient to formulate the animal feed and drinking water compositions so that the animal takes in a therapeutically appropriate quantity of the composition along with its diet. It can also be convenient to present the composition as a premix for addition to the feed or drinking water.

The nucleic acid molecules of the present invention can also be administered to a subject in combination with other therapeutic compounds to increase the overall therapeutic effect. The use of multiple compounds to treat an indication can increase the beneficial effects while reducing the presence of side effects.

It will be appreciated that the various compositions of the present invention can also be formulated for administering the nucleic acid molecules of the invention to specific cell types. For example, receptors such as the asialoglycoprotein receptor are unique to hepatocytes and bind branched galactose-terminal glycoproteins, such as asialoorosomucoid. Alternatively, some receptors such as the folate receptor are overexpressed in many cancer cells. The use of galactose, galactosamine, or folate based conjugates to transport exogenous compounds across cell membranes can provide a targeted delivery approach to, for example, the treatment of liver disease, cancers of the liver, or other cancers.

The nucleic acid molecules of the present invention may also be complexed with or covalently attached to nanoparticles, such as Hepatitis B virus or L envelope proteins.

Alternatively, certain the nucleic molecules of the present invention can be expressed within cells from eukaryotic promoters as previously discussed.

Viral vectors expressing the nucleic acids of the present invention may be constructed based upon, for example, adeno-associated virus, retrovirus, adenovirus, or alphavirus. The viral vectors can either be use to introduce the nucleic acid into cells, or alternatively, the viral vector can be packaged and infections used to introduce the nucleic acids of the present invention to cells. The viral vectors can provide for transient expression or stable expression of the nucleic acid molecules.

Therapeutic delivery of biolomolecules is generally as described in Bladon, C. (2002) "Pharmaceutical Chemistry: Therapeutic Aspects of Biomolecules" John Wiley & Sons Ltd.

Viral and gene therapy techniques are as generally described in "Viral Vectors for Gene Therapy: Methods and Protocols" Edited by Jules G Constant, Curtis A Machida (2003) Humana Press Inc., "Gene Delivery to Mammalian Cells: Viral Gene Transfer Techniques" Edited by William C Heiser (2004) Humana Press Inc., "Viruses in Human Gene Therapy" Edited by J.H. Vos (1995) Carolina Academic Press, and "Viral Therapy Of Human Cancers" Edited by J.G. Sinkovics and J.C. Horwath (2005) Marcel Dekker.

The present invention is also suitable for modulating the concentration of a nucleic acid in a cancerous cell or pre-cancerous cell.

In this case, an altered activity and/or concentration of a microRNA in a cancerous cell allows the concentration of the nucleic acid in the cell to be modulated by including in the nucleic acid a target site for binding of the microRNA. As will be appreciated, the modulation of the concentration of the nucleic acid is as compared to a similar cell not having an altered activity and/or expression of the microRNA.

For example, a reduced activity and/or concentration of a microRNA in a cancerous cell allows the concentration of the nucleic acid in the cell to be modulated by including in the nucleic acid a target site for binding of the microRNA.

Accordingly, the present invention also provides a method of modulating the concentration of a nucleic acid expressed in a cancerous cell, the cancerous cell having an altered activity and/or concentration of a microRNA as compared to a similar non-cancerous cell, the method including the step of introducing a target site for binding of the microRNA into the nucleic acid to be expressed in the cell.

Methods for cloning and introducing nucleic acids into cells are as previously discussed. Determination that the concentration of a nucleic acid may be accomplished by a suitable method known in the art, such as Northern analysis, RT-PCR or RNase protection.

The present invention is also suitable for detecting an altered microRNA activity and/or concentration in a cancerous or pre-cancerous cell, such as a cancerous cell in vitro, or a cancerous or pre-cancerous cell in an animal or human. In this case, the cancerous cells and non-cancerous cells both express a reporter nucleic acid including a target site for binding of a microRNA, and an altered activity of the microRNA may be detected in the cancerous cells by determining the level of expression of the reporter nucleic acid in the cancerous and non-cancerous cells, and detecting a change in the activity of the microRNA in the cancerous cells by a change in the expression of the reporter nucleic acid in the cancerous cells as compared to the level of expression of the reporter nucleic acid in the non-cancerous cells.

For example, the present invention is suitable for detecting a reduced microRNA activity and/or concentration in a cancerous or pre-cancerous cell. a reduced activity of the microRNA may be detected in the cancerous cells by determining the level of expression of the reporter nucleic acid in the cancerous and non-cancerous cells, and detecting a reduced activity of the microRNA in the cancerous cells by an increase in the expression of the reporter nucleic acid in the cancerous cells as compared to the level of expression of the reporter nucleic acid in the non-cancerous cells.

Accordingly, in another form the present invention provides a method of detecting altered microRNA activity and/or concentration in a cancerous or pre-cancerous cell, the method including the steps of:
determining the level of expression of a reporter nucleic acid in the cancerous or pre-cancerous cells and determining the level of expression of a reporter nucleic acid in non-cancerous cells; and
detecting a reduced activity of the microRNA in the cancerous cells by an increase in the expression of the reporter nucleic acid in the cancerous cells as compared to the level of expression of the reporter nucleic acid in the non-cancerous cells.

In one form, the cancerous cells are present in an animal or human subj ect.

The non-cancerous cell may be the same or similar cells. In one embodiment, the cancerous (or precancerous cells) and the non-cancerous cells are both present in an animal or human subj ect.

In one form, the method may be used to detect a reduced activity of the microRNA by detecting an increase in the expression of the reporter nucleic acid in the cancerous cells as compared to the level of expression of the reporter nucleic acid in the non-cancerous cells.

In one form, the reporter nucleic acid in the cancerous (or pre-cancerous cells) is the same or a similar reporter nucleic acid as present in the non-cancerous cells.

Suitable reporter nucleic acids are known in the art. For example, the reporter nucleic acid may produce a detectable product such as LacZ, or GFP. Alternatively, the reporter nucleic acid may be detected by an immunological detection method, with use of antibodies raised to the protein encoded by the reporter nucleic acid. Another method of detecting the reporter nucleic acid is by use of hybridization with a detectably labelled complementary nucleic acid probe.

Finally, standard techniques may be used for recombinant DNA technology, oligonucleotide synthesis, and tissue culture and transfection (e.g., electroporation, lipofection). Enzymatic reactions and purification techniques may be performed according to manufacturer's specifications or as commonly accomplished in the art or as described herein. The foregoing techniques and procedures may be generally performed according to conventional methods well known in the art and as described in various general and more specific references that are cited and discussed throughout the present specification. See e.g., Sambrook et al. Molecular Cloning: A Laboratory Manual (2d ed., Cold Spring Harbor Laboratory Press, Cold Spring Harbor, N.Y. (1989) and Ausubel, F. M. et al. (1989) Current Protocols in Molecular Biology, John Wiley & Sons, New York, N.Y.

### Description of the Preferred Embodiments

Reference will now be made to experiments that embody the above general principles of the present invention. However, it is to be understood that the following description is not to limit the generality of the above description.

### Example 1

### RNA Isolation From Tissue Samples and Cell Lines

Cell lines may be maintained in an appropriate medium. Colorectal tumors and the corresponding normal mucosae may be obtained from fresh surgical resections, following informed consent from patients, and then classified according to standard histopathological classification methods.

RNA may isolated from cell lines, using Trizol reagent (Invitrogen, Carlsbad, CA) according to the manufacturer's instructions. RNA may be purified from colorectal tissues using the procedure of Chomczynski, P. and Sacchi, N. (1987). Anal. Biochem. 162: 156-159.

### Example 2

### Cloning of MicroRNAs

miRNAs may be cloned essentially as described by Elbashir et al. (2001) Genes Dev. 15: 188-200, except that nucleic acids may be electroeluted from acrylamide gel slices using the Biotrap system (Schleicher and Schuell GmbH, Dassel, Germany). Briefly, small RNA fractions of between 18 and 26 bases may be size selected on a denaturing polyacrylamide gel. Adapter oligonucleotides, containing *Eco*RI restriction sites, may then be directionally ligated to the RNA molecules. The adapter-ligated RNA may then be amplified by RT-PCR. Concatamerized fragments, containing multimers of religated, *Eco*RI-digested PCR products, between 200 and 650 bp, are size selected on an agarose gel and recovered by electroelution. The concatamers may then be end-repaired and dA-tailed with Taq DNA polymerase, then cloned into pGEM T-easy (Promega, Madison, WI) or pTOPO (Invitrogen) according to the manufacturers' instructions. Plasmid inserts from the resultant colonies may be analyzed by PCR using primers to vector sequences. The nucleic acid sequence of selected inserts may then be determined following treatment of the PCR products with Exonuclease I and Shrimp Alkaline Phosphatase according to the ExoSAP-IT protocol (USB Corporation, Cleveland, OH). Clones created by this procedure will contain concatamers of PCR products, and generally likely to represent between two and five independent small RNAs.

### Example 3

### Northern Analysis

Total RNA (20 µg) may be separated on a 15% denaturing polyacrylamide gel. Loadings are visualized by ethidium bromide staining. The RNA may then be transferred to Hybond N+ nylon membrane by semi-dry blotting (OWL Separation Systems, Portsmouth, NH). Probes may be generated by T4 Polynucleotide Kinase (New England Biolabs, Beverly, MA) mediated end-labeling of DNA oligonucleotides with [γ-32P]ATP. To increase the specific activity of the probes, the miRNA sequence may be concatamerized as a trimer of direct repeats, then cloned into pGEM T-easy and the insert amplified using PCR with M13 forward and reverse primers. Antisense probes may then be synthesized using Taq polymerase-generated linear amplification from the Sephadex G-50-purified PCR products to incorporate multiple [α-32P]dCTP bases.

Filter hybridization may be performed in QuikHyb Solution (Stratagene, La Jolla, CA) containing 10⁶ cpm/ml probe for 1 h, with washes, as per the manufacturers' recommendations. Filters may be analyzed using a Fujifilm-BAS 2500 phoshorimager and signal intensity quantitated (as photostimulated luminescence/mm²) using Analytical Imaging Station (version 3.0) software (Imaging Research Inc., Brock University, Ontario, Canada). miRNA sequences may be identified by BLAST (as described in Altschul et al. (1990) J. Mol. Biol. 215: 403-410) by comparison to the Genbank and EMBL public nucleotide databases. MicroRNAs may also be identified by comparison with the databases of the miRNA registry. The secondary structures of putative pre-miRNA hairpins may be determined using the Mfold 3.1 algorithm (as described in Mathews et al. (1999) J. Mol. Biol. 288: 911-940). Potential mRNA target sequences may be identified by searching the Genbank nonredundant and dbEST databases using BLAST and FASTA algorithms (as described in Pearson, W. R. and Lipman, D. J. (1988) Proc. Natl. Acad. Sci. USA, 85: 2444-2448) algorithms.

### Example 4

### Identification of Colorectal MicroRNAs

Small RNA fragments (between 18 and 27 bases) in total RNA, purified from both a colonic adenocarcinoma and its matched normal mucosa, may be size fractionated and cloned. The clones from the cancer-derived sample and clones representing normal mucosa may then be sequenced. Sequence analysis and comparison with public database nucleotide sequences will enable identification of many of the clones or assignment to a possible genomic origin for the transcripts.

### Example 5

### Accumulation of MicroRNAs in Colorectal Tissues and in cancer cell lines

To confirm that the various sequences accumulate as miRNAs and investigate whether changes in miRNA steady-state levels are associated with neoplastic epithelium, Northern blot analysis may be undertaken against a panel of RNAs from matched colorectal cancer and normal mucosa specimens.

Northern blot analysis may be used to determine the levels of mature miRNAs and precursor hairpin molecules in cell lines derived from a variety of cancerous human tissues.

### Example 6

### Cloning of the miR145 target sequence into GFP

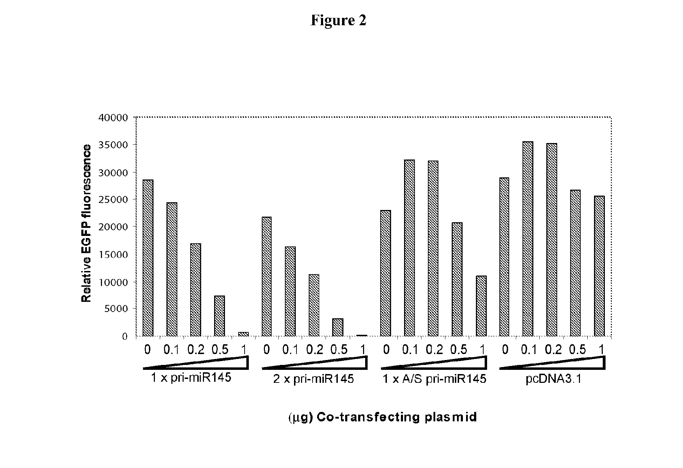

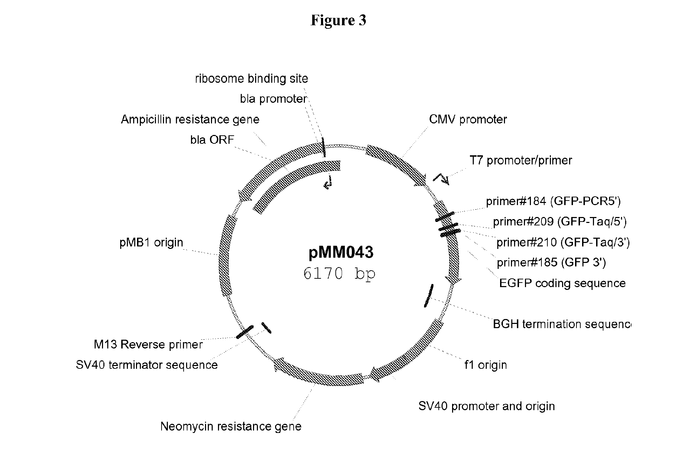

A mammalian Enhanced Green Fluorescence Protein (EGFP) expression cassette (pMM043; Figure 2) was created by directionally inserting the EGFP coding sequence from pEGFP1 (Clontech) as a *Bgl*II/*Not*I fragment, into *Bam*HI*lNot*I linearized pcDNA3.1(+) (Invitrogen). The unique *Not*I and *Xba*I sites in the reporter gene 3' untranslated region provided convenient sites for the insertion of miRNA-complementary and predicted *in vivo* target sequences. pMM095 (Figure 3) was created by annealing the oligonucleotides #527 (5'-CTAGCAGATCCTGGGAAAACTGGAC-3'; SEQ ID NO.1) and #528 (5'-CTAGGTCCAGTTTTCCCAGGATCTG-3'; SEQ ID NO.2), then ligating the hybrid, which contains the predicted RICS gene miR145-target sequence, into the XbaI site of pMM043. The nucleotide sequence of pMM095 is provided in the sequence listing and is designated SEQ ID NO. 153.

### Example 7

### Expression of GFP with the miR145 target sequence is repressed in cells that overexpress the miR145 precursor molecule

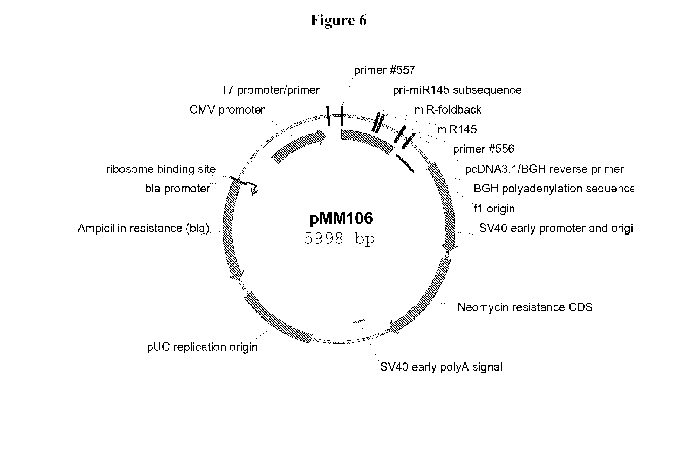

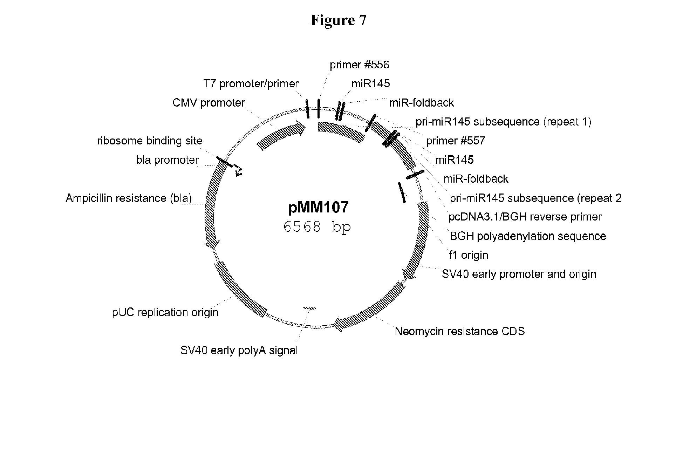

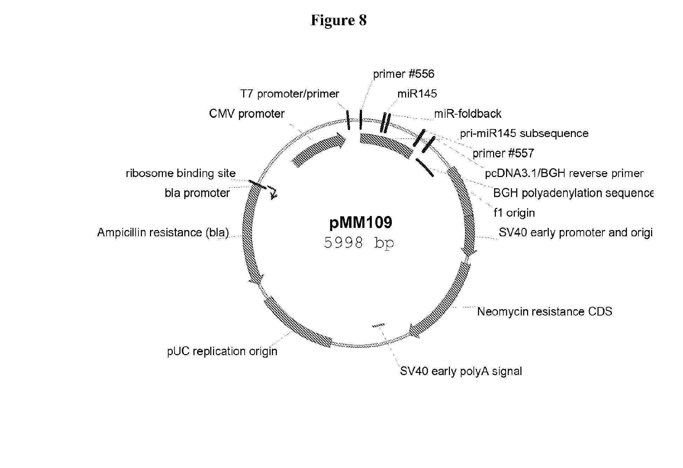

(i) Pri-miR145 expression constructs:
   A fragment of the pri-miR145 transcript was cloned by PCR amplification of the sequence corresponding to positions 184 and 734 of cDNA clone FLJ36638 fis (Genbank ID:21752921). The oligonucleotides used were #556 (5'-TCCGGTACTTTTCAGGGCAA-3'; SEQ ID NO.4) and #557 (5'-CAAGAAACGCATGCCTGATG-3'; SEQ ID NO.5) in a standard PCR reaction using 50 ng HeLa genomic DNA as template with cycling conditions: 94°C 3 minutes; 40 amplification cycles 94°C 30 sec., 55°C 30 sec., 72°C 1 min; 72°C 10 minutes and final extension 72°C 10 min. The 550 bp product was agarose gel purified and cloned into pGEMT-easy (Promega) to create plasmid pMM105. The EcoRI insert of pMM105 (SEQ ID NO. 154) was then ligated into EcoRI linearised pcDNA3.1(+) (Invitrogen) to create the expression constructs: pMM109 (single sense insert; Figure 8), pMM106 (single antisense insert; Figure 6), and pMM107 (tandem sense inserts; Figure 7). The nucleotide sequence of pMM106 is provided in the sequence listing and is designated SEQ ID NO. 155. The nucleotide sequence of pMM107 is provided in the sequence listing and is designated SEQ ID NO. 156. The nucleotide sequence of pMM109 is provided in the sequence listing and is designated SEQ ID NO. 157.
(ii) Transfections:
   Cotransfections of HeLa cells involved FuGene 6 (Roche)-mediated transfection of 0.1µg of pMM095 with between 0.1µg and 1µg pri-miR145 expression vector (pMM109, pMM106, pMM107) in 24 well culture plates, using standard FuGene 6 (Roche) protocols. EGFP activity was detected as direct fluorescence of live cells, 3 days following transfection, on a Typhoon fluorimager (Amersham Biosciences) and quantified using Imagequant software.

The miRNA, miR145, accumulates to only very low levels in HeLa (cervical carcinoma) cells. It was found that an enhanced Green Fluorescence Protein (EGFP) reporter gene construct containing the miR145 target sequence (from the RICS transcript) is as active in these cells as an EGFP construct lacking the target sequence, as detected by fluorescence of the transfected cells.

However, as shown in Figure 2, if cells are co-transfected with a construct that overexpresses the miR145 precursor molecule, pri-miR145, EGFP activity is severely repressed, with the repression occurring in a dose responsive manner.

### Example 8

### Incorporating miRNA target sequences into reporter and cytotoxic genes

A series of constructs will be created to contain combinations of the complementary target sequences for miR143 and miR145 in the 3'UTR of reporter genes (EGFP, LacZ, Renilla luciferase) within constitutive expression vectors. These vectors will include plasmids (for liposome mediated transfection and mouse transgenesis) and lentiviral systems.

As the contribution of independent targets sequences (or miRNA response elements; MREs) to the repression of a transcript are known to be cumulative, combinations that include up to four copies of each MRE will also be created.

The EGFP construct described previously will be used, in conjunction with clones that contain several (up to four copies of the miR145 and miR143 complementary sequences), cloned into the *Not*I site of the 3'UTR. LacZ constructs will be based on the synthetic, codon-optimised β-galactosidase sequence, described by Anson and Limberis (2004) J. Biotechnology 108: 17-30.

The LacZ plasmid vectors will be based on the pcDNA3.1(+) expression backbone and that target sequences will also be inserted into the *Not*I site of the 3'UTR.

Luciferase constructs will be created using the psiCHECK™-2 vector (Promega) by insertion of target sequences into the multiple cloning region in the 3'UTR of the synthetic Renilla luciferase gene. The psiCHECK™-2 vector also provides firefly luciferase as an internal control to normalise for transfection efficiency. Luciferase activities of transfected and transduced (in the case of lentiviral derivations) cells will be detected using the Dual Luciferase® Assay system (Promega) according to manufacturer's instructions.

### Example 9

### Effect of miRNA target sequences in cultured cells

The discovery that diseased (cancer) cells lack the repressive function provided by miR143 and miR145 allows us to address the possibility that we can exploit this phenomenon to control the expression of therapeutic genes in these cells.

Results from experiments using the RICS MRE in EGFP constructs show that the presence of miR145 will limit expression of foreign genes (that contain complementary 3'UTR sequences) in those cells.

A mammalian cell line that accumulates significant levels of mature miR143 or miR145 has not yet been identified. A lack of miR143 accumulation in cell lines has also been reported by others and is postulated to be a consequence of the control of fundamental processes (such as proliferation) by these miRNAs.

To generate systems in which these miRNAs can be induced, to mimic the status of "normal" cells, dox-inducible miR145 HeLa cell lines will be produced to allow an investigation of the stoichiometry between miR145 levels and target sequences and the ability to silence gene expression. This will enable us to determine, *in vitro,* whether a threshold level of cytoplasmic miR145 is required to suppress reporter gene (EGFP, LacZ, luciferase) activity and cytotoxic gene (herpes simplex virus thymidine kinase) function.

Doxycycline-inducible pri-miR145 expression constructs were created by incorporating the *Pme*I insert from pMM106 (containing the pri-miR145 subfragment described earlier) to displace EGFP in the Tet-inducible expression cassette pMM060 between the unique AscI (blunted) and *Pml*I sites. pMM060 utilises both the Tet-responsive promoter and tTR modified transrepressor described by Rossi et al. (1998) Nat Genet. 20(4):389-93, with the transrepressor under the control of constitutive murine phosphoglycerate kinase regulatory sequences. The construct with a single sense copy of the 550 bp pri-miR145 subsequence is called pMM110.

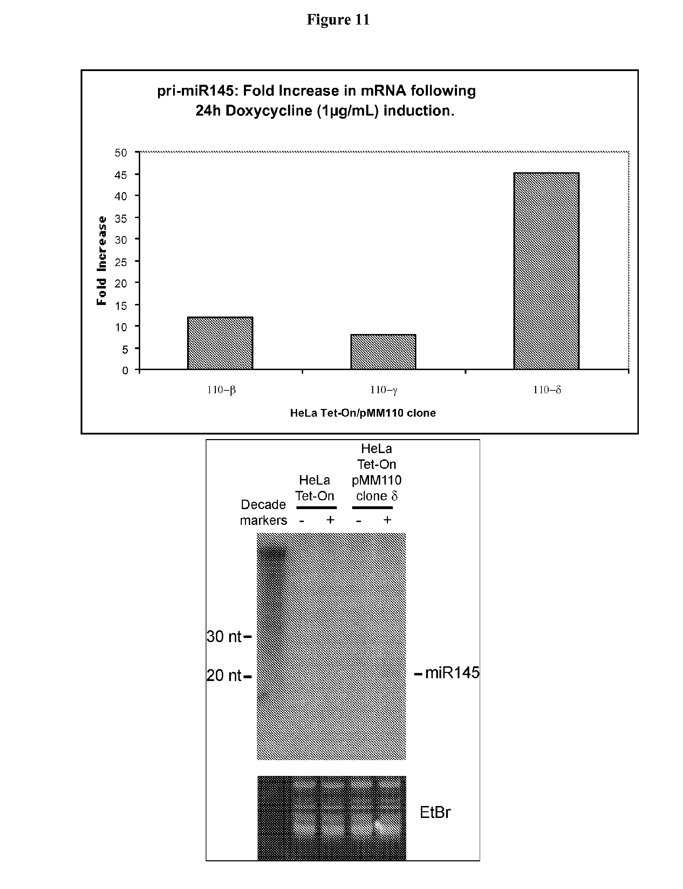

HeLa Tet-On cells (Clontech) have been stably-transformed with the pMM110 contruct using FuGene 6-mediated transfection and following selection on both genticin and puromycin, clonal lines have been isolated. Different HeLa Tet-On/pMM110 lines display varying levels of pri-miR145 induction and mature miR145 accumulation, following 24h exposure to 1µg doxycycline/mL growth medium. The data is shown in Figure 11.

### Example 10

### Effect of miRNA target sequences in primary colonic cells

It is necessary to determine whether miR143 and miR145 MREs will enable disease specific expression of transgenes in mamalian tissues. To develop a rapid assay for miR143/miR145 retarded gene expression in colonic epithelium and adenocarcinomas, the expression of LacZ (+/- multiple miR145 MREs in 3'UTR) in colonic mucosa and compare that with expression in tumour cells will be examined.

Normal murine intestinal tissues and tumours from an azoxymethane (AOM)-treated p53-knockout mouse (Hu et al. (2005) Int. J. Cancer 115: 561-567), will be maintained in culture to establish this study and also to refine the process and vectors.

Culture conditions will be essentially as described by Whitehead et al. (1999) Gastroenterology 117:858-65.

Normal intestinal mucosa, adenomatous and cancer tissues will also be obtained from the resections of consenting cancer patients and cultured as above.

Reporter gene constructs will be inserted into a lentiviral vector system as described in Anson and Fuller (2003) J. Gene Med. 5:829-838, and Fuller and Anson (2001) Human Gene Therapy 12: 2081-2093, and used to infect cultured explants.

All constructs will also incorporate a constitutive EYFP expression cassette to define and normalise the level of lentiviral infection between samples. Initial experiments will use only a constitutive EYFP expression cassette in the lentivirus vector, to establish this system. It will also be determined whether a dual luciferase assay (psiCHECK™2; Promega) is more informative, than the lacZ marker, in this system.

If this approach is successful, the *LacZ* reporter gene will be replaced with the conditional cytotoxic gene, thymidine kinase, to determine whether MRE-derived tissue specificity is sufficient to selectively ablate tumour cells. Naturally, this will lead to the creation of constructs with tissue-specific promoters to enhance disease-specificity.

### Example 11

### Effect of miRNA target sequences on gene expression in vivo

Transgenic mice will be created that express a *LacZ* reporter gene, containing multiple miR145 and miR143 complementary sequences in the 3'UTR, under the control of a constitutive promoter (CMV). The transgene construct will also comprise a second reporter gene (EGFP) that does not contain such targeting sequences, but uses the same promoter. Direct fluorescence (or immunohistochemical detection of EGFP) will define the tissue distribution of transgene expression while the subset of cells that also stain for LacZ activity will indicate those which are not affected by microRNA-mediated silencing.

Fluorescence will entail direct observation of fresh or paraformaldehyde-fixed tissues using an inverted fluorescence microscope. Fixed tissue may be pre-treated with 0.1 % sodium borohydride, to reduce autofluorescence. GFP specific antibodies (Living Colours® Peptide Antibody; Clontech) will be used for immunohistochemical detection, using standard procedures.

While the entire transgenic mouse will be assessed, particular attention will be paid to colorectal tissues.

Favourable transgenic mouse lines will be crossed with a p53 knockout mouse strain that is currently being used by the Young group to generate a murine model of colorectal cancer following administration of the carcinogen, azoxymethane (Hu et al. (2005) Int. J. Cancer 115: 561-567). The progeny of this cross will be examined for enhanced LacZ expression in tumour cells relative to surrounding epithelium.

To create transgenic mice, pronuclear injection of constructs (linear DNA fragments containing expression cassettes) into embryos isolated from pregnant, superovulated C57BL/6, females will be performed and embryos reimplanted commercially at the GenSA facility (IMVS, Adelaide). Resultant lines will be genotyped (by PCR) for appropriate genomic insertion of the injected sequences, as described in Rulicke T. and Hubscher U. (2000) Exp. Physiol. 85: 589-601.

These founder lines will be assessed for low copy number insertion of the intact introduced expression cassettes and screened for appropriate expression of the transgenes using real-time RT-PCR and immunohistochemistry. Relative copy number determinations will be made using PCR of genomic DNA and/or Southern Blot analysis. Real time RT-PCR will utilize transgene mRNA-specific primers and cDNA templates in a 1x SYBR green PCR Master Mix (Applied Biosystems) reaction. A Corbett Rotorgene 2000 (Corbett Research Pty. Ltd., Australia) will be used for PCR amplification and detection.

The final hybrid line will be inbred to ensure homozygosity for the transgenes, before cross-breeding with the p53 mutant mouse line and other murine models for cancer and polyposis.

### Example 12

### Effect of miRNA target sequences on gene expression in stem cells and their derivatives

MicroRNA target sequences in the 3'UTR of reporter and therapeutic genes will be used to assess the potential for exploiting tissue (or cell lineage)-specific microRNAs to limit transgene expression to defined cell lineages and tissues.

For example, target sequences for the neural-specific miRNAs, miR124a and miR9, will be inserted into the 3'UTR of the EGFP or LacZ reporter genes. A lentiviral delivery system will then deliver the synthetic gene into the genomes of murine embryonic stem cells. Transduced embryonic stem cells will then be induced to differentiate into a variety of cells types, including neural progenitors (using the stromal-cell derived induction method of Kawasaki et al. (2000) Neuron 28: 31-40). Reporter gene activity will be correlated with the expression of molecular markers to define which cell lineages allow expression of the introduced gene and which display miR9/124a-mediated silencing. Alternatively, the transduced murine ES cells will be transferred into blastocysts to generate chimeric mice, from which stable transgenic germ lines will be generated (Pfeifer et al. (2001) Proc. Natl. Acad. Sci. USA 99: 2140-2145). The spatial expression of reporter genes will be determined using direct detection (EGFP fluorescence or β-galactosidase staining) or immunohistochemistry.

Other experiments will study miRNA-mediated transgene regulation in cells that derive from transgenic adult stem cells. These will involve *in vitro* and *in vivo* studies of transduced bone marrow-derived stem cells, or haematopoietic progenitor cells, containing reporter genes (or therapeutic genes) with embedded miRNA target sequences. The target sequences will bind with haematopoietic lineage-specific miRNAs, such as miR142-3p. For example, the methodology may be accomplished as described in Brown et al. (2006) Nature Medicine 12: 585-591.

### Example 13

### The effect of increasing miRNA target sequences in the 3'UTR of a transgene

Cells of the stable pMM110 transgenic HeLa Tet On cell line, HTO110e, were grown in the presence, or absence, of 2µg doxycycline/mL medium and FuGene6-transfected, one day after plating, with 80 ng plasmid. The plasmids used for transfection were all derived from pMM043, with varying numbers of miR145 target sequences inserted in the EGFP 3'UTR NotI site. Plasmids were: pMM043 (no targets), pMM095 (1 target), pMM117 (2 targets), pMM119 (8 targets). In this cell line, doxycycline induces the expression of mature miR145 above the low background level present in HeLa cells. Values displayed are the mean fluorescence (n=3) at 46 hours after transfection.

The data is shown in Figure 12. The data demonstrates the Dox-inducible miR145 silencing of EGFP fluorescence in the transiently transfected cell line HTO110e, with the extent of silencing correlating with the number of miR145 target sequences present in the 3'UTR of EGFP.

Finally, it will be appreciated that various modifications and variations of the methods and compositions of the invention described herein will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are apparent to those skilled in the art are intended to be within the scope of the present invention.

## Claims

1. A method of promoting development of a cell *in vitro*, the method including introducing into the cell a nucleic acid with the capacity to promote development of the cell, the nucleic acid including a target site for binding of a microRNA, wherein the activity and/or concentration of the microRNA in the cell results in a level of activity and/or concentration of the nucleic acid in the cell sufficient to promote development of the cell, and wherein the nucleic acid encodes a cytokine or a therapeutic protein, or a fragment or variant thereof.

2. A method according to claim 1, wherein the nucleic acid encodes a gene selected from the group consisting of granulocyte macrophage colony stimulating factor, granulocyte colony stimulating factor, Interleukin 11 and tumour necrosis factor alpha.

3. A method according to claim 1 or claim 2, wherein the cell is selected from the group consisting of a cancerous cell, a pre-cancerous cell, a non-human embryonic stem cell, an adult stem cell, a haemopoietic cell, a haemopoietic precursor cell, an adipocyte, a neuronal cell, a non-human sperm cell, a non-human sperm producing cell, a pancreatic islet cell, and a virally infected cell.

4. A method according to any one of claims 1 to 3, wherein the microRNA or the binding site is a binding site for a microRNA selected from the group consisting of hsa-let-7a-1, hsa-let-7a-2, hsa-let-7a-3, hsa-let-7b, hsa-let-7c, hsa-let-7f, hsa-miR-10b, hsa-miR-15a, hsa-miR-15b, hsa-miR-16, hsa-miR-19b mature miRNA, hsa-miR-20, hsa-miR-21, hsa-miR-22, hsa-miR-23a, hsa-miR-24, has-miR-189, hsa-miR-26, hsa-miR-26b, hsa-miR-26a, hsa-miR-27b, hsa-miR-29a, hsa-miR30a-3p, hsa-miR-141, hsa-miR-142-5p, hsa-miR-142-3p, hsa-miR-143, hsa-miR-145, hsa-miR-192, hsa-miR-194, hsa-miR-199b, hsa-miR-200b, hsa-miR-200c, hsa-miR320, hsa-miR-321, hsa-miR-30a-5p, hsa-miR-29b, hsa-miR- 125b, hsa-miR-125a, hsa-miR-126*, hsa-miR-126, hsa-miR-188, hsa-miR-331, hsa-miR-181b-1, hsa-miR-155, hsa-miR-124a, hsa-miR-9 and the corresponding orthologues of the aforementioned microRNAs.

5. A nucleic acid with the capacity to promote development of a cell for use in promoting development of the cell to treat and/or prevent a disease, condition or state in an animal or human subject, wherein the nucleic acid includes a target site for binding of a microRNA, wherein the activity and/or concentration of the microRNA in the cell results in a level of activity and/or concentration of the nucleic acid in the cell sufficient to promote development of the cell, and wherein the nucleic acid encodes a cytokine or a therapeutic protein, or a fragment or variant thereof.

6. The nucleic acid according to claim 5, wherein the nucleic acid encodes a gene selected from the group consisting of granulocyte macrophage colony stimulating factor, granulocyte colony stimulating factor, Interleukin 11 and tumour necrosis factor alpha.

7. The nucleic acid according to claim 5 or claim 6, wherein the cell is selected from the group consisting of a cancerous cell, a pre-cancerous cell, a non-human embryonic stem cell, an adult stem cell, a haemopoietic cell, a haemopoietic precursor cell, an adipocyte, a neuronal cell, a non-human sperm cell, a non-human sperm producing cell, a pancreatic islet cell, and a virally infected cell.

8. The nucleic acid according to any one of claims 5 to 7, wherein the microRNA or the binding site is a binding site for a microRNA selected from the group consisting of hsa-let-7a-1, hsa-let-7a-2, hsa-let-7a-3, hsa-let-7b, hsa-let-7c, hsa-let-7f, hsa-miR-10b, hsa-miR-15a, hsa-miR-15b, hsa-miR-16, hsa-miR-19b mature miRNA, hsa-miR-20, hsa-miR-21, hsa-miR-22, hsa-miR-23a, hsa-miR-24, has-miR-189, hsa-miR-26, hsa-miR-26b, hsa-miR-26a, hsa-miR-27b, hsa-miR-29a, hsa-miR30a-3p, hsa-miR-141, hsa-miR-142-5p, hsa-miR-142-3p, hsa-miR-143, hsa-miR-145, hsa-miR-192, hsa-miR-194, hsa-miR-199b, hsa-miR-200b, hsa-miR-200c, hsa-miR320, hsa-miR-321, hsa-miR-30a-5p, hsa-miR-29b, hsa-miR- 125b, hsa-miR-125a, hsa-miR-126*, hsa-miR-126, hsa-miR-188, hsa-miR-331, hsa-miR-181b-1, hsa-miR-155, hsa-miR-124a, hsa-miR-9 and the corresponding orthologues of the aforementioned microRNAs.

9. The nucleic acid according to any one of claims 5 to 8, wherein said disease, condition or state is selected from the group consisting of a cancer, including colorectal cancer, lung cancer, thymus cancer, bladder cancer, breast cancer and prostate cancer; human B cell chronic lymphocytic leukemia; B cell (Burkitt) Lymphoma; a disease or disorder of pancreatic endocrine cells including diabetes; a disease or condition associated with viral infection of cells including EBV, HIV, Hepatitis and Herpes infection of cells; 5q-myelodysplastic syndrome (macrocytic anaemia); a disease or conditions associated with haemopoietic dysfunction, an autoimmune and inflammatory diseases including Crohn's disease; fragile X mental retardation; Di George syndrome; Wilms' tumour; a disease or condition associated with neuron dysfunction; a disease or condition associated with adipocyte dysfunction; a disease that can be treated with embryonic or adult stem cells; and a disease or condition associated with sperm producing cells.

10. Use of a nucleic acid with the capacity to promote development of a cell, in the manufacture of a medicament for promoting development of the cell to treat and/or prevent a disease, condition or state in an animal or human subj ect, wherein the nucleic acid includes a target site for binding of a microRNA, wherein the activity and/or concentration of the microRNA in the cell results in a level of activity and/or concentration of the nucleic acid in the cell sufficient to promote development of the cell, and wherein the nucleic acid encodes a cytokine or a therapeutic protein, or a fragment or variant thereof.

11. The use according to claim 10, wherein the nucleic acid encodes a gene selected from the group consisting of granulocyte macrophage colony stimulating factor, granulocyte colony stimulating factor, Interleukin 11 and tumour necrosis factor alpha.

12. The use according to claim 10 or claim 11, wherein the cell is selected from the group consisting of a cancerous cell, a pre-cancerous cell, a non-human embryonic stem cell, an adult stem cell, a haemopoietic cell, a haemopoietic precursor cell, an adipocyte, a neuronal cell, a non-human sperm cell, a non-human sperm producing cell, a pancreatic islet cell, and a virally infected cell.

13. The use according to any one of claims 10 to 12, wherein the microRNA or the binding site is a binding site for a microRNA selected from the group consisting of hsa-let-7a-1, hsa-let-7a-2, hsa-let-7a-3, hsa-let-7b, hsa-let-7c, hsa-let-7f, hsa-miR-10b, hsa-miR-15a, hsa-miR-15b, hsa-miR-16, hsa-miR-19b mature miRNA, hsa-miR-20, hsa-miR-21, hsa-miR-22, hsa-miR-23a, hsa-miR-24, has-miR-189, hsa-miR-26, hsa-miR-26b, hsa-miR-26a, hsa-miR-27b, hsa-miR-29a, hsa-miR30a-3p, hsa-miR-141, hsa-miR-142-5p, hsa-miR-142-3p, hsa-miR-143, hsa-miR-145, hsa-miR-192, hsa-miR-194, hsa-miR-199b, hsa-miR-200b, hsa-miR-200c, hsa-miR320, hsa-miR-321, hsa-miR-30a-5p, hsa-miR-29b, hsa-miR- 125b, hsa-miR-125a, hsa-miR-126*, hsa-miR-126, hsa-miR-188, hsa-miR-331, hsa-miR-181b-1, hsa-miR-155, hsa-miR-124a, hsa-miR-9 and the corresponding orthologues of the aforementioned microRNAs.

14. The use according to any one of claims 10 to 13, wherein said disease, condition or state is selected from the group consisting of a cancer, including colorectal cancer, lung cancer, thymus cancer, bladder cancer, breast cancer and prostate cancer; human B cell chronic lymphocytic leukemia; B cell (Burkitt) Lymphoma; a disease or disorder of pancreatic endocrine cells including diabetes; a disease or condition associated with viral infection of cells including EBV, HIV, Hepatitis and Herpes infection of cells; 5q-myelodysplastic syndrome (macrocytic anaemia); a disease or conditions associated with haemopoietic dysfunction, an autoimmune and inflammatory diseases including Crohn's disease; fragile X mental retardation; Di George syndrome; Wilms' tumour; a disease or condition associated with neuron dysfunction; a disease or condition associated with adipocyte dysfunction; a disease that can be treated with embryonic or adult stem cells; and a disease or condition associated with sperm producing cells.


SEQUENCE LISTING

<110>  Southern Adelaide Health Service - Flinders Medical Centre

<120>  Targeting Cells With Altered microRNA Expression

<130>  27.20.97428/01

<140>  PCT/AU2006/000750
<141>  2006-06-02

<160>  171

<170>  PatentIn version 3.3

<210>  1
<211>  80
<212>  RNA
<213>  Homo sapiens

<400>  1
ugggaugagg uaguagguug uauaguuuua gggucacacc caccacuggg agauaacuau      60

acaaucuacu gucuuuccua                                                  80


<210>  2
<211>  22
<212>  RNA
<213>  Homo sapiens

<400>  2
ugagguagua gguuguauag uu                                               22


<210>  3
<211>  72
<212>  RNA
<213>  Homo sapiens

<400>  3
agguugaggu aguagguugu auaguuuaga auuacaucaa gggagauaac uguacagccu      60

ccuagcuuuc cu                                                          72


<210>  4
<211>  22
<212>  RNA
<213>  Homo sapiens

<400>  4
ugagguagua gguuguauag uu                                               22


<210>  5
<211>  74
<212>  RNA
<213>  Homo sapiens

<400>  5
gggugaggua guagguugua uaguuugggg cucugcccug cuaugggaua acuauacaau      60

cuacugucuu uccu                                                        74


<210>  6
<211>  22

<212>  RNA
<213>  Homo sapiens

<400>  6
ugagguagua gguuguauag uu                                              22


<210>  7
<211>  83
<212>  RNA
<213>  Homo sapiens

<400>  7
cggggugagg uaguagguug ugugguuuca gggcagugau guugccccuc ggaagauaac      60

uauacaaccu acugccuucc cug                                             83


<210>  8
<211>  22
<212>  RNA
<213>  Homo sapiens

<400>  8
ugagguagua gguugugugg uu                                              22


<210>  9
<211>  84
<212>  RNA
<213>  Homo sapiens

<400>  9
gcauccgggu ugagguagua gguuguaugg uuuagaguua cacccuggga guuaacugua      60

caaccuucua gcuuuccuug gagc                                            84


<210>  10
<211>  22
<212>  RNA
<213>  Homo sapiens

<400>  10
ugagguagua gguuguaugg uu                                              22


<210>  11
<211>  87
<212>  RNA
<213>  Homo sapiens

<400>  11
ucagagugag guaguagauu guauaguugu gggguaguga uuuuacccug uucaggagau      60

aacuauacaa ucuauugccu ucccuga                                         87


<210>  12
<211>  22
<212>  RNA
<213>  Homo sapiens

<400>  12
ugagguagua gauuguauag uu                                              22


<210>  13

<211>  83
<212>  RNA
<213>  Homo sapiens

<400>  13
ugugggauga gguaguagau uguauaguuu uagggucaua ccccaucuug gagauaacua      60

uacagucuac ugucuuuccc acg                                              83


<210>  14
<211>  22
<212>  RNA
<213>  Homo sapiens

<400>  14
ugagguagua gauuguauag uu                                               22


<210>  15
<211>  110
<212>  RNA
<213>  Homo sapiens

<400>  15
ccagagguug uaacguuguc uauauauacc cuguagaacc gaauuugugu gguauccgua      60

uagucacaga uucgauucua ggggaauaua uggucgaugc aaaaacuuca                110


<210>  16
<211>  22
<212>  RNA
<213>  Homo sapiens

<400>  16
uacccuguag aaccgaauuu gu                                               22


<210>  17
<211>  98
<212>  RNA
<213>  Homo sapiens

<400>  17
uugaggccuu aaaguacugu agcagcacau caugguuuac augcuacagu caagaugcga      60

aucauuauuu gcugcucuag aaauuuaagg aaauucau                              98


<210>  18
<211>  22
<212>  RNA
<213>  Homo sapiens

<400>  18
uagcagcaca ucaugguuua ca                                               22


<210>  19
<211>  89
<212>  RNA
<213>  Homo sapiens

<400>  19
gucagcagug ccuuagcagc acguaaauau uggcguuaag auucuaaaau uaucuccagu      60

auuaacugug cugcugaagu aagguugac                                        89

<210>  20
<211>  22
<212>  RNA
<213>  Homo sapiens

<400>  20
uagcagcacg uaaauauugg cg                                                22


<210>  21
<211>  81
<212>  RNA
<213>  Homo sapiens

<400>  21
guuccacucu agcagcacgu aaauauuggc guagugaaau auauauuaaa caccaauauu      60

acugugcugc uuuaguguga c                                                 81


<210>  22
<211>  22
<212>  RNA
<213>  Homo sapiens

<400>  22
uagcagcacg uaaauauugg cg                                                22


<210>  23
<211>  87
<212>  RNA
<213>  Homo sapiens

<400>  23
cacuguucua ugguuaguuu ugcagguuug cauccagcug ugugauauuc ugcugugcaa      60

auccaugcaa aacugacugu gguagug                                           87


<210>  24
<211>  23
<212>  RNA
<213>  Homo sapiens

<400>  24
ugugcaaauc caugcaaaac uga                                               23


<210>  25
<211>  96
<212>  RNA
<213>  Homo sapiens

<400>  25
acauugcuac uuacaauuag uuuugcaggu uugcauuuca gcguauauau guauaugugg      60

cugugcaaau ccaugcaaaa cugauuguga uaaugu                                 96


<210>  26
<211>  23
<212>  RNA
<213>  Homo sapiens

<400>  26

ugugcaaauc caugcaaaac uga                                              23

<210>  27
<211>  71
<212>  RNA
<213>  Homo sapiens

<400>  27
guagcacuaa agugcuuaua gugcagguag uguuuaguua ucuacugcau uaugagcacu      60

uaaaguacug c                                                           71

<210>  28
<211>  23
<212>  RNA
<213>  Homo sapiens

<400>  28
uaaagugcuu auagugcagg uag                                              23

<210>  29
<211>  72
<212>  RNA
<213>  Homo sapiens

<400>  29
ugucggguag cuuaucagac ugauguugac uguugaaucu cauggcaaca ccagucgaug      60

ggcugucuga ca                                                          72

<210>  30
<211>  22
<212>  RNA
<213>  Homo sapiens

<400>  30
uagcuuauca gacugauguu ga                                               22

<210>  31
<211>  85
<212>  RNA
<213>  Homo sapiens

<400>  31
ggcugagccg caguaguucu ucaguggcaa gcuuuauguc cugacccagc uaaagcugcc      60

aguugaagaa cuguugcccu cugcc                                            85

<210>  32
<211>  22
<212>  RNA
<213>  Homo sapiens

<400>  32
aagcugccag uugaagaacu gu                                               22

<210>  33
<211>  73
<212>  RNA
<213>  Homo sapiens

<400>  33
ggccggcugg gguuccuggg gaugggauuu gcuuccuguc acaaaucaca uugccaggga      60

uuuccaaccg acc                                                          73


<210>  34
<211>  21
<212>  RNA
<213>  Homo sapiens

<400>  34
aucacauugc cagggauuuc c                                                 21


<210>  35
<211>  68
<212>  RNA
<213>  Homo sapiens

<400>  35
cuccggugcc uacugagcug auaucaguuc ucauuuuaca cacuggcuca guucagcagg      60

aacaggag                                                                68


<210>  36
<211>  22
<212>  RNA
<213>  Homo sapiens

<400>  36
uggcucaguu cagcaggaac ag                                                22


<210>  37
<211>  23
<212>  RNA
<213>  Homo sapiens

<400>  37
gugccuacug agcugauauc agu                                               23


<210>  38
<211>  73
<212>  RNA
<213>  Homo sapiens

<400>  38
cucugccucc cgugccuacu gagcugaaac acaguugguu uguguacacu ggcucaguuc      60

agcaggaaca ggg                                                          73


<210>  39
<211>  22
<212>  RNA
<213>  Homo sapiens

<400>  39
uggcucaguu cagcaggaac ag                                                22


<210>  40
<211>  77
<212>  RNA
<213>  Homo sapiens

<400>  40
guggccucgu ucaaguaauc caggauaggc ugugcagguc ccaaugggcc uauucuuggu      60

uacuugcacg gggacgc                                                      77


<210>  41
<211>  22
<212>  RNA
<213>  Homo sapiens

<400>  41
auucaaguaa uccaggauag gc                                                22


<210>  42
<211>  77
<212>  RNA
<213>  Homo sapiens

<400>  42
ccgggaccca guucaaguaa uucaggauag guugugugcu guccagccug uucuccauua      60

cuuggcucgg ggaccgg                                                      77


<210>  43
<211>  22
<212>  RNA
<213>  Homo sapiens

<400>  43
uucaaguaau ucaggauagg uu                                                22


<210>  44
<211>  84
<212>  RNA
<213>  Homo sapiens

<400>  44
ggcuguggcu ggauucaagu aauccaggau aggcuguuuc caucugugag gccuauucuu      60

gauuacuugu uucuggaggc agcu                                              84


<210>  45
<211>  21
<212>  RNA
<213>  Homo sapiens

<400>  45
uucaaguaau ccaggauagg c                                                 21


<210>  46
<211>  97
<212>  RNA
<213>  Homo sapiens

<400>  46
accucucuaa caaggugcag agcuuagcug auuggugaac agugauuggu uuccgcuuug      60

uucacagugg cuaaguucug caccugaaga gaaggug                                97


<210>  47

<211>  21
<212>  RNA
<213>  Homo sapiens

<400>  47
uucacagugg cuaaguucug c                                                21


<210>  48
<211>  64
<212>  RNA
<213>  Homo sapiens

<400>  48
augacugauu ucuuuuggug uucagaguca auauaauuuu cuagcaccau cugaaaucgg      60

uuau                                                                   64


<210>  49
<211>  21
<212>  RNA
<213>  Homo sapiens

<400>  49
uagcaccauc ugaaaucggu u                                                21


<210>  50
<211>  71
<212>  RNA
<213>  Homo sapiens

<400>  50
gcgacuguaa acauccucga cuggaagcug ugaagccaca gaugggcuuu cagucggaug      60

uuugcagcug c                                                           71


<210>  51
<211>  22
<212>  RNA
<213>  Homo sapiens

<400>  51
cuuucagucg gauguuugca gc                                               22


<210>  52
<211>  22
<212>  RNA
<213>  Homo sapiens

<400>  52
uguaaacauc cucgacugga ag                                               22


<210>  53
<211>  95
<212>  RNA
<213>  Homo sapiens

<400>  53
cggccggccc uggguccauc uuccaguaca guguuggaug gucuaauugu gaagcuccua      60

acacugucug guaaagaugg cucccgggug gguuc                                 95

<210>  54
<211>  22
<212>  RNA
<213>  Homo sapiens

<400>  54
uaacacuguc ugguaaagau gg                                                22


<210>  55
<211>  87
<212>  RNA
<213>  Homo sapiens

<400>  55
gacagugcag ucacccauaa aguagaaagc acuacuaaca gcacuggagg guguaguguu      60

uccuacuuua uggaugagug uacugug                                           87


<210>  56
<211>  20
<212>  RNA
<213>  Homo sapiens

<400>  56
cauaaaguag aaagcacuac                                                   20


<210>  57
<211>  23
<212>  RNA
<213>  Homo sapiens

<400>  57
uguaguguuu ccuacuuuau gga                                               23


<210>  58
<211>  106
<212>  RNA
<213>  Homo sapiens

<400>  58
gcgcagcgcc cugucuccca gccugaggug cagugcugca ucucugguca guugggaguc      60

ugagaugaag cacuguagcu caggaagaga gaaguuguuc ugcagc                     106


<210>  59
<211>  22
<212>  RNA
<213>  Homo sapiens

<400>  59
ugagaugaag cacuguagcu ca                                                22


<210>  60
<211>  88
<212>  RNA
<213>  Homo sapiens

<400>  60
caccuugucc ucacggucca guuuucccag gaaucccuua gaugcuaaga uggggauucc      60

uggaaauacu guucuugagg ucaugguu                                          88

<210> 61
<211> 24
<212> RNA
<213> Homo sapiens

<400> 61
guccaguuuu cccaggaauc ccuu 24


<210> 62
<211> 110
<212> RNA
<213> Homo sapiens

<400> 62
gccgagaccg agugcacagg gcucugaccu augaauugac agccagugcu cucgucuccc 60

cucuggcugc caauuccaua ggucacaggu auguucgccu caaugccagc 110


<210> 63
<211> 21
<212> RNA
<213> Homo sapiens

<400> 63
cugaccuaug aauugacagc c 21


<210> 64
<211> 85
<212> RNA
<213> Homo sapiens

<400> 64
augguguuau caaguguaac agcaacucca uguggacugu guaccaauuu ccaguggaga 60

ugcuguuacu uuugaugguu accaa 85


<210> 65
<211> 22
<212> RNA
<213> Homo sapiens

<400> 65
uguaacagca acuccaugug ga 22


<210> 66
<211> 85
<212> RNA
<213> Homo sapiens

<400> 66
ugguucccgc ccccuguaac agcaacucca uguggaagug cccacugguu ccaguggggc 60

ugcuguuauc uggggcgagg gccag 85


<210> 67
<211> 22
<212> RNA
<213> Homo sapiens

<400> 67
uguaacagca acuccaugug ga 22

<210>  68
<211>  110
<212>  RNA
<213>  Homo sapiens

<400>  68
ccagaggaca ccuccacucc gucuacccag uguuuagacu aucuguucag gacucccaaa      60

uuguacagua gucugcacau ugguuaggcu gggcuggguu agacccucgg                110


<210>  69
<211>  23
<212>  RNA
<213>  Homo sapiens

<400>  69
cccaguguuu agacuaucug uuc                                              23


<210>  70
<211>  95
<212>  RNA
<213>  Homo sapiens

<400>  70
ccagcucggg cagccguggc caucuuacug ggcagcauug gauggaguca ggucucuaau      60

acugccuggu aaugaugacg gcggagcccu gcacg                                 95


<210>  71
<211>  23
<212>  RNA
<213>  Homo sapiens

<400>  71
uaauacugcc ugguaaugau gac                                              23


<210>  72
<211>  68
<212>  RNA
<213>  Homo sapiens

<400>  72
cccucgucuu acccagcagu guuugggugc gguugggagu cucuaauacu gccggguaau      60

gauggagg                                                               68


<210>  73
<211>  22
<212>  RNA
<213>  Homo sapiens

<400>  73
uaauacugcc ggguaaugau gg                                               22


<210>  74
<211>  82
<212>  RNA
<213>  Homo sapiens

<400>  74

gcuucgcucc ccuccgccuu cucuucccgg uucuucccgg agucgggaaa agcuggguug     60

agagggcgaa aaaggaugag gu                                              82


<210>  75
<211>  23
<212>  RNA
<213>  Homo sapiens

<400>  75
aaaagcuggg uugagagggc gaa                                             23


<210>  76
<211>  21
<212>  RNA
<213>  Homo sapiens

<400>  76
   uaagccaggg auuugggguu c                                                21


<210>  77
<211>  71
<212>  RNA
<213>  Homo sapiens

<400>  77
gcgacuguaa acauccucga cuggaagcug ugaagccaca gaugggcuuu cagucggaug     60

uuugcagcug c                                                          71


<210>  78
<211>  22
<212>  RNA
<213>  Homo sapiens

<400>  78
cuuucagucg gauguuugca gc                                              22


<210>  79
<211>  22
<212>  RNA
<213>  Homo sapiens

<400>  79
uguaaacauc cucgacugga ag                                              22


<210>  80
<211>  81
<212>  RNA
<213>  Homo sapiens

<400>  80
cuucaggaag cugguuucau auggugguuu agauuuaaau agugauuguc uagcaccauu     60

ugaaaucagu guucuugggg g                                               81


<210>  81
<211>  23
<212>  RNA
<213>  Homo sapiens

<400>  81
uagcaccauu ugaaaucagu guu                                              23


<210>  82
<211>  88
<212>  RNA
<213>  Homo sapiens

<400>  82
ugcgcuccuc ucagucccug agacccuaac uugugauguu uaccguuuaa auccacgggu      60

uaggcucuug ggagcugcga gucgugcu                                         88


<210>  83
<211>  22
<212>  RNA
<213>  Homo sapiens

<400>  83
ucccugagac ccuaacuugu ga                                               22


<210>  84
<211>  86
<212>  RNA
<213>  Homo sapiens

<400>  84
ugccagucuc uaggucccug agacccuuua accugugagg acauccaggg ucacagguga      60

gguucuuggg agccuggcgu cuggcc                                           86


<210>  85
<211>  23
<212>  RNA
<213>  Homo sapiens

<400>  85
ucccugagac ccuuuaaccu gug                                              23


<210>  86
<211>  89
<212>  RNA
<213>  Homo sapiens

<400>  86
accagacuuu uccuaguccc ugagacccua acuugugagg uauuuuagua acaucacaag      60

ucaggcucuu gggaccuagg cggagggga                                         89


<210>  87
<211>  22
<212>  RNA
<213>  Homo sapiens

<400>  87
ucccugagac ccuaacuugu ga                                               22


<210>  88
<211>  83
<212>  RNA
<213>  Homo sapiens

<400>  88
ccuuggagua aaguagcagc acauaauggu uuguggauuu ugaaaaggug caggccauau      60

ugugcugccu caaaaauaca agg                                              83


<210>  89
<211>  22
<212>  RNA
<213>  Homo sapiens

<400>  89
uagcagcaca uaaugguuug ug                                               22


<210>  90
<211>  85
<212>  RNA
<213>  Homo sapiens

<400>  90
cgcuggcgac gggacauuau uacuuuuggu acgcgcugug acacuucaaa cucguaccgu      60

gaguaauaau gcgccgucca cggca                                            85


<210>  91
<211>  21
<212>  RNA
<213>  Homo sapiens

<400>  91
cauuauuacu uuugguacgc g                                                21


<210>  92
<211>  21
<212>  RNA
<213>  Homo sapiens

<400>  92
ucguaccgug aguaauaaug c                                                21


<210>  93
<211>  86
<212>  RNA
<213>  Homo sapiens

<400>  93
ugcucccucu cucacauccc uugcauggug gagggugagc uuucugaaaa ccccucccac      60

augcagguuu ugcaggaugg cgagcc                                           86


<210>  94
<211>  22
<212>  RNA
<213>  Homo sapiens

<400>  94
caucccuugc augguggagg gu                                               22


<210>  95
<211>  94
<212>  RNA

<213>  Homo sapiens

<400>  95
gaguuugguu uuguuugggu uuguucuagg uaugguccca gggaucccag aucaaaccag      60

gccccugggc cuauccuaga accaaccuaa gcuc                                   94


<210>  96
<211>  21
<212>  RNA
<213>  Homo sapiens

<400>  96
gccccugggc cuauccuaga a                                                 21


<210>  97
<211>  65
<212>  RNA
<213>  Homo sapiens

<400>  97
cuguuaaugc uaaucgugau agggguuuuu gccuccaacu gacuccuaca uauuagcauu      60

aacag                                                                   65


<210>  98
<211>  22
<212>  RNA
<213>  Homo sapiens

<400>  98
uuaaugcuaa ucgugauagg gg                                                22


<210>  99
<211>  22
<212>  RNA
<213>  Homo sapiens

<400>  99
aacuauacaa ccuacuaccu ca                                                22


<210>  100
<211>  22
<212>  RNA
<213>  Homo sapiens

<400>  100
aacuauacaa ccuacuaccu ca                                                22


<210>  101
<211>  22
<212>  RNA
<213>  Homo sapiens

<400>  101
aacuauacaa ccuacuaccu ca                                                22


<210>  102
<211>  22
<212>  RNA
<213>  Homo sapiens

<400>  102
aaccacacaa ccuacuaccu ca                                                22

<210>  103
<211>  22
<212>  RNA
<213>  Homo sapiens

<400>  103
aaccauacaa ccuacuaccu ca                                                22

<210>  104
<211>  22
<212>  RNA
<213>  Homo sapiens

<400>  104
aacuauacaa ucuacuaccu ca                                                22

<210>  105
<211>  22
<212>  RNA
<213>  Homo sapiens

<400>  105
aacuauacaa ucuacuaccu ca                                                22

<210>  106
<211>  22
<212>  RNA
<213>  Homo sapiens

<400>  106
acaaauucgg uucuacaggg ua                                                22

<210>  107
<211>  22
<212>  RNA
<213>  Homo sapiens

<400>  107
uguaaaccau gaugugcugc ua                                                22

<210>  108
<211>  22
<212>  RNA
<213>  Homo sapiens

<400>  108
cgccaauauu uacgugcugc ua                                                22

<210>  109
<211>  22
<212>  RNA
<213>  Homo sapiens

<400>  109
cgccaauauu uacgugcugc ua                                                22

<210>  110
<211>  23
<212>  RNA
<213>  Homo sapiens

<400>  110
ucaguuuugc auggauuugc aca                                              23


<210>  111
<211>  23
<212>  RNA
<213>  Homo sapiens

<400>  111
ucaguuuugc auggauuugc aca                                              23


<210>  112
<211>  23
<212>  RNA
<213>  Homo sapiens

<400>  112
cuaccugcac uauaagcacu uua                                              23


<210>  113
<211>  22
<212>  RNA
<213>  Homo sapiens

<400>  113
ucaacaucag ucugauaagc ua                                               22


<210>  114
<211>  22
<212>  RNA
<213>  Homo sapiens

<400>  114
acaguucuuc aacuggcagc uu                                               22


<210>  115
<211>  21
<212>  RNA
<213>  Homo sapiens

<400>  115
ggaaaucccu ggcaauguga u                                                21


<210>  116
<211>  22
<212>  RNA
<213>  Homo sapiens

<400>  116
cuguuccugc ugaacugagc ca                                               22


<210>  117
<211>  23
<212>  RNA
<213>  Homo sapiens

<400>  117
acugauauca gcucaguagg cac                                              23


<210>  118
<211>  22
<212>  RNA
<213>  Homo sapiens

<400>  118
cuguuccugc ugaacugagc ca                                               22


<210>  119
<211>  22
<212>  RNA
<213>  Homo sapiens

<400>  119
gccuauccug gauuacuuga au                                               22


<210>  120
<211>  22
<212>  RNA
<213>  Homo sapiens

<400>  120
aaccuauccu gaauuacuug aa                                               22


<210>  121
<211>  21
<212>  RNA
<213>  Homo sapiens

<400>  121
gccuauccug gauuacuuga a                                                21


<210>  122
<211>  21
<212>  RNA
<213>  Homo sapiens

<400>  122
gcagaacuua gccacuguga a                                                21


<210>  123
<211>  21
<212>  RNA
<213>  Homo sapiens

<400>  123
aaccgauuuc agauggugcu a                                                21


<210>  124
<211>  22
<212>  RNA
<213>  Homo sapiens

<400>  124
gcugcaaaca uccgacugaa ag                                               22


<210>  125

<211>  22
<212>  RNA
<213>  Homo sapiens

<400>  125
cuuccagucg aggauguuua ca                                                22


<210>  126
<211>  22
<212>  RNA
<213>  Homo sapiens

<400>  126
ccaucuuuac cagacagugu ua                                                22


<210>  127
<211>  20
<212>  RNA
<213>  Homo sapiens

<400>  127
guagugcuuu cuacuuuaug                                                   20


<210>  128
<211>  23
<212>  RNA
<213>  Homo sapiens

<400>  128
uccauaaagu aggaaacacu aca                                               23


<210>  129
<211>  22
<212>  RNA
<213>  Homo sapiens

<400>  129
ugagcuacag ugcuucaucu ca                                                22


<210>  130
<211>  24
<212>  RNA
<213>  Homo sapiens

<400>  130
aagggauucc ugggaaaacu ggac                                              24


<210>  131
<211>  21
<212>  RNA
<213>  Homo sapiens

<400>  131
ggcugucaau ucauagguca g                                                 21


<210>  132
<211>  22
<212>  RNA
<213>  Homo sapiens

<400>  132

uccacaugga guugcuguua ca                                              22

<210>  133
<211>  22
<212>  RNA
<213>  Homo sapiens

<400>  133
uccacaugga guugcuguua ca                                              22

<210>  134
<211>  23
<212>  RNA
<213>  Homo sapiens

<400>  134
gaacagauag ucuaaacacu ggg                                             23

<210>  135
<211>  23
<212>  RNA
<213>  Homo sapiens

<400>  135
gucaucauua ccaggcagua uua                                             23

<210>  136
<211>  22
<212>  RNA
<213>  Homo sapiens

<400>  136
ccaucauuac ccggcaguau ua                                              22

<210>  137
<211>  23
<212>  RNA
<213>  Homo sapiens

<400>  137
uucgcccucu caacccagcu uuu                                             23

<210>  138
<211>  21
<212>  RNA
<213>  Homo sapiens

<400>  138
gaacccacaa ucccuggcuu a                                               21

<210>  139
<211>  22
<212>  RNA
<213>  Homo sapiens

<400>  139
gcugcaaaca uccgacugaa ag                                              22

<210>  140
<211>  22

<212>  RNA
<213>  Homo sapiens

<400>  140
cuuccagucg aggauguuua ca                                                22


<210>  141
<211>  23
<212>  RNA
<213>  Homo sapiens

<400>  141
aacacugauu ucaaauggug cua                                               23


<210>  142
<211>  22
<212>  RNA
<213>  Homo sapiens

<400>  142
ucacaaguua gggucucagg ga                                                22


<210>  143
<211>  23
<212>  RNA
<213>  Homo sapiens

<400>  143
cacagguuaa agggucucag gga                                               23


<210>  144
<211>  22
<212>  RNA
<213>  Homo sapiens

<400>  144
ucacaaguua gggucucagg ga                                                22


<210>  145
<211>  22
<212>  RNA
<213>  Homo sapiens

<400>  145
cacaaaccau uaugugcugc ua                                                22


<210>  146
<211>  21
<212>  RNA
<213>  Homo sapiens

<400>  146
cgcguaccaa aaguaauaau g                                                 21


<210>  147
<211>  21
<212>  RNA
<213>  Homo sapiens

<400>  147
gcauuauuac ucacgguacg a                                                 21

<210>  148
<211>  22
<212>  RNA
<213>  Homo sapiens

<400>  148
acccuccacc augcaaggga ug                                                22


<210>  149
<211>  21
<212>  RNA
<213>  Homo sapiens

<400>  149
uucuaggaua ggcccagggg c                                                 21


<210>  150
<211>  22
<212>  RNA
<213>  Homo sapiens

<400>  150
ccccuaucac gauuagcauu aa                                                22


<210>  151
<211>  1131
<212>  DNA
<213>  herpes simplex virus 7

<400>  151
atggcttctc acgccggcca acagcacgcg cctgcgttcg gtcaggctgc tcgtgcgagc      60

gggcctaccg acggccgcgc ggcgtcccgt cctagccatc gccagggggc ctccggagcc     120

cgcggggatc cggagctgcc cacgctgctg cgggtttata tagacggacc ccacggggtg     180

gggaagacca ccacctccgc gcagctgatg gaggccctgg ggccgcgcga caatatcgtc     240

tacgtccccg agccgatgac ttactggcag gtgctggggg cctccgagac cctgacgaac     300

atctacaaca cgcagcaccg tctggaccgc ggcgagatat cggccgggga ggcggcggtg     360

gtaatgacca gcgcccagat aacaatgagc acgccttatg cggcgacgga cgccgttttg     420

gctcctcata tcggggggga ggctgtgggc ccgcaagccc cgcccccggc cctcaccctt     480

gttttcgacc ggcaccctat cgcctccctg ctgtgctacc cggccgcgcg gtacctcatg     540

ggaagcatga ccccccaggc cgtgttggcg ttcgtggccc tcatgccccc gaccgcgccc     600

ggcacgaacc tggtcctggg tgtccttccg gaggccgaac acgccgaccg cctggccaga     660

cgccaacgcc cgggcgagcg gcttgacctg gccatgctgt ccgccattcg ccgtgtctac     720

gatctactcg ccaacacggt gcggtacctg cagcgcggcg ggaggtggcg ggaggactgg     780

ggccggctga cgggggtcgc cgcggcgacc ccgcgccccg accccgagga cggcgcgggg     840

tctctgcccc gcatcgagga cacgctgttt gccctgttcc gcgttcccga gctgctggcc     900

cccaacgggg acttgtacca catttttgcc tgggtcttgg acgtcttggc cgaccgcctc     960

cttccgatgc atctatttgt cctggattac gatcagtcgc ccgtcgggtg tcgagacgcc    1020

ctgttgcgcc tcaccgccgg gatgatccca acccgcgtca caaccgccgg gtccatcgcc 1080

gagatacgcg acctggcgcg cacgtttgcc cgcgaggtgg ggggagttta g 1131

<210> 152
<211> 1284
<212> DNA
<213> Escherichia coli

<400> 152
gtgtcgaata acgctttaca aacaattatt aacgcccggt taccaggcga agaggggctg 60

tggcagattc atctgcagga cggaaaaatc agcgccattg atgcgcaatc cggcgtgatg 120

cccataactg aaaacagcct ggatgccgaa caaggtttag ttataccgcc gtttgtggag 180

ccacatattc acctggacac cacgcaaacc gccggacaac cgaactggaa tcagtccggc 240

acgctgtttg aaggcattga acgctgggcc gagcgcaaag cgttattaac ccatgacgat 300

gtgaaacaac gcgcatggca aacgctgaaa tggcagattg ccaacggcat tcagcatgtg 360

cgtacccatg tcgatgtttc ggatgcaacg ctaactgcgc tgaaagcaat gctggaagtg 420

aagcaggaag tcgcgccgtg gattgatctg caaatcgtcg ccttccctca ggaagggatt 480

ttgtcgtatc ccaacggtga agcgttgctg gaagaggcgt tacgcttagg ggcagatgta 540

gtgggggcga ttccgcattt tgaatttacc cgtgaatacg gcgtggagtc gctgcataaa 600

accttcgccc tggcgcaaaa atacgaccgt ctcatcgacg ttcactgtga tgagatcgat 660

gacgagcagt cgcgctttgt cgaaaccgtt gctgccctgg cgcaccatga aggcatgggc 720

gcgcgagtca ccgccagcca caccacggca atgcactcct ataacggggc gtatacctca 780

cgcctgttcc gcttgctgaa aatgtccggt attaactttg tcgccaaccc gctggtcaat 840

attcatctgc aaggacgttt cgatacgtat ccaaaacgtc gcggcatcac gcgcgttaaa 900

gagatgctgg agtccggcat taacgtctgc tttggtcacg atgatgtctt cgatccgtgg 960

tatccgctgg gaacggcgaa tatgctgcaa gtgctgcata tggggctgca tgtttgccag 1020

ttgatgggct acgggcagat taacgatggc ctgaatttaa tcacccacca cagcgcaagg 1080

acgttgaatt tgcaggatta cggcattgcc gccggaaaca gcgccaacct gattatcctg 1140

ccggctgaaa atgggtttga tgcgctgcgc cgtcaggttc cggtacgtta ttcggtacgt 1200

ggcggcaagg tgattgccag cacacaaccg gcacaaacca ccgtatatct ggagcagcca 1260

gaagccatcg attacaaacg ttga 1284

<210> 153
<211> 6195
<212> DNA
<213> plasmid pMM095

<400> 153
gacggatcgg gagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240
gattattgac tagttattaa tagtaatcaa ttacggggtc attagttcat agcccatata 300
tggagttccg cgttacataa cttacggtaa atggcccgcc tggctgaccg cccaacgacc 360
cccgcccatt gacgtcaata atgacgtatg ttcccatagt aacgccaata gggactttcc 420
attgacgtca atgggtggag tatttacggt aaactgccca cttggcagta catcaagtgt 480
atcatatgcc aagtacgccc cctattgacg tcaatgacgg taaatggccc gcctggcatt 540
atgcccagta catgacctta tgggactttc ctacttggca gtacatctac gtattagtca 600
tcgctattac catggtgatg cggttttggc agtacatcaa tgggcgtgga tagcggtttg 660
actcacgggg atttccaagt ctccacccca ttgacgtcaa tgggagtttg ttttggcacc 720
aaaatcaacg ggactttcca aaatgtcgta acaactccgc cccattgacg caaatgggcg 780
gtaggcgtgt acggtgggag gtctatataa gcagagctct ctggctaact agagaaccca 840
ctgcttactg gcttatcgaa attaatacga ctcactatag ggagacccaa gctggctagc 900
gtttaaactt aagcttggta ccgagctcgg atctcgagct caagcttcga attctgcagt 960
cgacggtacc gcgggcccgg gatccaccgg tcgccaccat ggtgagcaag ggcgaggagc 1020
tgttcaccgg ggtggtgccc atcctggtcg agctggacgg cgacgtaaac ggccacaagt 1080
tcagcgtgtc cggcgagggc gagggcgatg ccacctacgg caagctgacc ctgaagttca 1140
tctgcaccac cggcaagctg cccgtgccct ggcccaccct cgtgaccacc ctgacctacg 1200
gcgtgcagtg cttcagccgc taccccgacc acatgaagca gcacgacttc ttcaagtccg 1260
ccatgcccga aggctacgtc caggagcgca ccatcttctt caaggacgac ggcaactaca 1320
agacccgcgc cgaggtgaag ttcgagggcg acaccctggt gaaccgcatc gagctgaagg 1380
gcatcgactt caaggaggac ggcaacatcc tggggcacaa gctggagtac aactacaaca 1440
gccacaacgt ctatatcatg gccgacaagc agaagaacgg catcaaggtg aacttcaaga 1500
tccgccacaa catcgaggac ggcagcgtgc agctcgccga ccactaccag cagaacaccc 1560
ccatcggcga cggccccgtg ctgctgcccg acaaccacta cctgagcacc cagtccgccc 1620
tgagcaaaga ccccaacgag aagcgcgatc acatggtcct gctggagttc gtgaccgccg 1680
ccgggatcac tctcggcatg gacgagctgt acaagtaaag cggccgctcg agtctagcag 1740
atcctgggaa aactggacct agagggcccg tttaaacccg ctgatcagcc tcgactgtgc 1800
cttctagttg ccagccatct gttgtttgcc cctcccccgt gccttccttg accctggaag 1860
gtgccactcc cactgtcctt tcctaataaa atgaggaaat tgcatcgcat tgtctgagta 1920
ggtgtcattc tattctgggg ggtggggtgg ggcaggacag caagggggag gattgggaag 1980
acaatagcag gcatgctggg gatgcggtgg gctctatggc ttctgaggcg gaaagaacca 2040
gctggggctc tagggggtat ccccacgcgc cctgtagcgg cgcattaagc gcggcgggtg 2100
tggtggttac gcgcagcgtg accgctacac ttgccagcgc cctagcgccc gctcctttcg 2160
ctttcttccc ttcctttctc gccacgttcg ccggctttcc ccgtcaagct ctaaatcggg 2220

ggctcccttt agggttccga tttagtgctt tacggcacct cgaccccaaa aaacttgatt 2280
agggtgatgg ttcacgtagt gggccatcgc cctgatagac ggtttttcgc cctttgacgt 2340
tggagtccac gttctttaat agtggactct tgttccaaac tggaacaaca ctcaacccta 2400
tctcggtcta ttcttttgat ttataaggga ttttgccgat ttcggcctat tggttaaaaa 2460
atgagctgat ttaacaaaaa tttaacgcga attaattctg tggaatgtgt gtcagttagg 2520
gtgtggaaag tccccaggct ccccagcagg cagaagtatg caaagcatgc atctcaatta 2580
gtcagcaacc aggtgtggaa agtccccagg ctccccagca ggcagaagta tgcaaagcat 2640
gcatctcaat tagtcagcaa ccatagtccc gcccctaact ccgcccatcc cgcccctaac 2700
tccgcccagt tccgcccatt ctccgcccca tggctgacta atttttttta tttatgcaga 2760
ggccgaggcc gcctctgcct ctgagctatt ccagaagtag tgaggaggct tttttggagg 2820
cctaggcttt tgcaaaaagc tcccgggagc ttgtatatcc attttcggat ctgatcaaga 2880
gacaggatga ggatcgtttc gcatgattga acaagatgga ttgcacgcag gttctccggc 2940
cgcttgggtg gagaggctat tcggctatga ctgggcacaa cagacaatcg gctgctctga 3000
tgccgccgtg ttccggctgt cagcgcaggg gcgcccggtt ctttttgtca agaccgacct 3060
gtccggtgcc ctgaatgaac tgcaggacga ggcagcgcgg ctatcgtggc tggccacgac 3120
gggcgttcct tgcgcagctg tgctcgacgt tgtcactgaa gcgggaaggg actggctgct 3180
attgggcgaa gtgccggggc aggatctcct gtcatctcac cttgctcctg ccgagaaagt 3240
atccatcatg gctgatgcaa tgcggcggct gcatacgctt gatccggcta cctgcccatt 3300
cgaccaccaa gcgaaacatc gcatcgagcg agcacgtact cggatggaag ccggtcttgt 3360
cgatcaggat gatctggacg aagagcatca ggggctcgcg ccagccgaac tgttcgccag 3420
gctcaaggcg cgcatgcccg acggcgagga tctcgtcgtg acccatggcg atgcctgctt 3480
gccgaatatc atggtggaaa atggccgctt ttctggattc atcgactgtg gccggctggg 3540
tgtggcggac cgctatcagg acatagcgtt ggctacccgt gatattgctg aagagcttgg 3600
cggcgaatgg gctgaccgct tcctcgtgct ttacggtatc gccgctcccg attcgcagcg 3660
catcgccttc tatcgccttc ttgacgagtt cttctgagcg ggactctggg gttcgaaatg 3720
accgaccaag cgacgcccaa cctgccatca cgagatttcg attccaccgc cgccttctat 3780
gaaaggttgg gcttcggaat cgttttccgg gacgccggct ggatgatcct ccagcgcggg 3840
gatctcatgc tggagttctt cgcccacccc aacttgttta ttgcagctta taatggttac 3900
aaataaagca atagcatcac aaatttcaca aataaagcat ttttttcact gcattctagt 3960
tgtggtttgt ccaaactcat caatgtatct tatcatgtct gtataccgtc gacctctagc 4020
tagagcttgg cgtaatcatg gtcatagctg tttcctgtgt gaaattgtta tccgctcaca 4080
attccacaca acatacgagc cggaagcata aagtgtaaag cctggggtgc ctaatgagtg 4140
agctaactca cattaattgc gttgcgctca ctgcccgctt tccagtcggg aaacctgtcg 4200
tgccagctgc attaatgaat cggccaacgc gcggggagag gcggtttgcg tattgggcgc 4260

tcttccgctt cctcgctcac tgactcgctg cgctcggtcg ttcggctgcg gcgagcggta 4320

tcagctcact caaaggcggt aatacggtta tccacagaat caggggataa cgcaggaaag 4380

aacatgtgag caaaaggcca gcaaaaggcc aggaaccgta aaaaggccgc gttgctggcg 4440

tttttccata ggctccgccc ccctgacgag catcacaaaa atcgacgctc aagtcagagg 4500

tggcgaaacc cgacaggact ataaagatac caggcgtttc cccctggaag ctccctcgtg 4560

cgctctcctg ttccgaccct gccgcttacc ggatacctgt ccgcctttct cccttcggga 4620

agcgtggcgc tttctcatag ctcacgctgt aggtatctca gttcggtgta ggtcgttcgc 4680

tccaagctgg gctgtgtgca cgaacccccc gttcagcccg accgctgcgc cttatccggt 4740

aactatcgtc ttgagtccaa cccggtaaga cacgacttat cgccactggc agcagccact 4800

ggtaacagga ttagcagagc gaggtatgta ggcggtgcta cagagttctt gaagtggtgg 4860

cctaactacg gctacactag aagaacagta tttggtatct gcgctctgct gaagccagtt 4920

accttcggaa aaagagttgg tagctcttga tccggcaaac aaaccaccgc tggtagcggt 4980

ttttttgttt gcaagcagca gattacgcgc agaaaaaaag gatctcaaga agatcctttg 5040

atcttttcta cggggtctga cgctcagtgg aacgaaaact cacgttaagg gattttggtc 5100

atgagattat caaaaaggat cttcacctag atccttttaa attaaaaatg aagttttaaa 5160

tcaatctaaa gtatatatga gtaaacttgg tctgacagtt accaatgctt aatcagtgag 5220

gcacctatct cagcgatctg tctatttcgt tcatccatag ttgcctgact ccccgtcgtg 5280

tagataacta cgatacggga gggcttacca tctggcccca gtgctgcaat gataccgcga 5340

gacccacgct caccggctcc agatttatca gcaataaacc agccagccgg aagggccgag 5400

cgcagaagtg gtcctgcaac tttatccgcc tccatccagt ctattaattg ttgccgggaa 5460

gctagagtaa gtagttcgcc agttaatagt ttgcgcaacg ttgttgccat tgctacaggc 5520

atcgtggtgt cacgctcgtc gtttggtatg gcttcattca gctccggttc ccaacgatca 5580

aggcgagtta catgatcccc catgttgtgc aaaaaagcgg ttagctcctt cggtcctccg 5640

atcgttgtca gaagtaagtt ggccgcagtg ttatcactca tggttatggc agcactgcat 5700

aattctctta ctgtcatgcc atccgtaaga tgcttttctg tgactggtga gtactcaacc 5760

aagtcattct gagaatagtg tatgcggcga ccgagttgct cttgcccggc gtcaatacgg 5820

gataataccg cgccacatag cagaacttta aaagtgctca tcattggaaa acgttcttcg 5880

gggcgaaaac tctcaaggat cttaccgctg ttgagatcca gttcgatgta acccactcgt 5940

gcacccaact gatcttcagc atcttttact ttcaccagcg tttctgggtg agcaaaaaca 6000

ggaaggcaaa atgccgcaaa aaagggaata agggcgacac ggaaatgttg aatactcata 6060

ctcttccttt ttcaatatta ttgaagcatt tatcagggtt attgtctcat gagcggatac 6120

atatttgaat gtatttagaa aaataaacaa ataggggttc cgcgcacatt tccccgaaaa 6180

gtgccacctg acgtc 6195

<210>  154
<211>  3567
<212>  DNA
<213>  plasmid pMM105

<400>  154
aattcgattt ccggtacttt tcagggcaat tgaagttccg gtcactactc ccccccagag      60

caataagcca catccggcga cgtgtggcac cccaccctgg ctgctacaga tggggctgga      120

tgcagaagag aactccagct ggtccttagg gacacggcgg ccttggcgct gaaggccact      180

cgctcccacc ttgtcctcac ggtccagttt tcccaggaat cccttagatg ctaagatggg      240

gattcctgga aatactgttc ttgaggtcat ggtttcacag ctggatttgc ctccttccca      300

ccccacagtt gccccccaat ggggcctcgg ctggctcaca ggatgagggt tcaagaagaa      360

ggctgtccct ggaggtaaga gggcttatga accatgttcc aaacctttgc gttgcttttc      420

tttccatcgt gtctatttca taacatccct gtgaggctgg atgtgggaac ttcagcactg      480

ccgtactctt gggaaatttg tccaaggcca cccggctgag cagcggttga accaggacac      540

atcaggcatg cgtttcttga atcactagtg aattcgcggc cgcctgcagg tcgaccatat      600

gggagagctc ccaacgcgtt ggatgcatag cttgagtatt ctatagtgtc acctaaatag      660

cttggcgtaa tcatggtcat agctgtttcc tgtgtgaaat tgttatccgc tcacaattcc      720

acacaacata cgagccggaa gcataaagtg taaagcctgg ggtgcctaat gagtgagcta      780

actcacatta attgcgttgc gctcactgcc cgctttccag tcgggaaacc tgtcgtgcca      840

gctgcattaa tgaatcggcc aacgcgcggg gagaggcggt ttgcgtattg ggcgctcttc      900

cgcttcctcg ctcactgact cgctgcgctc ggtcgttcgg ctgcggcgag cggtatcagc      960

tcactcaaag gcggtaatac ggttatccac agaatcaggg gataacgcag gaaagaacat     1020

gtgagcaaaa ggccagcaaa aggccaggaa ccgtaaaaag gccgcgttgc tggcgttttt     1080

ccataggctc cgcccccctg acgagcatca caaaaatcga cgctcaagtc agaggtggcg     1140

aaacccgaca ggactataaa gataccaggc gtttccccct ggaagctccc tcgtgcgctc     1200

tcctgttccg accctgccgc ttaccggata cctgtccgcc tttctccctt cgggaagcgt     1260

ggcgctttct catagctcac gctgtaggta tctcagttcg gtgtaggtcg ttcgctccaa     1320

gctgggctgt gtgcacgaac cccccgttca gcccgaccgc tgcgccttat ccggtaacta     1380

tcgtcttgag tccaacccgg taagacacga cttatcgcca ctggcagcag ccactggtaa     1440

caggattagc agagcgaggt atgtaggcgg tgctacagag ttcttgaagt ggtggcctaa     1500

ctacggctac actagaagaa cagtatttgg tatctgcgct ctgctgaagc cagttacctt     1560

cggaaaaaga gttggtagct cttgatccgg caaacaaacc accgctggta gcggtggttt     1620

ttttgtttgc aagcagcaga ttacgcgcag aaaaaaagga tctcaagaag atcctttgat     1680

cttttctacg gggtctgacg ctcagtggaa cgaaaactca cgttaaggga ttttggtcat     1740

gagattatca aaaaggatct tcacctagat ccttttaaat taaaaatgaa gttttaaatc     1800

aatctaaagt atatatgagt aaacttggtc tgacagttac caatgcttaa tcagtgaggc     1860

acctatctca gcgatctgtc tatttcgttc atccatagtt gcctgactcc ccgtcgtgta 1920

gataactacg atacgggagg gcttaccatc tggccccagt gctgcaatga taccgcgaga 1980

cccacgctca ccggctccag atttatcagc aataaaccag ccagccggaa gggccgagcg 2040

cagaagtggt cctgcaactt tatccgcctc catccagtct attaattgtt gccgggaagc 2100

tagagtaagt agttcgccag ttaatagttt gcgcaacgtt gttgccattg ctacaggcat 2160

cgtggtgtca cgctcgtcgt ttggtatggc ttcattcagc tccggttccc aacgatcaag 2220

gcgagttaca tgatccccca tgttgtgcaa aaaagcggtt agctccttcg gtcctccgat 2280

cgttgtcaga agtaagttgg ccgcagtgtt atcactcatg gttatggcag cactgcataa 2340

ttctcttact gtcatgccat ccgtaagatg cttttctgtg actggtgagt actcaaccaa 2400

gtcattctga gaatagtgta tgcggcgacc gagttgctct tgcccggcgt caatacggga 2460

taataccgcg ccacatagca gaactttaaa agtgctcatc attggaaaac gttcttcggg 2520

gcgaaaactc tcaaggatct taccgctgtt gagatccagt tcgatgtaac ccactcgtgc 2580

acccaactga tcttcagcat cttttacttt caccagcgtt tctgggtgag caaaaacagg 2640

aaggcaaaat gccgcaaaaa agggaataag ggcgacacgg aaatgttgaa tactcatact 2700

cttccttttt caatattatt gaagcattta tcagggttat tgtctcatga gcggatacat 2760

atttgaatgt atttagaaaa ataaacaaat aggggttccg cgcacatttc cccgaaaagt 2820

gccacctgat gcggtgtgaa ataccgcaca gatgcgtaag gagaaaatac cgcatcagga 2880

aattgtaagc gttaatattt tgttaaaatt cgcgttaaat ttttgttaaa tcagctcatt 2940

ttttaaccaa taggccgaaa tcggcaaaat cccttataaa tcaaaagaat agaccgagat 3000

agggttgagt gttgttccag tttggaacaa gagtccacta ttaaagaacg tggactccaa 3060

cgtcaaaggg cgaaaaaccg tctatcaggg cgatggccca ctacgtgaac catcacccta 3120

atcaagtttt ttggggtcga ggtgccgtaa agcactaaat cggaacccta aagggagccc 3180

ccgatttaga gcttgacggg gaaagccggc gaacgtggcg agaaaggaag ggaagaaagc 3240

gaaaggagcg ggcgctaggg cgctggcaag tgtagcggtc acgctgcgcg taaccaccac 3300

acccgccgcg cttaatgcgc cgctacaggg cgcgtccatt cgccattcag gctgcgcaac 3360

tgttgggaag ggcgatcggt gcgggcctct tcgctattac gccagctggc gaaaggggga 3420

tgtgctgcaa ggcgattaag ttgggtaacg ccagggtttt cccagtcacg acgttgtaaa 3480

acgacggcca gtgaattgta atacgactca ctatagggcg aattgggccc gacgtcgcat 3540

gctcccggcc gccatggcgg ccgcggg 3567

<210> 155
<211> 5998
<212> DNA
<213> plasmid pMM106

<400> 155
aattcactag tgattcaaga aacgcatgcc tgatgtgtcc tggttcaacc gctgctcagc 60

cgggtggcct tggacaaatt tcccaagagt acggcagtgc tgaagttccc acatccagcc 120

tcacagggat gttatgaaat agacacgatg gaaagaaaag caacgcaaag gtttggaaca 180
tggttcataa gccctcttac ctccagggac agccttcttc ttgaaccctc atcctgtgag 240
ccagccgagg ccccattggg gggcaactgt ggggtgggaa ggaggcaaat ccagctgtga 300
aaccatgacc tcaagaacag tatttccagg aatccccatc ttagcatcta agggattcct 360
gggaaaactg gaccgtgagg acaaggtggg agcgagtggc cttcagcgcc aaggccgccg 420
tgtccctaag gaccagctgg agttctcttc tgcatccagc cccatctgta gcagccaggg 480
tggggtgcca cacgtcgccg gatgtggctt attgctctgg gggggagtag tgaccggaac 540
ttcaattgcc ctgaaaagta ccggaaatcg aattctgcag atatccagca cagtggcggc 600
cgctcgagtc tagagggccc gtttaaaccc gctgatcagc ctcgactgtg ccttctagtt 660
gccagccatc tgttgtttgc ccctcccccg tgccttcctt gaccctggaa ggtgccactc 720
ccactgtcct ttcctaataa aatgaggaaa ttgcatcgca ttgtctgagt aggtgtcatt 780
ctattctggg gggtggggtg gggcaggaca gcaaggggga ggattgggaa gacaatagca 840
ggcatgctgg ggatgcggtg ggctctatgg cttctgaggc ggaaagaacc agctggggct 900
ctagggggta tccccacgcg ccctgtagcg gcgcattaag cgcggcgggt gtggtggtta 960
cgcgcagcgt gaccgctaca cttgccagcg ccctagcgcc cgctcctttc gctttcttcc 1020
cttcctttct cgccacgttc gccggctttc cccgtcaagc tctaaatcgg gggctccctt 1080
tagggttccg atttagtgct ttacggcacc tcgaccccaa aaaacttgat tagggtgatg 1140
gttcacgtag tgggccatcg ccctgataga cggtttttcg ccctttgacg ttggagtcca 1200
cgttctttaa tagtggactc ttgttccaaa ctggaacaac actcaaccct atctcggtct 1260
attcttttga tttataaggg attttgccga tttcggccta ttggttaaaa aatgagctga 1320
tttaacaaaa atttaacgcg aattaattct gtggaatgtg tgtcagttag ggtgtggaaa 1380
gtccccaggc tccccagcag gcagaagtat gcaaagcatg catctcaatt agtcagcaac 1440
caggtgtgga aagtccccag gctccccagc aggcagaagt atgcaaagca tgcatctcaa 1500
ttagtcagca accatagtcc cgcccctaac tccgcccatc ccgcccctaa ctccgcccag 1560
ttccgcccat tctccgcccc atggctgact aatttttttt atttatgcag aggccgaggc 1620
cgcctctgcc tctgagctat tccagaagta gtgaggaggc ttttttggag gcctaggctt 1680
ttgcaaaaag ctcccgggag cttgtatatc cattttcgga tctgatcaag agacaggatg 1740
aggatcgttt cgcatgattg aacaagatgg attgcacgca ggttctccgg ccgcttgggt 1800
ggagaggcta ttcggctatg actgggcaca acagacaatc ggctgctctg atgccgccgt 1860
gttccggctg tcagcgcagg ggcgcccggt tctttttgtc aagaccgacc tgtccggtgc 1920
cctgaatgaa ctgcaggacg aggcagcgcg gctatcgtgg ctggccacga cgggcgttcc 1980
ttgcgcagct gtgctcgacg ttgtcactga agcgggaagg gactggctgc tattgggcga 2040
agtgccgggg caggatctcc tgtcatctca ccttgctcct gccgagaaag tatccatcat 2100
ggctgatgca atgcggcggc tgcatacgct tgatccggct acctgcccat tcgaccacca 2160

agcgaaacat cgcatcgagc gagcacgtac tcggatggaa gccggtcttg tcgatcagga 2220
tgatctggac gaagagcatc aggggctcgc gccagccgaa ctgttcgcca ggctcaaggc 2280
gcgcatgccc gacggcgagg atctcgtcgt gacccatggc gatgcctgct tgccgaatat 2340
catggtggaa aatggccgct tttctggatt catcgactgt ggccggctgg gtgtggcgga 2400
ccgctatcag gacatagcgt tggctacccg tgatattgct gaagagcttg gcggcgaatg 2460
ggctgaccgc ttcctcgtgc tttacggtat cgccgctccc gattcgcagc gcatcgcctt 2520
ctatcgcctt cttgacgagt tcttctgagc gggactctgg ggttcgaaat gaccgaccaa 2580
gcgacgccca acctgccatc acgagatttc gattccaccg ccgccttcta tgaaaggttg 2640
ggcttcggaa tcgttttccg ggacgccggc tggatgatcc tccagcgcgg ggatctcatg 2700
ctggagttct tcgcccaccc caacttgttt attgcagctt ataatggtta caaataaagc 2760
aatagcatca caaatttcac aaataaagca tttttttcac tgcattctag ttgtggtttg 2820
tccaaactca tcaatgtatc ttatcatgtc tgtataccgt cgacctctag ctagagcttg 2880
gcgtaatcat ggtcatagct gtttcctgtg tgaaattgtt atccgctcac aattccacac 2940
aacatacgag ccggaagcat aaagtgtaaa gcctggggtg cctaatgagt gagctaactc 3000
acattaattg cgttgcgctc actgcccgct ttccagtcgg gaaacctgtc gtgccagctg 3060
cattaatgaa tcggccaacg cgcggggaga ggcggtttgc gtattgggcg ctcttccgct 3120
tcctcgctca ctgactcgct gcgctcggtc gttcggctgc ggcgagcggt atcagctcac 3180
tcaaaggcgg taatacggtt atccacagaa tcaggggata acgcaggaaa gaacatgtga 3240
gcaaaaggcc agcaaaaggc caggaaccgt aaaaaggccg cgttgctggc gtttttccat 3300
aggctccgcc cccctgacga gcatcacaaa aatcgacgct caagtcagag gtggcgaaac 3360
ccgacaggac tataaagata ccaggcgttt ccccctggaa gctccctcgt gcgctctcct 3420
gttccgaccc tgccgcttac cggatacctg tccgcctttc tcccttcggg aagcgtggcg 3480
ctttctcata gctcacgctg taggtatctc agttcggtgt aggtcgttcg ctccaagctg 3540
ggctgtgtgc acgaaccccc cgttcagccc gaccgctgcg ccttatccgg taactatcgt 3600
cttgagtcca acccggtaag acacgactta tcgccactgg cagcagccac tggtaacagg 3660
attagcagag cgaggtatgt aggcggtgct acagagttct tgaagtggtg gcctaactac 3720
ggctacacta gaagaacagt atttggtatc tgcgctctgc tgaagccagt taccttcgga 3780
aaaagagttg gtagctcttg atccggcaaa caaaccaccg ctggtagcgg tttttttgtt 3840
tgcaagcagc agattacgcg cagaaaaaaa ggatctcaag aagatccttt gatcttttct 3900
acggggtctg acgctcagtg gaacgaaaac tcacgttaag ggattttggt catgagatta 3960
tcaaaaagga tcttcaccta gatcctttta aattaaaaat gaagttttaa atcaatctaa 4020
agtatatatg agtaaacttg gtctgacagt taccaatgct taatcagtga ggcacctatc 4080
tcagcgatct gtctatttcg ttcatccata gttgcctgac tccccgtcgt gtagataact 4140
acgatacggg agggcttacc atctggcccc agtgctgcaa tgataccgcg agacccacgc 4200

tcaccggctc cagatttatc agcaataaac cagccagccg gaagggccga gcgcagaagt 4260

ggtcctgcaa ctttatccgc ctccatccag tctattaatt gttgccggga agctagagta 4320

agtagttcgc cagttaatag tttgcgcaac gttgttgcca ttgctacagg catcgtggtg 4380

tcacgctcgt cgtttggtat ggcttcattc agctccggtt cccaacgatc aaggcgagtt 4440

acatgatccc ccatgttgtg caaaaaagcg gttagctcct tcggtcctcc gatcgttgtc 4500

agaagtaagt tggccgcagt gttatcactc atggttatgg cagcactgca taattctctt 4560

actgtcatgc catccgtaag atgcttttct gtgactggtg agtactcaac caagtcattc 4620

tgagaatagt gtatgcggcg accgagttgc tcttgcccgg cgtcaatacg ggataatacc 4680

gcgccacata gcagaacttt aaaagtgctc atcattggaa aacgttcttc ggggcgaaaa 4740

ctctcaagga tcttaccgct gttgagatcc agttcgatgt aacccactcg tgcacccaac 4800

tgatcttcag catcttttac tttcaccagc gtttctgggt gagcaaaaac aggaaggcaa 4860

aatgccgcaa aaaagggaat aagggcgaca cggaaatgtt gaatactcat actcttcctt 4920

tttcaatatt attgaagcat ttatcagggt tattgtctca tgagcggata catatttgaa 4980

tgtatttaga aaaataaaca aataggggtt ccgcgcacat ttccccgaaa agtgccacct 5040

gacgtcgacg gatcgggaga tctcccgatc ccctatggtg cactctcagt acaatctgct 5100

ctgatgccgc atagttaagc cagtatctgc tccctgcttg tgtgttggag gtcgctgagt 5160

agtgcgcgag caaaatttaa gctacaacaa ggcaaggctt gaccgacaat tgcatgaaga 5220

atctgcttag ggttaggcgt tttgcgctgc ttcgcgatgt acgggccaga tatacgcgtt 5280

gacattgatt attgactagt tattaatagt aatcaattac ggggtcatta gttcatagcc 5340

catatatgga gttccgcgtt acataactta cggtaaatgg cccgcctggc tgaccgccca 5400

acgacccccg cccattgacg tcaataatga cgtatgttcc catagtaacg ccaataggga 5460

ctttccattg acgtcaatgg gtggagtatt tacggtaaac tgcccacttg gcagtacatc 5520

aagtgtatca tatgccaagt acgcccccta ttgacgtcaa tgacggtaaa tggcccgcct 5580

ggcattatgc ccagtacatg accttatggg actttcctac ttggcagtac atctacgtat 5640

tagtcatcgc tattaccatg gtgatgcggt tttggcagta catcaatggg cgtggatagc 5700

ggtttgactc acggggattt ccaagtctcc accccattga cgtcaatggg agtttgtttt 5760

ggcaccaaaa tcaacgggac tttccaaaat gtcgtaacaa ctccgcccca ttgacgcaaa 5820

tgggcggtag gcgtgtacgg tgggaggtct atataagcag agctctctgg ctaactagag 5880

aacccactgc ttactggctt atcgaaatta atacgactca ctatagggag acccaagctg 5940

gctagcgttt aaacttaagc ttggtaccga gctcggatcc actagtccag tgtggtgg 5998

<210> 156
<211> 6568
<212> DNA
<213> plasmid pMM107

<400> 156

aattcgattt ccggtacttt tcagggcaat tgaagttccg gtcactactc ccccccagag 60
caataagcca catccggcga cgtgtggcac cccaccctgg ctgctacaga tggggctgga 120
tgcagaagag aactccagct ggtccttagg gacacggcgg ccttggcgct gaaggccact 180
cgctcccacc ttgtcctcac ggtccagttt tcccaggaat cccttagatg ctaagatggg 240
gattcctgga aatactgttc ttgaggtcat ggtttcacag ctggatttgc ctccttccca 300
ccccacagtt gccccccaat ggggcctcgg ctggctcaca ggatgagggt tcaagaagaa 360
ggctgtccct ggaggtaaga gggcttatga accatgttcc aaacctttgc gttgcttttc 420
tttccatcgt gtctatttca taacatccct gtgaggctgg atgtgggaac ttcagcactg 480
ccgtactctt gggaaatttg tccaaggcca cccggctgag cagcggttga accaggacac 540
atcaggcatg cgtttcttga atcactagtg aattcgattt ccggtacttt tcagggcaat 600
tgaagttccg gtcactactc ccccccagag caataagcca catccggcga cgtgtggcac 660
cccaccctgg ctgctacaga tggggctgga tgcagaagag aactccagct ggtccttagg 720
gacacggcgg ccttggcgct gaaggccact cgctcccacc ttgtcctcac ggtccagttt 780
tcccaggaat cccttagatg ctaagatggg gattcctgga aatactgttc ttgaggtcat 840
ggtttcacag ctggatttgc ctccttccca ccccacagtt gccccccaat ggggcctcgg 900
ctggctcaca ggatgagggt tcaagaagaa ggctgtccct ggaggtaaga gggcttatga 960
accatgttcc aaacctttgc gttgcttttc tttccatcgt gtctatttca taacatccct 1020
gtgaggctgg atgtgggaac ttcagcactg ccgtactctt gggaaatttg tccaaggcca 1080
cccggctgag cagcggttga accaggacac atcaggcatg cgtttcttga atcactagtg 1140
aattctgcag atatccagca cagtggcggc cgctcgagtc tagagggccc gtttaaaccc 1200
gctgatcagc ctcgactgtg ccttctagtt gccagccatc tgttgtttgc ccctcccccg 1260
tgccttcctt gaccctggaa ggtgccactc ccactgtcct ttcctaataa aatgaggaaa 1320
ttgcatcgca ttgtctgagt aggtgtcatt ctattctggg gggtggggtg gggcaggaca 1380
gcaaggggga ggattgggaa gacaatagca ggcatgctgg ggatgcggtg ggctctatgg 1440
cttctgaggc ggaaagaacc agctggggct ctagggggta tccccacgcg ccctgtagcg 1500
gcgcattaag cgcggcgggt gtggtggtta cgcgcagcgt gaccgctaca cttgccagcg 1560
ccctagcgcc cgctcctttc gctttcttcc cttcctttct cgccacgttc gccggctttc 1620
cccgtcaagc tctaaatcgg gggctccctt tagggttccg atttagtgct ttacggcacc 1680
tcgaccccaa aaaacttgat tagggtgatg gttcacgtag tgggccatcg ccctgataga 1740
cggtttttcg ccctttgacg ttggagtcca cgttctttaa tagtggactc ttgttccaaa 1800
ctggaacaac actcaaccct atctcggtct attcttttga tttataaggg attttgccga 1860
tttcggccta ttggttaaaa aatgagctga tttaacaaaa atttaacgcg aattaattct 1920
gtggaatgtg tgtcagttag ggtgtggaaa gtccccaggc tccccagcag gcagaagtat 1980
gcaaagcatg catctcaatt agtcagcaac caggtgtgga aagtccccag gctccccagc 2040

aggcagaagt atgcaaagca tgcatctcaa ttagtcagca accatagtcc cgcccctaac 2100
tccgcccatc ccgcccctaa ctccgcccag ttccgcccat tctccgcccc atggctgact 2160
aatttttttt atttatgcag aggccgaggc cgcctctgcc tctgagctat tccagaagta 2220
gtgaggaggc ttttttggag gcctaggctt ttgcaaaaag ctcccgggag cttgtatatc 2280
cattttcgga tctgatcaag agacaggatg aggatcgttt cgcatgattg aacaagatgg 2340
attgcacgca ggttctccgg ccgcttgggt ggagaggcta ttcggctatg actgggcaca 2400
acagacaatc ggctgctctg atgccgccgt gttccggctg tcagcgcagg ggcgcccggt 2460
tctttttgtc aagaccgacc tgtccggtgc cctgaatgaa ctgcaggacg aggcagcgcg 2520
gctatcgtgg ctggccacga cgggcgttcc ttgcgcagct gtgctcgacg ttgtcactga 2580
agcgggaagg gactggctgc tattgggcga agtgccgggg caggatctcc tgtcatctca 2640
ccttgctcct gccgagaaag tatccatcat ggctgatgca atgcggcggc tgcatacgct 2700
tgatccggct acctgcccat tcgaccacca agcgaaacat cgcatcgagc gagcacgtac 2760
tcggatggaa gccggtcttg tcgatcagga tgatctggac gaagagcatc aggggctcgc 2820
gccagccgaa ctgttcgcca ggctcaaggc gcgcatgccc gacggcgagg atctcgtcgt 2880
gacccatggc gatgcctgct tgccgaatat catggtggaa aatggccgct tttctggatt 2940
catcgactgt ggccggctgg gtgtggcgga ccgctatcag gacatagcgt tggctacccg 3000
tgatattgct gaagagcttg gcggcgaatg ggctgaccgc ttcctcgtgc tttacggtat 3060
cgccgctccc gattcgcagc gcatcgcctt ctatcgcctt cttgacgagt tcttctgagc 3120
gggactctgg ggttcgaaat gaccgaccaa gcgacgccca acctgccatc acgagatttc 3180
gattccaccg ccgccttcta tgaaaggttg ggcttcggaa tcgttttccg ggacgccggc 3240
tggatgatcc tccagcgcgg ggatctcatg ctggagttct tcgcccaccc caacttgttt 3300
attgcagctt ataatggtta caaataaagc aatagcatca caaatttcac aaataaagca 3360
tttttttcac tgcattctag ttgtggtttg tccaaactca tcaatgtatc ttatcatgtc 3420
tgtataccgt cgacctctag ctagagcttg gcgtaatcat ggtcatagct gtttcctgtg 3480
tgaaattgtt atccgctcac aattccacac aacatacgag ccggaagcat aaagtgtaaa 3540
gcctggggtg cctaatgagt gagctaactc acattaattg cgttgcgctc actgcccgct 3600
ttccagtcgg gaaacctgtc gtgccagctg cattaatgaa tcggccaacg cgcggggaga 3660
ggcggtttgc gtattgggcg ctcttccgct tcctcgctca ctgactcgct gcgctcggtc 3720
gttcggctgc ggcgagcggt atcagctcac tcaaaggcgg taatacggtt atccacagaa 3780
tcaggggata acgcaggaaa gaacatgtga gcaaaaggcc agcaaaaggc caggaaccgt 3840
aaaaaggccg cgttgctggc gtttttccat aggctccgcc cccctgacga gcatcacaaa 3900
aatcgacgct caagtcagag gtggcgaaac ccgacaggac tataaagata ccaggcgttt 3960
ccccctggaa gctccctcgt gcgctctcct gttccgaccc tgccgcttac cggatacctg 4020
tccgcctttc tcccttcggg aagcgtggcg ctttctcata gctcacgctg taggtatctc 4080

agttcggtgt aggtcgttcg ctccaagctg ggctgtgtgc acgaaccccc cgttcagccc 4140
gaccgctgcg ccttatccgg taactatcgt cttgagtcca acccggtaag acacgactta 4200
tcgccactgg cagcagccac tggtaacagg attagcagag cgaggtatgt aggcggtgct 4260
acagagttct tgaagtggtg gcctaactac ggctacacta gaagaacagt atttggtatc 4320
tgcgctctgc tgaagccagt taccttcgga aaaagagttg gtagctcttg atccggcaaa 4380
caaaccaccg ctggtagcgg tttttttgtt tgcaagcagc agattacgcg cagaaaaaaa 4440
ggatctcaag aagatccttt gatcttttct acggggtctg acgctcagtg gaacgaaaac 4500
tcacgttaag ggattttggt catgagatta tcaaaaagga tcttcaccta gatcctttta 4560
aattaaaaat gaagttttaa atcaatctaa agtatatatg agtaaacttg gtctgacagt 4620
taccaatgct taatcagtga ggcacctatc tcagcgatct gtctatttcg ttcatccata 4680
gttgcctgac tccccgtcgt gtagataact acgatacggg agggcttacc atctggcccc 4740
agtgctgcaa tgataccgcg agacccacgc tcaccggctc cagatttatc agcaataaac 4800
cagccagccg gaagggccga gcgcagaagt ggtcctgcaa ctttatccgc ctccatccag 4860
tctattaatt gttgccggga agctagagta agtagttcgc cagttaatag tttgcgcaac 4920
gttgttgcca ttgctacagg catcgtggtg tcacgctcgt cgtttggtat ggcttcattc 4980
agctccggtt cccaacgatc aaggcgagtt acatgatccc ccatgttgtg caaaaaagcg 5040
gttagctcct tcggtcctcc gatcgttgtc agaagtaagt tggccgcagt gttatcactc 5100
atggttatgg cagcactgca taattctctt actgtcatgc catccgtaag atgcttttct 5160
gtgactggtg agtactcaac caagtcattc tgagaatagt gtatgcggcg accgagttgc 5220
tcttgcccgg cgtcaatacg ggataatacc gcgccacata gcagaacttt aaaagtgctc 5280
atcattggaa aacgttcttc ggggcgaaaa ctctcaagga tcttaccgct gttgagatcc 5340
agttcgatgt aacccactcg tgcacccaac tgatcttcag catcttttac tttcaccagc 5400
gtttctgggt gagcaaaaac aggaaggcaa aatgccgcaa aaaagggaat aagggcgaca 5460
cggaaatgtt gaatactcat actcttcctt tttcaatatt attgaagcat ttatcagggt 5520
tattgtctca tgagcggata catatttgaa tgtatttaga aaaataaaca aataggggtt 5580
ccgcgcacat ttccccgaaa agtgccacct gacgtcgacg gatcgggaga tctcccgatc 5640
ccctatggtg cactctcagt acaatctgct ctgatgccgc atagttaagc cagtatctgc 5700
tccctgcttg tgtgttggag gtcgctgagt agtgcgcgag caaaatttaa gctacaacaa 5760
ggcaaggctt gaccgacaat tgcatgaaga atctgcttag ggttaggcgt tttgcgctgc 5820
ttcgcgatgt acgggccaga tatacgcgtt gacattgatt attgactagt tattaatagt 5880
aatcaattac ggggtcatta gttcatagcc catatatgga gttccgcgtt acataactta 5940
cggtaaatgg cccgcctggc tgaccgccca acgacccccg cccattgacg tcaataatga 6000
cgtatgttcc catagtaacg ccaataggga ctttccattg acgtcaatgg gtggagtatt 6060
tacggtaaac tgcccacttg gcagtacatc aagtgtatca tatgccaagt acgcccccta 6120

ttgacgtcaa tgacggtaaa tggcccgcct ggcattatgc ccagtacatg accttatggg 6180

actttcctac ttggcagtac atctacgtat tagtcatcgc tattaccatg gtgatgcggt 6240

tttggcagta catcaatggg cgtggatagc ggtttgactc acggggattt ccaagtctcc 6300

accccattga cgtcaatggg agtttgtttt ggcaccaaaa tcaacgggac tttccaaaat 6360

gtcgtaacaa ctccgcccca ttgacgcaaa tgggcggtag gcgtgtacgg tgggaggtct 6420

atataagcag agctctctgg ctaactagag aacccactgc ttactggctt atcgaaatta 6480

atacgactca ctatagggag acccaagctg gctagcgttt aaacttaagc ttggtaccga 6540

gctcggatcc actagtccag tgtggtgg 6568

<210> 157
<211> 5998
<212> DNA
<213> plasmid pMM109

<400> 157
aattcgattt ccggtacttt tcagggcaat tgaagttccg gtcactactc ccccccagag 60

caataagcca catccggcga cgtgtggcac cccaccctgg ctgctacaga tggggctgga 120

tgcagaagag aactccagct ggtccttagg gacacggcgg ccttggcgct gaaggccact 180

cgctcccacc ttgtcctcac ggtccagttt tcccaggaat cccttagatg ctaagatggg 240

gattcctgga aatactgttc ttgaggtcat ggtttcacag ctggatttgc ctccttccca 300

ccccacagtt gccccccaat ggggcctcgg ctggctcaca ggatgagggt tcaagaagaa 360

ggctgtccct ggaggtaaga gggcttatga accatgttcc aaacctttgc gttgcttttc 420

tttccatcgt gtctatttca taacatccct gtgaggctgg atgtgggaac ttcagcactg 480

ccgtactctt gggaaatttg tccaaggcca cccggctgag cagcggttga accaggacac 540

atcaggcatg cgtttcttga atcactagtg aattctgcag atatccagca cagtggcggc 600

cgctcgagtc tagagggccc gtttaaaccc gctgatcagc ctcgactgtg ccttctagtt 660

gccagccatc tgttgtttgc ccctcccccg tgccttcctt gaccctggaa ggtgccactc 720

ccactgtcct ttcctaataa aatgaggaaa ttgcatcgca ttgtctgagt aggtgtcatt 780

ctattctggg gggtggggtg gggcaggaca gcaaggggga ggattgggaa gacaatagca 840

ggcatgctgg ggatgcggtg ggctctatgg cttctgaggc ggaaagaacc agctggggct 900

ctagggggta tccccacgcg ccctgtagcg gcgcattaag cgcggcgggt gtggtggtta 960

cgcgcagcgt gaccgctaca cttgccagcg ccctagcgcc cgctcctttc gctttcttcc 1020

cttcctttct cgccacgttc gccggctttc cccgtcaagc tctaaatcgg gggctccctt 1080

tagggttccg atttagtgct ttacggcacc tcgaccccaa aaaacttgat tagggtgatg 1140

gttcacgtag tgggccatcg ccctgataga cggtttttcg ccctttgacg ttggagtcca 1200

cgttctttaa tagtggactc ttgttccaaa ctggaacaac actcaaccct atctcggtct 1260

attcttttga tttataaggg attttgccga tttcggccta ttggttaaaa aatgagctga 1320

tttaacaaaa atttaacgcg aattaattct gtggaatgtg tgtcagttag ggtgtggaaa 1380

gtccccaggc tccccagcag gcagaagtat gcaaagcatg catctcaatt agtcagcaac 1440
caggtgtgga aagtccccag gctccccagc aggcagaagt atgcaaagca tgcatctcaa 1500
ttagtcagca accatagtcc cgcccctaac tccgcccatc ccgcccctaa ctccgcccag 1560
ttccgcccat tctccgcccc atggctgact aatttttttt atttatgcag aggccgaggc 1620
cgcctctgcc tctgagctat tccagaagta gtgaggaggc ttttttggag gcctaggctt 1680
ttgcaaaaag ctcccgggag cttgtatatc cattttcgga tctgatcaag agacaggatg 1740
aggatcgttt cgcatgattg aacaagatgg attgcacgca ggttctccgg ccgcttgggt 1800
ggagaggcta ttcggctatg actgggcaca acagacaatc ggctgctctg atgccgccgt 1860
gttccggctg tcagcgcagg ggcgcccggt tctttttgtc aagaccgacc tgtccggtgc 1920
cctgaatgaa ctgcaggacg aggcagcgcg gctatcgtgg ctggccacga cgggcgttcc 1980
ttgcgcagct gtgctcgacg ttgtcactga agcgggaagg gactggctgc tattgggcga 2040
agtgccgggg caggatctcc tgtcatctca ccttgctcct gccgagaaag tatccatcat 2100
ggctgatgca atgcggcggc tgcatacgct tgatccggct acctgcccat tcgaccacca 2160
agcgaaacat cgcatcgagc gagcacgtac tcggatggaa gccggtcttg tcgatcagga 2220
tgatctggac gaagagcatc aggggctcgc gccagccgaa ctgttcgcca ggctcaaggc 2280
gcgcatgccc gacggcgagg atctcgtcgt gacccatggc gatgcctgct tgccgaatat 2340
catggtggaa aatggccgct tttctggatt catcgactgt ggccggctgg gtgtggcgga 2400
ccgctatcag gacatagcgt tggctacccg tgatattgct gaagagcttg gcggcgaatg 2460
ggctgaccgc ttcctcgtgc tttacggtat cgccgctccc gattcgcagc gcatcgcctt 2520
ctatcgcctt cttgacgagt tcttctgagc gggactctgg ggttcgaaat gaccgaccaa 2580
gcgacgccca acctgccatc acgagatttc gattccaccg ccgccttcta tgaaaggttg 2640
ggcttcggaa tcgttttccg ggacgccggc tggatgatcc tccagcgcgg ggatctcatg 2700
ctggagttct tcgcccaccc caacttgttt attgcagctt ataatggtta caaataaagc 2760
aatagcatca caaatttcac aaataaagca tttttttcac tgcattctag ttgtggtttg 2820
tccaaactca tcaatgtatc ttatcatgtc tgtataccgt cgacctctag ctagagcttg 2880
gcgtaatcat ggtcatagct gtttcctgtg tgaaattgtt atccgctcac aattccacac 2940
aacatacgag ccggaagcat aaagtgtaaa gcctggggtg cctaatgagt gagctaactc 3000
acattaattg cgttgcgctc actgcccgct ttccagtcgg gaaacctgtc gtgccagctg 3060
cattaatgaa tcggccaacg cgcggggaga ggcggtttgc gtattgggcg ctcttccgct 3120
tcctcgctca ctgactcgct gcgctcggtc gttcggctgc ggcgagcggt atcagctcac 3180
tcaaaggcgg taatacggtt atccacagaa tcaggggata acgcaggaaa gaacatgtga 3240
gcaaaaggcc agcaaaaggc caggaaccgt aaaaaggccg cgttgctggc gtttttccat 3300
aggctccgcc cccctgacga gcatcacaaa aatcgacgct caagtcagag gtggcgaaac 3360
ccgacaggac tataaagata ccaggcgttt ccccctggaa gctccctcgt gcgctctcct 3420

gttccgaccc tgccgcttac cggatacctg tccgcctttc tcccttcggg aagcgtggcg 3480
ctttctcata gctcacgctg taggtatctc agttcggtgt aggtcgttcg ctccaagctg 3540
ggctgtgtgc acgaaccccc cgttcagccc gaccgctgcg ccttatccgg taactatcgt 3600
cttgagtcca acccggtaag acacgactta tcgccactgg cagcagccac tggtaacagg 3660
attagcagag cgaggtatgt aggcggtgct acagagttct tgaagtggtg gcctaactac 3720
ggctacacta gaagaacagt atttggtatc tgcgctctgc tgaagccagt taccttcgga 3780
aaaagagttg gtagctcttg atccggcaaa caaaccaccg ctggtagcgg tttttttgtt 3840
tgcaagcagc agattacgcg cagaaaaaaa ggatctcaag aagatccttt gatcttttct 3900
acggggtctg acgctcagtg gaacgaaaac tcacgttaag ggattttggt catgagatta 3960
tcaaaaagga tcttcaccta gatcctttta aattaaaaat gaagttttaa atcaatctaa 4020
agtatatatg agtaaacttg gtctgacagt taccaatgct taatcagtga ggcacctatc 4080
tcagcgatct gtctatttcg ttcatccata gttgcctgac tccccgtcgt gtagataact 4140
acgatacggg agggcttacc atctggcccc agtgctgcaa tgataccgcg agacccacgc 4200
tcaccggctc cagatttatc agcaataaac cagccagccg gaagggccga gcgcagaagt 4260
ggtcctgcaa ctttatccgc ctccatccag tctattaatt gttgccggga agctagagta 4320
agtagttcgc cagttaatag tttgcgcaac gttgttgcca ttgctacagg catcgtggtg 4380
tcacgctcgt cgtttggtat ggcttcattc agctccggtt cccaacgatc aaggcgagtt 4440
acatgatccc ccatgttgtg caaaaaagcg gttagctcct tcggtcctcc gatcgttgtc 4500
agaagtaagt tggccgcagt gttatcactc atggttatgg cagcactgca taattctctt 4560
actgtcatgc catccgtaag atgcttttct gtgactggtg agtactcaac caagtcattc 4620
tgagaatagt gtatgcggcg accgagttgc tcttgcccgg cgtcaatacg ggataatacc 4680
gcgccacata gcagaacttt aaaagtgctc atcattggaa aacgttcttc ggggcgaaaa 4740
ctctcaagga tcttaccgct gttgagatcc agttcgatgt aacccactcg tgcacccaac 4800
tgatcttcag catcttttac tttcaccagc gtttctgggt gagcaaaaac aggaaggcaa 4860
aatgccgcaa aaaagggaat aagggcgaca cggaaatgtt gaatactcat actcttcctt 4920
tttcaatatt attgaagcat ttatcagggt tattgtctca tgagcggata catatttgaa 4980
tgtatttaga aaaataaaca aataggggtt ccgcgcacat ttccccgaaa agtgccacct 5040
gacgtcgacg gatcgggaga tctcccgatc ccctatggtg cactctcagt acaatctgct 5100
ctgatgccgc atagttaagc cagtatctgc tccctgcttg tgtgttggag gtcgctgagt 5160
agtgcgcgag caaaatttaa gctacaacaa ggcaaggctt gaccgacaat tgcatgaaga 5220
atctgcttag ggttaggcgt tttgcgctgc ttcgcgatgt acgggccaga tatacgcgtt 5280
gacattgatt attgactagt tattaatagt aatcaattac ggggtcatta gttcatagcc 5340
catatatgga gttccgcgtt acataactta cggtaaatgg cccgcctggc tgaccgccca 5400
acgacccccg cccattgacg tcaataatga cgtatgttcc catagtaacg ccaataggga 5460

ctttccattg acgtcaatgg gtggagtatt tacggtaaac tgcccacttg gcagtacatc 5520

aagtgtatca tatgccaagt acgcccccta ttgacgtcaa tgacggtaaa tggcccgcct 5580

ggcattatgc ccagtacatg accttatggg actttcctac ttggcagtac atctacgtat 5640

tagtcatcgc tattaccatg gtgatgcggt tttggcagta catcaatggg cgtggatagc 5700

ggtttgactc acggggattt ccaagtctcc accccattga cgtcaatggg agtttgtttt 5760

ggcaccaaaa tcaacgggac tttccaaaat gtcgtaacaa ctccgcccca ttgacgcaaa 5820

tgggcggtag gcgtgtacgg tgggaggtct atataagcag agctctctgg ctaactagag 5880

aacccactgc ttactggctt atcgaaatta atacgactca ctatagggag acccaagctg 5940

gctagcgttt aaacttaagc ttggtaccga gctcggatcc actagtccag tgtggtgg 5998

<210> 158
<211> 6606
<212> DNA
<213> plasmid pMM-TK/miRTarg

<400> 158
gacggatcgg gagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggag tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctagc 900

gtttaaactt aagcttggta ccgagctcgg atctcgagct caagcttcga attctgcagt 960

cgacggtacc gcgggcccgg gatccaccgg tcgccaccat ggcttctcac gccggccaac 1020

agcacgcgcc tgcgttcggt caggctgctc gtgcgagcgg gcctaccgac ggccgcgcgg 1080

cgtcccgtcc tagccatcgc cagggggcct ccggagcccg cggggatccg gagctgccca 1140

cgctgctgcg ggtttatata gacggacccc acggggtggg gaagaccacc acctccgcgc 1200

agctgatgga ggccctgggg ccgcgcgaca atatcgtcta cgtccccgag ccgatgactt 1260

actggcaggt gctgggggcc tccgagaccc tgacgaacat ctacaacacg cagcaccgtc 1320
tggaccgcgg cgagatatcg gccggggagg cggcggtggt aatgaccagc gcccagataa 1380
caatgagcac gccttatgcg gcgacggacg ccgttttggc tcctcatatc gggggggagg 1440
ctgtgggccc gcaagccccg cccccggccc tcacccttgt tttcgaccgg caccctatcg 1500
cctccctgct gtgctacccg gccgcgcggt acctcatggg aagcatgacc ccccaggccg 1560
tgttggcgtt cgtggccctc atgcccccga ccgcgcccgg cacgaacctg gtcctgggtg 1620
tccttccgga ggccgaacac gccgaccgcc tggccagacg ccaacgcccg ggcgagcggc 1680
ttgacctggc catgctgtcc gccattcgcc gtgtctacga tctactcgcc aacacggtgc 1740
ggtacctgca gcgcggcggg aggtggcggg aggactgggg ccggctgacg ggggtcgccg 1800
cggcgacccc gcgccccgac cccgaggacg gcgcggggtc tctgccccgc atcgaggaca 1860
cgctgtttgc cctgttccgc gttcccgagc tgctggcccc caacggggac ttgtaccaca 1920
tttttgcctg ggtcttggac gtcttggccg accgcctcct tccgatgcat ctatttgtcc 1980
tggattacga tcagtcgccc gtcgggtgtc gagacgccct gttgcgcctc accgccggga 2040
tgatcccaac ccgcgtcaca accgccgggt ccatcgccga gatacgcgac ctggcgcgca 2100
cgtttgcccg cgaggtgggg ggagtttaga gcggccgctc gagtctagca gatcctggga 2160
aaactggacc tagagggccc gtttaaaccc gctgatcagc ctcgactgtg ccttctagtt 2220
gccagccatc tgttgtttgc ccctcccccg tgccttcctt gaccctggaa ggtgccactc 2280
ccactgtcct ttcctaataa aatgaggaaa ttgcatcgca ttgtctgagt aggtgtcatt 2340
ctattctggg gggtggggtg gggcaggaca gcaaggggga ggattgggaa gacaatagca 2400
ggcatgctgg ggatgcggtg ggctctatgg cttctgaggc ggaaagaacc agctggggct 2460
ctagggggta tccccacgcg ccctgtagcg gcgcattaag cgcggcgggt gtggtggtta 2520
cgcgcagcgt gaccgctaca cttgccagcg ccctagcgcc cgctcctttc gctttcttcc 2580
cttcctttct cgccacgttc gccggctttc cccgtcaagc tctaaatcgg gggctccctt 2640
tagggttccg atttagtgct ttacggcacc tcgaccccaa aaaacttgat tagggtgatg 2700
gttcacgtag tgggccatcg ccctgataga cggtttttcg ccctttgacg ttggagtcca 2760
cgttctttaa tagtggactc ttgttccaaa ctggaacaac actcaaccct atctcggtct 2820
attcttttga tttataaggg attttgccga tttcggccta ttggttaaaa aatgagctga 2880
tttaacaaaa atttaacgcg aattaattct gtggaatgtg tgtcagttag ggtgtggaaa 2940
gtccccaggc tccccagcag gcagaagtat gcaaagcatg catctcaatt agtcagcaac 3000
caggtgtgga aagtccccag gctccccagc aggcagaagt atgcaaagca tgcatctcaa 3060
ttagtcagca accatagtcc cgcccctaac tccgcccatc ccgcccctaa ctccgcccag 3120
ttccgcccat tctccgcccc atggctgact aatttttttt atttatgcag aggccgaggc 3180
cgcctctgcc tctgagctat tccagaagta gtgaggaggc ttttttggag gcctaggctt 3240
ttgcaaaaag ctcccgggag cttgtatatc cattttcgga tctgatcaag agacaggatg 3300

aggatcgttt cgcatgattg aacaagatgg attgcacgca ggttctccgg ccgcttgggt 3360
ggagaggcta ttcggctatg actgggcaca acagacaatc ggctgctctg atgccgccgt 3420
gttccggctg tcagcgcagg ggcgcccggt tctttttgtc aagaccgacc tgtccggtgc 3480
cctgaatgaa ctgcaggacg aggcagcgcg gctatcgtgg ctggccacga cgggcgttcc 3540
ttgcgcagct gtgctcgacg ttgtcactga agcgggaagg gactggctgc tattgggcga 3600
agtgccgggg caggatctcc tgtcatctca ccttgctcct gccgagaaag tatccatcat 3660
ggctgatgca atgcggcggc tgcatacgct tgatccggct acctgcccat tcgaccacca 3720
agcgaaacat cgcatcgagc gagcacgtac tcggatggaa gccggtcttg tcgatcagga 3780
tgatctggac gaagagcatc aggggctcgc gccagccgaa ctgttcgcca ggctcaaggc 3840
gcgcatgccc gacggcgagg atctcgtcgt gacccatggc gatgcctgct tgccgaatat 3900
catggtggaa aatggccgct tttctggatt catcgactgt ggccggctgg gtgtggcgga 3960
ccgctatcag gacatagcgt tggctacccg tgatattgct gaagagcttg gcggcgaatg 4020
ggctgaccgc ttcctcgtgc tttacggtat cgccgctccc gattcgcagc gcatcgcctt 4080
ctatcgcctt cttgacgagt tcttctgagc gggactctgg ggttcgaaat gaccgaccaa 4140
gcgacgccca acctgccatc acgagatttc gattccaccg ccgccttcta tgaaaggttg 4200
ggcttcggaa tcgttttccg ggacgccggc tggatgatcc tccagcgcgg ggatctcatg 4260
ctggagttct tcgcccaccc caacttgttt attgcagctt ataatggtta caaataaagc 4320
aatagcatca caaatttcac aaataaagca tttttttcac tgcattctag ttgtggtttg 4380
tccaaactca tcaatgtatc ttatcatgtc tgtataccgt cgacctctag ctagagcttg 4440
gcgtaatcat ggtcatagct gtttcctgtg tgaaattgtt atccgctcac aattccacac 4500
aacatacgag ccggaagcat aaagtgtaaa gcctggggtg cctaatgagt gagctaactc 4560
acattaattg cgttgcgctc actgcccgct ttccagtcgg gaaacctgtc gtgccagctg 4620
cattaatgaa tcggccaacg cgcggggaga ggcggtttgc gtattgggcg ctcttccgct 4680
tcctcgctca ctgactcgct gcgctcggtc gttcggctgc ggcgagcggt atcagctcac 4740
tcaaaggcgg taatacggtt atccacagaa tcaggggata acgcaggaaa gaacatgtga 4800
gcaaaaggcc agcaaaaggc caggaaccgt aaaaaggccg cgttgctggc gtttttccat 4860
aggctccgcc cccctgacga gcatcacaaa aatcgacgct caagtcagag gtggcgaaac 4920
ccgacaggac tataaagata ccaggcgttt ccccctggaa gctccctcgt gcgctctcct 4980
gttccgaccc tgccgcttac cggatacctg tccgcctttc tcccttcggg aagcgtggcg 5040
ctttctcata gctcacgctg taggtatctc agttcggtgt aggtcgttcg ctccaagctg 5100
ggctgtgtgc acgaaccccc cgttcagccc gaccgctgcg ccttatccgg taactatcgt 5160
cttgagtcca acccggtaag acacgactta tcgccactgg cagcagccac tggtaacagg 5220
attagcagag cgaggtatgt aggcggtgct acagagttct tgaagtggtg gcctaactac 5280
ggctacacta gaagaacagt atttggtatc tgcgctctgc tgaagccagt taccttcgga 5340

aaaagagttg gtagctcttg atccggcaaa caaaccaccg ctggtagcgg tttttttgtt 5400

tgcaagcagc agattacgcg cagaaaaaaa ggatctcaag aagatccttt gatcttttct 5460

acggggtctg acgctcagtg gaacgaaaac tcacgttaag ggattttggt catgagatta 5520

tcaaaaagga tcttcaccta gatcctttta aattaaaaat gaagttttaa atcaatctaa 5580

agtatatatg agtaaacttg gtctgacagt taccaatgct taatcagtga ggcacctatc 5640

tcagcgatct gtctatttcg ttcatccata gttgcctgac tccccgtcgt gtagataact 5700

acgatacggg agggcttacc atctggcccc agtgctgcaa tgataccgcg agacccacgc 5760

tcaccggctc cagatttatc agcaataaac cagccagccg gaagggccga gcgcagaagt 5820

ggtcctgcaa ctttatccgc ctccatccag tctattaatt gttgccggga agctagagta 5880

agtagttcgc cagttaatag tttgcgcaac gttgttgcca ttgctacagg catcgtggtg 5940

tcacgctcgt cgtttggtat ggcttcattc agctccggtt cccaacgatc aaggcgagtt 6000

acatgatccc ccatgttgtg caaaaaagcg gttagctcct tcggtcctcc gatcgttgtc 6060

agaagtaagt tggccgcagt gttatcactc atggttatgg cagcactgca taattctctt 6120

actgtcatgc catccgtaag atgcttttct gtgactggtg agtactcaac caagtcattc 6180

tgagaatagt gtatgcggcg accgagttgc tcttgcccgg cgtcaatacg ggataatacc 6240

gcgccacata gcagaacttt aaaagtgctc atcattggaa aacgttcttc ggggcgaaaa 6300

ctctcaagga tcttaccgct gttgagatcc agttcgatgt aacccactcg tgcacccaac 6360

tgatcttcag catcttttac tttcaccagc gtttctgggt gagcaaaaac aggaaggcaa 6420

aatgccgcaa aaaagggaat aagggcgaca cggaaatgtt gaatactcat actcttcctt 6480

tttcaatatt attgaagcat ttatcagggt tattgtctca tgagcggata catatttgaa 6540

tgtatttaga aaaataaaca aataggggtt ccgcgcacat ttccccgaaa agtgccacct 6600

gacgtc 6606

<210> 159
<211> 6759
<212> DNA
<213> plasmid pMM1-CD/miRTarg

<400> 159
gacggatcgg gagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggag tatttacggt aaactgccca cttggcagta catcaagtgt 480

atcatatgcc aagtacgccc cctattgacg tcaatgacgg taaatggccc gcctggcatt 540

atgcccagta catgacctta tgggactttc ctacttggca gtacatctac gtattagtca 600

tcgctattac catggtgatg cggttttggc agtacatcaa tgggcgtgga tagcggtttg 660

actcacgggg atttccaagt ctccacccca ttgacgtcaa tgggagtttg ttttggcacc 720

aaaatcaacg ggactttcca aaatgtcgta acaactccgc cccattgacg caaatgggcg 780

gtaggcgtgt acggtgggag gtctatataa gcagagctct ctggctaact agagaaccca 840

ctgcttactg gcttatcgaa attaatacga ctcactatag ggagacccaa gctggctagc 900

gtttaaactt aagcttggta ccgagctcgg atctcgagct caagcttcga attctgcagt 960

cgacggtacc gcgggcccgg gatccaccgg tcgccaccat gtcgaataac gctttacaaa 1020

caattattaa cgcccggtta ccaggcgaag aggggctgtg gcagattcat ctgcaggacg 1080

gaaaaatcag cgccattgat gcgcaatccg gcgtgatgcc cataactgaa aacagcctgg 1140

atgccgaaca aggtttagtt ataccgccgt ttgtggagcc acatattcac ctggacacca 1200

cgcaaaccgc cggacaaccg aactggaatc agtccggcac gctgtttgaa ggcattgaac 1260

gctgggccga gcgcaaagcg ttattaaccc atgacgatgt gaaacaacgc gcatggcaaa 1320

cgctgaaatg gcagattgcc aacggcattc agcatgtgcg tacccatgtc gatgtttcgg 1380

atgcaacgct aactgcgctg aaagcaatgc tggaagtgaa gcaggaagtc gcgccgtgga 1440

ttgatctgca aatcgtcgcc ttccctcagg aagggatttt gtcgtatccc aacggtgaag 1500

cgttgctgga agaggcgtta cgcttagggg cagatgtagt gggggcgatt ccgcattttg 1560

aatttacccg tgaatacggc gtggagtcgc tgcataaaac cttcgccctg gcgcaaaaat 1620

acgaccgtct catcgacgtt cactgtgatg agatcgatga cgagcagtcg cgctttgtcg 1680

aaaccgttgc tgccctggcg caccatgaag gcatgggcgc gcgagtcacc gccagccaca 1740

ccacggcaat gcactcctat aacggggcgt atacctcacg cctgttccgc ttgctgaaaa 1800

tgtccggtat taactttgtc gccaacccgc tggtcaatat tcatctgcaa ggacgtttcg 1860

atacgtatcc aaaacgtcgc ggcatcacgc gcgttaaaga gatgctggag tccggcatta 1920

acgtctgctt tggtcacgat gatgtcttcg atccgtggta tccgctggga acggcgaata 1980

tgctgcaagt gctgcatatg gggctgcatg tttgccagtt gatgggctac gggcagatta 2040

acgatggcct gaatttaatc acccaccaca gcgcaaggac gttgaatttg caggattacg 2100

gcattgccgc cggaaacagc gccaacctga ttatcctgcc ggctgaaaat gggtttgatg 2160

cgctgcgccg tcaggttccg gtacgttatt cggtacgtgg cggcaaggtg attgccagca 2220

cacaaccggc acaaaccacc gtatatctgg agcagccaga agccatcgat tacaaacgtt 2280

gaagcggccg ctcgagtcta gcagatcctg ggaaaactgg acctagaggg cccgtttaaa 2340

cccgctgatc agcctcgact gtgccttcta gttgccagcc atctgttgtt tgcccctccc 2400

ccgtgccttc cttgaccctg gaaggtgcca ctcccactgt cctttcctaa taaaatgagg 2460

aaattgcatc gcattgtctg agtaggtgtc attctattct ggggggtggg gtggggcagg 2520

acagcaaggg ggaggattgg gaagacaata gcaggcatgc tggggatgcg gtgggctcta 2580

tggcttctga ggcggaaaga accagctggg gctctagggg gtatccccac gcgccctgta 2640
gcggcgcatt aagcgcggcg ggtgtggtgg ttacgcgcag cgtgaccgct acacttgcca 2700
gcgccctagc gcccgctcct ttcgctttct tcccttcctt tctcgccacg ttcgccggct 2760
ttccccgtca agctctaaat cgggggctcc ctttagggtt ccgatttagt gctttacggc 2820
acctcgaccc caaaaaactt gattagggtg atggttcacg tagtgggcca tcgccctgat 2880
agacggtttt tcgccctttg acgttggagt ccacgttctt taatagtgga ctcttgttcc 2940
aaactggaac aacactcaac cctatctcgg tctattcttt tgatttataa gggattttgc 3000
cgatttcggc ctattggtta aaaaatgagc tgatttaaca aaaatttaac gcgaattaat 3060
tctgtggaat gtgtgtcagt tagggtgtgg aaagtcccca ggctccccag caggcagaag 3120
tatgcaaagc atgcatctca attagtcagc aaccaggtgt ggaaagtccc caggctcccc 3180
agcaggcaga agtatgcaaa gcatgcatct caattagtca gcaaccatag tcccgcccct 3240
aactccgccc atcccgcccc taactccgcc cagttccgcc cattctccgc cccatggctg 3300
actaattttt tttatttatg cagaggccga ggccgcctct gcctctgagc tattccagaa 3360
gtagtgagga ggcttttttg gaggcctagg cttttgcaaa aagctcccgg gagcttgtat 3420
atccattttc ggatctgatc aagagacagg atgaggatcg tttcgcatga ttgaacaaga 3480
tggattgcac gcaggttctc cggccgcttg ggtggagagg ctattcggct atgactgggc 3540
acaacagaca atcggctgct ctgatgccgc cgtgttccgg ctgtcagcgc aggggcgccc 3600
ggttcttttt gtcaagaccg acctgtccgg tgccctgaat gaactgcagg acgaggcagc 3660
gcggctatcg tggctggcca cgacgggcgt tccttgcgca gctgtgctcg acgttgtcac 3720
tgaagcggga agggactggc tgctattggg cgaagtgccg gggcaggatc tcctgtcatc 3780
tcaccttgct cctgccgaga aagtatccat catggctgat gcaatgcggc ggctgcatac 3840
gcttgatccg gctacctgcc cattcgacca ccaagcgaaa catcgcatcg agcgagcacg 3900
tactcggatg gaagccggtc ttgtcgatca ggatgatctg gacgaagagc atcaggggct 3960
cgcgccagcc gaactgttcg ccaggctcaa ggcgcgcatg cccgacggcg aggatctcgt 4020
cgtgacccat ggcgatgcct gcttgccgaa tatcatggtg gaaaatggcc gcttttctgg 4080
attcatcgac tgtggccggc tgggtgtggc ggaccgctat caggacatag cgttggctac 4140
ccgtgatatt gctgaagagc ttggcggcga atgggctgac cgcttcctcg tgctttacgg 4200
tatcgccgct cccgattcgc agcgcatcgc cttctatcgc cttcttgacg agttcttctg 4260
agcgggactc tggggttcga aatgaccgac caagcgacgc ccaacctgcc atcacgagat 4320
ttcgattcca ccgccgcctt ctatgaaagg ttgggcttcg gaatcgtttt ccgggacgcc 4380
ggctggatga tcctccagcg cggggatctc atgctggagt tcttcgccca ccccaacttg 4440
tttattgcag cttataatgg ttacaaataa agcaatagca tcacaaattt cacaaataaa 4500
gcattttttt cactgcattc tagttgtggt ttgtccaaac tcatcaatgt atcttatcat 4560
gtctgtatac cgtcgacctc tagctagagc ttggcgtaat catggtcata gctgtttcct 4620

gtgtgaaatt gttatccgct cacaattcca cacaacatac gagccggaag cataaagtgt 4680
aaagcctggg gtgcctaatg agtgagctaa ctcacattaa ttgcgttgcg ctcactgccc 4740
gctttccagt cgggaaacct gtcgtgccag ctgcattaat gaatcggcca acgcgcgggg 4800
agaggcggtt tgcgtattgg gcgctcttcc gcttcctcgc tcactgactc gctgcgctcg 4860
gtcgttcggc tgcggcgagc ggtatcagct cactcaaagg cggtaatacg gttatccaca 4920
gaatcagggg ataacgcagg aaagaacatg tgagcaaaag gccagcaaaa ggccaggaac 4980
cgtaaaaagg ccgcgttgct ggcgtttttc cataggctcc gcccccctga cgagcatcac 5040
aaaaatcgac gctcaagtca gaggtggcga aacccgacag gactataaag ataccaggcg 5100
tttccccctg gaagctccct cgtgcgctct cctgttccga ccctgccgct taccggatac 5160
ctgtccgcct ttctcccttc gggaagcgtg gcgctttctc atagctcacg ctgtaggtat 5220
ctcagttcgg tgtaggtcgt tcgctccaag ctgggctgtg tgcacgaacc ccccgttcag 5280
cccgaccgct gcgccttatc cggtaactat cgtcttgagt ccaacccggt aagacacgac 5340
ttatcgccac tggcagcagc cactggtaac aggattagca gagcgaggta tgtaggcggt 5400
gctacagagt tcttgaagtg gtggcctaac tacggctaca ctagaagaac agtatttggt 5460
atctgcgctc tgctgaagcc agttaccttc ggaaaaagag ttggtagctc ttgatccggc 5520
aaacaaacca ccgctggtag cggttttttt gtttgcaagc agcagattac gcgcagaaaa 5580
aaaggatctc aagaagatcc tttgatcttt tctacggggt ctgacgctca gtggaacgaa 5640
aactcacgtt aagggatttt ggtcatgaga ttatcaaaaa ggatcttcac ctagatcctt 5700
ttaaattaaa aatgaagttt taaatcaatc taaagtatat atgagtaaac ttggtctgac 5760
agttaccaat gcttaatcag tgaggcacct atctcagcga tctgtctatt tcgttcatcc 5820
atagttgcct gactccccgt cgtgtagata actacgatac gggagggctt accatctggc 5880
cccagtgctg caatgatacc gcgagaccca cgctcaccgg ctccagattt atcagcaata 5940
aaccagccag ccggaagggc cgagcgcaga agtggtcctg caactttatc cgcctccatc 6000
cagtctatta attgttgccg ggaagctaga gtaagtagtt cgccagttaa tagtttgcgc 6060
aacgttgttg ccattgctac aggcatcgtg gtgtcacgct cgtcgtttgg tatggcttca 6120
ttcagctccg gttcccaacg atcaaggcga gttacatgat cccccatgtt gtgcaaaaaa 6180
gcggttagct ccttcggtcc tccgatcgtt gtcagaagta agttggccgc agtgttatca 6240
ctcatggtta tggcagcact gcataattct cttactgtca tgccatccgt aagatgcttt 6300
tctgtgactg gtgagtactc aaccaagtca ttctgagaat agtgtatgcg gcgaccgagt 6360
tgctcttgcc cggcgtcaat acgggataat accgcgccac atagcagaac tttaaaagtg 6420
ctcatcattg gaaaacgttc ttcggggcga aaactctcaa ggatcttacc gctgttgaga 6480
tccagttcga tgtaacccac tcgtgcaccc aactgatctt cagcatcttt tactttcacc 6540
agcgtttctg ggtgagcaaa aacaggaagg caaaatgccg caaaaaaggg aataagggcg 6600
acacggaaat gttgaatact catactcttc ctttttcaat attattgaag catttatcag 6660

ggttattgtc tcatgagcgg atacatattt gaatgtattt agaaaaataa acaaataggg 6720

gttccgcgca catttccccg aaaagtgcca cctgacgtc 6759

<210> 160
<211> 64
<212> RNA
<213> Homo sapiens

<400> 160
ccccgcgacg agccccucgc acaaaccgga ccugagcguu uuguucguuc ggcucgcgug 60

aggc 64

<210> 161
<211> 22
<212> RNA
<213> Homo sapiens

<400> 161
uuuguucguu cggcucgcgu ga 22

<210> 162
<211> 110
<212> RNA
<213> Homo sapiens

<400> 162
ccugugcaga gauuauuuuu uaaaagguca caaucaacau ucauugcugu cgguggguug 60

aacugugugg acaagcucac ugaacaauga augcaacugu ggccccgcuu 110

<210> 163
<211> 22
<212> RNA
<213> Homo sapiens

<400> 163
aacauucauu gcugucggug gg 22

<210> 164
<211> 22
<212> RNA
<213> Homo sapiens

<400> 164
ucacgcgagc cgaacgaaca aa 22

<210> 165
<211> 22
<212> RNA
<213> Homo sapiens

<400> 165
cccaccgaca gcaaugaaug uu 22

<210> 166
<211> 87
<212> RNA
<213> Homo sapiens

<400>  166
ugagggcccc ucugcguguu cacagcggac cuugauuuaa ugucuauaca auuaaggcac      60

gcggugaaug ccaagagagg cgccucc                                          87


<210>  167
<211>  22
<212>  RNA
<213>  Homo sapiens

<400>  167
uuaaggcacg cggugaaugc ca                                               22


<210>  168
<211>  87
<212>  RNA
<213>  Homo sapiens

<400>  168
ggaagcgagu uguuaucuuu gguuaucuag cuguaugagu guauuggucu ucauaaagcu      60

agauaaccga aaguaaaaac uccuuca                                          87


<210>  169
<211>  23
<212>  RNA
<213>  Homo sapiens

<400>  169
ucuuugguua ucuagcugua uga                                              23


<210>  170
<211>  22
<212>  RNA
<213>  Homo sapiens

<400>  170
uggcauucac cgcgugccuu aa                                               22


<210>  171
<211>  23
<212>  RNA
<213>  Homo sapiens

<400>  171
ucauacagcu agauaaccaa aga                                              23